# EUROPEAN PATENT APPLICATION

(11) **EP 2 862 855 A1**
(43) Date of publication of application: **22.04.2015**
(21) Application number: 13804194.2
(22) Date of filing: 13.06.2013
(51) Int. Cl.: C07D 249/06, A61K 31/4439, A61K 31/506, A61P 1/00, A61P 5/00, A61P 9/12, A61P 25/00, A61P 25/04, A61P 25/06, A61P 25/08, A61P 25/14, A61P 25/16, A61P 25/18, A61P 25/20, A61P 25/22, A61P 25/24, A61P 25/28, A61P 29/00, A61P 37/02, C07D 401/14

(54) **BRANCHED CHAIN ALKYL HETEROAROMATIC RING DERIVATIVE**

(30) Priority: 15.06.2012 JP 2012135278; 09.11.2012 JP 2012246834
(71) Applicant: Taisho Pharmaceutical Co., Ltd., Tokyo 170-8633 (JP)
(72) Inventor: ARAKI Yuko, Tokyo 170-8633 (JP); NOZAWA Dai, Tokyo 170-8633 (JP); SUZUKI Ryo, Tokyo 170-8633 (JP); OHTA Hiroshi, Tokyo 170-8633 (JP); FUTAMURA Aya, Tokyo 170-8633 (JP); ABE Masahito, Tokyo 170-8633 (JP); AMADA Hideaki, Tokyo 170-8633 (JP); KONISHI Kazuhide, Tokyo 170-8633 (JP); OGATA Yuya, Tokyo 170-8633 (JP)
(74) Representative: Hoffmann Eitle
(86) International application number: PCT/JP2013/066314
(87) International publication number: WO 2013/187466

(57) **Abstract**

A branched chain alkyl heteroaromatic ring derivative represented by formula (Ia) or a pharmaceutically acceptable salt thereof is useful for treatment or prophylaxis of diseases such as sleep disorder, depression, anxiety disorder, panic disorder, schizophrenia, drug dependence, Alzheimer's disease, Parkinson's disease, Huntington's disease, eating disorder, cephalalgia, hemicrania, pain, digestive diseases, epilepsy, inflammation, immune-related diseases, endocrine-related diseases and hypertension, on the basis of the orexin (OX) receptor antagonist activity.

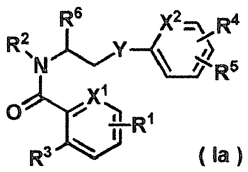

## Description

### Technical Field

The present invention relates to a compound having an orexin (OX) receptor antagonistic activity and a pharmaceutically acceptable salt thereof, and a therapeutic or preventive drug for disease such as sleep disorder, depression, anxiety disorder, panic disorder, schizophrenia, drug dependence, Alzheimer's disease, Parkinson's disease, Huntington's disease, eating disorder, headache, migraine, pain, gastrointestinal disease, epilepsy, inflammation, immunological diseases, endocrine diseases or hypertension, containing such a compound or salt as an active ingredient.

### Background Art

Orexin is a neuropeptide spliced from prepro-orexin, which is expressed specifically in the lateral hypothalamic area. Up to date, OX-A composed of 33 amino acids and OX-B composed of 28 amino acids have been identified, both of which are involved in the regulation of sleep-wake pattern and the regulation of feeding.

Both OX-A and OX-B act on OX receptors. Two subtypes, OX1 and OX2 receptors, of the OX receptors have been cloned so far, and both of which are known to be seven-transmembrane G protein-coupled receptors expressed mainly in the brain. OX1 receptor is coupled specifically with Gq among the G protein subclasses, whereas OX2 receptor is coupled with Gq and Gi/o (see Non Patent Literature 1 and Non Patent Literature 2).

Ox receptor subtypes are selectively expressed in the brain, and OX1 receptor is expressed in high density in the locus coeruleus, which is the nuclei originis of noradrenergic neurons, whereas OX2 receptor is expressed in high density in the tuberomammillary nucleus, which is the nuclei originis of histaminergic neuron (see Non Patent Literature 3, Non Patent Literature 4 and Non Patent Literature 5). The expression of both OX1 receptor and OX2 receptor are found in the raphe nucleus, which is the nuclei originis of serotoninergic neuron, and in the ventral tegmental area, which is the nuclei originis of dopaminergic neuron (see Non Patent Literature 3). The orexin neurons project to the monoaminergic neuron at the brain stem and the hypothalamus and have excitatory effects to these neurons, and further the expression of OX2 receptor is also found in the cholinergic neuron at the brain stem responsible for regulating REM sleep and have effects to the nucleus activities thereof (see Non Patent Literature 3 and Non Patent literature 4).

In recent years, OX1 and OX2 receptors are focused on the role of the sleep-wake regulation, and the usefulness of OX receptor antagonists have been studied. When OX-A is intracerebroventricularly administered to a rat, increased spontaneous locomotor activity (see Non Patent Literature 6 and Non Patent Literature 7), increased stereotyped behavior (see Non Patent Literature 7), increased time spent awake (see Non Patent Literature 6), and the like, were observed. Decreased REM sleep produced by OX-A administration is completely antagonized by the pretreatment of an OX receptor antagonist (see Non Patent Literature 8). Further, it is reported that locomotor activity is reduced, sleep latency is decreased, and amounts of non-REM sleep and REM sleep are increased by administering an orally available OX1 and OX2 receptors antagonist (see Non Patent Literature 9 and Non Patent Literature 10).

Patent Literature 1 discloses a pyrazole derivative as the compound having OX receptor antagonistic activities but does not disclose the compound having the pyrazole-branched chain alkylamide skeleton as described in the present application. Also, compounds, for example, having various structures described in Non Patent Literature 11 are generally known as OX receptor antagonists but the compounds having the heteroaromatic ring-branched chain alkylamide skeleton described in the present application are not disclosed. Meanwhile, Patent Literature 2 discloses compounds having a pyrazole-ethylamide skeleton and Patent Literature 3 discloses compounds having a heteroaromatic ring-branched chain alkylamide skeleton but Patent Literature 2 and Patent literature 3 do not disclose the OX receptor antagonistic activities or the compounds having the heteroaromatic ring-branched chain alkylamide skeleton described in the present application.

### Citation List

### Patent Literature

Patent Literature 1: WO2003/002559
Patent Literature 2: WO2008/062878
Patent Literature 3: WO2011/051540

Non Patent Literature 1: Zhu Y et al., J. Pharmacol. Sci., 92, 259-266, 2003.
Non Patent Literature 2: Zeitzer JM et al., Trends Pharmacol. Sci., 27, 368-374, 2006.
Non Patent Literature 3: Marcus JN et al., J. Comp. Neurol., 435, 6-25, 2001.
Non Patent Literature 4: Trivedi JP et al., FEBS Lett., 438, 71-75, 1998.
Non Patent Literature 5: Yamanaka A et al., Biochem. Biophys. Res. Commun., 290, 1237-1245, 2002.
Non Patent Literature 6: Hagan JJ et al., Proc. Natl. Acad. Sci. USA, 96, 10911-10916, 1999.
Non Patent Literature 7: Nakamura T et al., Brain Res., 873, 181-187, 2000.
Non Patent Literature 8: Smith MI et al., Neurosci. Lett., 341, 256-258, 2003.
Non Patent Literature 9: Brisbare-Roch C et al., Nat. Med., 13, 150-155, 2007.
Non Patent Literature 10: Cox CD et al., J. Med. Chem., 53, 5320-5332, 2010.
Non Patent Literature 11: John G et al., ChemMedChem., 5, 1197-1214, 2010.

### Summary of Invention

### Technical Problem

An object of the present invention is to find a novel compound which has an OX receptor antagonistic activity and provide a therapeutic or preventive drug for disease such as sleep disorder, depression, anxiety disorder, panic disorder, schizophrenia, drug dependence, Alzheimer's disease, Parkinson's disease, Huntington's disease, eating disorder, headache, migraine, pain, gastrointestinal disease, epilepsy, inflammation, immunological diseases, endocrine related disease or hypertension. More specifically, the object of the present invention is to provide a novel compound which exhibits good pharmacokinetics and safety together with a good OX receptor antagonistic activity.

### Solution to Problems

The present inventors extensively studied on novel skeleton compounds having an antagonistic activity against orexin receptors and found that certain branched chain alkyl heteroaromatic ring derivatives represented by the following formulae have good OX receptor antagonistic activities, whereby the present invention was accomplished.
Hereinafter, the present invention is described in detail. The aspects of the present invention (hereinafter referred to as "compound of the present invention") are as follows.
(1) A compound represented by formula (Ia): 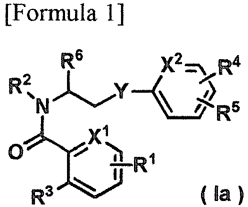 wherein,
   X¹ and X² are the same or different and represent a nitrogen atom or a formula CH;
   Y represents any of the structures in a following formula group (a): 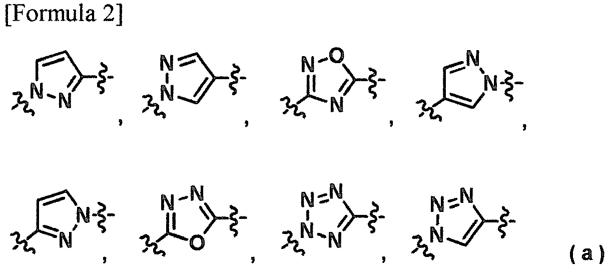
   R¹ represents a hydrogen atom, a halogen atom or a C₁₋₆ alkyl group;
   R² represents a C₁₋₆ alkyl group (the C₁₋₆ alkyl group may optionally be substituted with one substituent selected from the group consisting of a hydroxy group, a C₁₋₆ alkoxy group, a C₁₋₆ alkylsulfonyl group, a diC₁₋₆ alkylamino group and a cyano group);
   R³ represents a triazolyl group or a pyrimidinyl group;
   R⁴ and R⁵ are the same or different and represent a hydrogen atom, a halogen atom, a C₁₋₆ alkyl group, a hydroxyl group or a C₁₋₆ alkoxy group; and
   R⁶ represents a C₁₋₆ alkyl group;
   or a pharmaceutically acceptable salt thereof.
(2) A compound represented by formula (Ia): 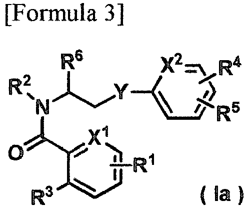 wherein,
   X¹ and X² are the same or different and represent a nitrogen atom or a formula CH;
   Y represents any of the structures in a following formula group (a): 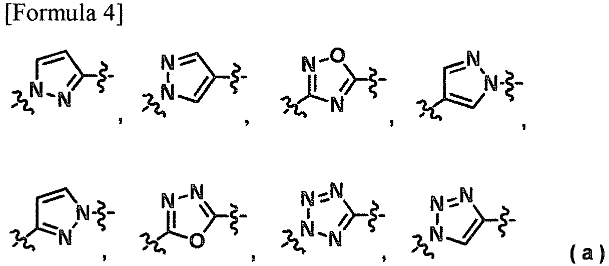
   R¹ represents a hydrogen atom, a halogen atom or a C₁₋₆ alkyl group;
   R² represents a C₁₋₆ alkyl group (the C₁₋₆ alkyl group may optionally be substituted with one
   substituent selected from the group consisting of a hydroxy group and a C₁₋₆ alkoxy group);
   R³ represents a triazolyl group or a pyrimidinyl group;
   R⁴ represents a halogen atom;
   R⁵ represents a hydrogen atom or a halogen atom; and
   R⁶ represents a C₁₋₆ alkyl group;
   or a pharmaceutically acceptable salt thereof.
(3) The compound or a pharmaceutically acceptable salt thereof according to (1) or (2), wherein Y in the above formula (Ia) is any of the structures in a following formula group (a1): 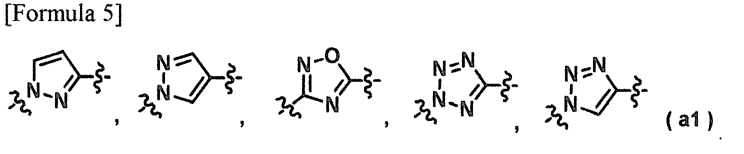
(4) The compound or a pharmaceutically acceptable salt thereof according to any one of (1) to (3), wherein R¹ in the above formula (Ia) is a hydrogen atom, a fluorine atom or a methyl group.
(5) The compound or a pharmaceutically acceptable salt thereof according to any one of (1) to (4), wherein R² in the above formula (Ia) is a methyl group or an ethyl group (the ethyl group may optionally be substituted with one substituent selected from the group consisting of a hydroxyl group and a methoxy group).
(6) The compound or a pharmaceutically acceptable salt thereof according to any one of (1) to (5), wherein R⁴ in the above formula (Ia) is a fluorine atom.
(7) The compound or a pharmaceutically acceptable salt thereof according to any one of (1) to (6), wherein R⁵ in the above formula (Ia) is a hydrogen atom or a fluorine atom.
(8) The compound or a pharmaceutically acceptable salt thereof according to any one of (1) to (7), wherein R⁶ in the above formula (Ia) is a C₁₋₃ alkyl group.
(9) The compound or a pharmaceutically acceptable salt thereof according to any one of (1) to (8), wherein Y in the above formula (Ia) is any of the structures in a following formula group (a2): 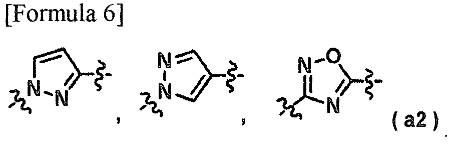
(10) The compound or a pharmaceutically acceptable salt thereof according to any one of (1) to (8), wherein Y in the above formula (Ia) is a following formula (a3): 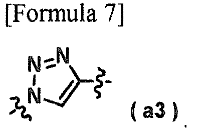
(11) The compound or a pharmaceutically acceptable salt thereof according to any one of (1) to (10), wherein R² in the above formula (Ia) is an ethyl group.
(12) The compound or a pharmaceutically acceptable salt thereof according to any one of (1) to (11), wherein R⁶ in the above formula (Ia) is a methyl group.
(13) The compound or a pharmaceutically acceptable salt thereof according to any one of (1) to (12), wherein the above formula (Ia) is represented by formula (IIa): 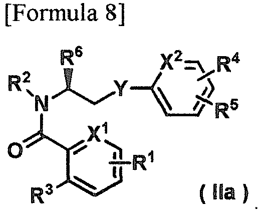
(14) The compound or a pharmaceutically acceptable salt thereof according to any one of (1) to (13), wherein the above formula (Ia) is represented by formula (IIIa): 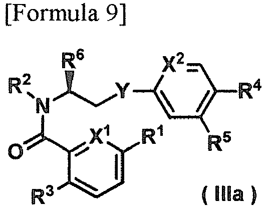
(15) A compound represented by formula (I): 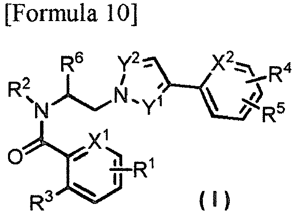 wherein,
   X¹ and X² are the same or different and represent a nitrogen atom or a formula CH; either one of Y¹ and Y² represents a nitrogen atom, and the other represents CH;
   R¹ represents a hydrogen atom, a halogen atom or a C₁₋₆ alkyl group;
   R² represents a C₁₋₆ alkyl group;
   R³ represents a triazolyl group or a pyrimidinyl group;
   R⁴ represents a halogen atom;
   R⁵ represents a hydrogen atom or a halogen atom; and
   R⁶ represents a C₁₋₆ alkyl group;
   or a pharmaceutically acceptable salt thereof.
(16) The compound or a pharmaceutically acceptable salt thereof according to (15), wherein R¹ in the above formula (I) is a hydrogen atom, a fluorine atom or a methyl group.
(17) The compound or a pharmaceutically acceptable salt thereof according to any of (15) or (16), wherein R² in the above formula (I) is a methyl group or an ethyl group.
(18) The compound or a pharmaceutically acceptable salt thereof according to any one of (15) to (17), wherein R⁴ in the above formula (I) is a fluorine atom.
(19) The compound or a pharmaceutically acceptable salt thereof according to any one of (15) to (18), wherein R⁵ in the above formula (I) is a hydrogen atom or a fluorine atom.
(20) The compound or a pharmaceutically acceptable salt thereof according to any one of (15) to (19), wherein R⁶ in the above formula (I) is a C₁₋₃ alkyl group.
(21) The compound or a pharmaceutically acceptable salt thereof according to any one of (15) to (20), wherein R⁶ in the above formula (I) is a methyl group.
(22) The compound or a pharmaceutically acceptable salt thereof according to any one of (15) to (21), wherein the above formula (I) is represented by formula (II): 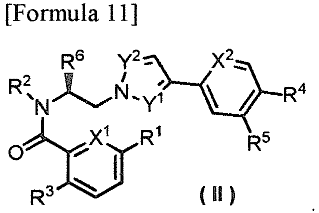
(23) The compound or a pharmaceutically acceptable salt thereof according to (1) above, which is a species or a mixture of two or more species selected from:
   N-ethyl-N-{(2S)-1-[3-(5-fluoropyridin-2-yl)-1H-pyrazol-1-yl]propan-2-yl}-5-methyl-2-(2H-1,2,3-triazol-2-yl)benzamide,
   N-ethyl-N-{(2R)-1-[3-(5-fluoropyridin-2-yl)-1H-pyrazol-1-yl]propan-2-yl}-5-methyl-2-(2H-1,2,3-triazol-2-yl)benzamide,
   N-ethyl-N-{(2S)-1-[3-(4-fluorophenyl)-1H-pyrazol-1-yl]propan-2-yl}-6-methyl-3-(2H-1,2,3-triazol-2-yl)pyridine-2-carboxamide,
   N-{(2S)-1-[3-(5-fluoropyridin-2-yl)-1H-pyrazol-1-yl]propan-2-yl}-N-methyl-2-(pyrimidin-2-yl)benzamide,
   5-fluoro-N-{(2S)-1-[3-(5-fluoropyridin-2-yl)-1H-pyrazol-1-yl]propan-2-yl}-N-methyl-2-(pyrimidin-2-yl)benzamide,
   N-{(2S)-1-[3-(5-fluoropyridin-2-yl)-1H-pyrazol-1-yl]propan-2-yl}-N,5-dimethyl-2-(pyrimidin-2-yl)benzamide,
   N-ethyl-N-{(2S)-1-[3-(5-fluoropyridin-2-yl)-1H-pyrazol-1-yl]propan-2-yl}-2-(pyrimidin-2-yl)benzamide,
   N-ethyl-5-fluoro-N-{(2S)-1-[3-(5-fluoropyridin-2-yl)-1H-pyrazol-1-yl]propan-2-yl}-2-(pyrimidin-2-yl)benzamide,
   N-ethyl-N-{(2S)-1-[3-(4-fluorophenyl)-1H-pyrazol-1-yl]propan-2-yl}-2-(pyrimidin-2-yl)benzamide,
   N-ethyl-5-fluoro-N-{(2S)-1-[3-(4-fluorophenyl)-1H-pyrazol-1-yl]propan-2-yl}-2-(pyrimidin-2-yl)benzamide,
   N-ethyl-2-fluoro-N-{(2S)-1-[3-(4-fluorophenyl)-1H-pyrazol-1-yl]propan-2-yl}-6-(pyrimidin-2-yl)benzamide,
   N-ethyl-4-fluoro-N-{(2S)-1-[3-(4-fluorophenyl)-1H-pyrazol-1-yl]propan-2-yl}-2-(pyrimidin-2-yl)benzamide,
   N-ethyl-N-{(2S)-1-[3-(4-fluorophenyl)-1H-pyrazol-1-yl]propan-2-yl}-6-methyl-3-(pyrimidin-2-yl)pyridine-2-carboxamide,
   N-{(2S)-1-[3-(3,4-difluorophenyl)-1H-pyrazol-1-yl]propan-2-yl}-N-ethyl-6-methyl-3-(2H-1,2,3-triazol-2-yl)pyridine-2-carboxamide,
   N-{(2S)-1-[3-(3,4-difluorophenyl)-1H-pyrazol-1-yl]propan-2-yl}-N-ethyl-5-fluoro-2-(pyrimidin-2-yl)benzamide,
   N-ethyl-N-{(2S)-1-[3-(5-fluoropyridin-2-yl)-1H-pyrazol-1-yl]propan-2-yl}-2-(2H-1,2,3-triazol-2-yl)benzamide,
   N-{(2S)-1-[3-(5-fluoropyridin-2-yl)-1H-pyrazol-1-yl]propan-2-yl}-N,5-dimethyl-2-(2H-1,2,3-triazol-2-yl)benzamide,
   N-{(2S)-1-[3-(5-fluoropyridin-2-yl)-1H-pyrazol-1-yl]propan-2-yl}-N-methyl-2-(2H-1,2,3-triazol-2-yl)benzamide,
   N-ethyl-N-{(2S)-1-[4-(5-fluoropyridin-2-yl)-1H-pyrazol-1-yl]propan-2-yl}-2-(2H-1,2,3-triazol-2-yl)benzamide,
   N-{(2S)-1-[4-(5-fluoropyridin-2-yl)-1H-pyrazol-1-yl]propan-2-yl}-N,5-dimethyl-2-(2H-1,2,3-triazol-2-yl)benzamide,
   N-{(2S)-1-[3-(5-fluoropyridin-2-yl)-1H-pyrazol-1-yl]butan-2-yl}-N,5-dimethyl-2-(2H-1,2,3-triazol-2-yl)benzamide,
   N-ethyl-N-{(2S)-1-[4-(5-fluoropyridin-2-yl)-1H-pyrazol-1-yl]propan-2-yl}-5-methyl-2-(2H-1,2,3-triazol-2-yl)benzamide,
   N-ethyl-N-{(2S)-1-[4-(4-fluorophenyl)-1H-pyrazol-1-yl]propan-2-yl}-6-methyl-3-(2H-1,2,3-triazol-2-yl)pyridine-2-carboxamide,
   N-{(2S)-1-[4-(3,4-difluorophenyl)-1H-pyrazol-1-yl]propan-2-yl}-N-ethyl-6-methyl-3-(2H-1,2,3-triazol-2-yl)pyridine-2-carboxamide,
   N-{{(2S)-1-[4-(4-fluorophenyl)-1H-pyrazol-1-yl]propan-2-yl}-N,6-dimethyl-3-(2H-1,2,3-triazol-2-yl)pyridine-2-carboxamide,
   N-{(2S)-1-[4-(3,4-difluorophenyl)-1H-pyrazol-1-yl]propan-2-yl}-N,6-dimethyl-3-(2H-1,2,3-triazol-2-yl)pyridine-2-carboxamide,
   N-ethyl-5-fluoro-N-{(2S)-1-[4-(4-fluorophenyl)-1H-pyrazol-1-yl]propan-2-yl}-2-(pyrimidin-2-yl)benzamide,
   N-{(2S)-1-[4-(3,4-difluorophenyl)-1H-pyrazol-1-yl]propan-2-yl}-N-ethyl-5-fluoro-2-(pyrimidin-2-yl)benzamide,
   5-fluoro-N-{(2S)-1-[4-(4-fluorophenyl)-1H-pyrazol-1-yl]propan-2-yl}-N-methyl-2-(pyrimidin-2-yl)benzamide,
   N-{(2S)-1-[4-(3,4-difluorophenyl)-1H-pyrazol-1-yl]propan-2-yl}-5-fluoro-N-methyl-2-(pyrimidin-2-yl)benzamide,
   N-ethyl-5-fluoro-N-{(2S)-1-[4-(5-fluoropyridin-2-yl)-1H-pyrazol-1-yl]propan-2-yl}-2-(pyrimidin-2-yl)benzamide,
   N-ethyl-N-{(2S)-1-[4-(5-fluoropyridin-2-yl)-1H-pyrazol-1-yl]propan-2-yl}-5-methyl-2-(pyrimidin-2-yl)benzamide,
   N-ethyl-5-fluoro-N-{(2S)-1-[4-(5-fluoropyridin-2-yl)-1H-pyrazol-1-yl]propan-2-yl}-2-(2H-1,2,3-triazol-2-yl)benzamide,
   5-chloro-N-ethyl-N-{(2S)-1-[4-(5-fluoropyridin-2-yl)-1H-pyrazol-1-yl]propan-2-yl}-2-(pyrimidin-2-yl)benzamide,
   N-{(2S)-1-[4-(5-fluoropyridin-2-yl)-1H-pyrazol-1-yl]propan-2-yl}-N-(2-methoxyethyl)-5-methyl-2-(2H-1,2,3-triazol-2-yl)benzamide,
   N-{(2S)-1-[3-(4-fluorophenyl)-1H-pyrazol-1-yl]propan-2-yl}-N-(2-methoxyethyl)-5-methyl-2-(2H-1,2,3-triazol-2-yl)benzamide,
   N-{(2S)-1-[3-(5-fluoropyridin-2-yl)-1H-pyrazol-1-yl]propan-2-yl}-N-(2-methoxyethyl)-5-methyl-2-(2H-1,2,3-triazol-2-yl)benzamide,
   N-{(2S)-1-[4-(5-fluoropyridin-2-yl)-1H-pyrazol-1-yl]propan-2-yl}-N-(2-hydroxyethyl)-5-methyl-2-(2H-1,2,3-triazol-2-yl)benzamide,
   N-{(2S)-1-[3-(4-fluorophenyl)-1H-pyrazol-1-yl]propan-2-yl}-N-(2-hydroxyethyl)-5-methyl-2-(2H-1,2,3-triazol-2-yl)benzamide,
   N-{(2S)-1-[4-(4-fluorophenyl)-1H-pyrazol-1-yl]propan-2-yl}-N-(2-hydroxyethyl)-5-methyl-2-(2H-1,2,3-triazol-2-yl)benzamide,
   N-{(2S)-1-[3-(5-fluoropyridin-2-yl)-1H-pyrazol-1-yl]propan-2-yl}-N-(2-hydroxyethyl)-5-methyl-2-(2H-1,2,3-triazol-2-yl)benzamide,
   N-{(2S)-1-[5-(5-fluoropyridin-2-yl)-1,2,4-oxadiazol-3-yl]propan-2-yl}-N,5-dimethyl-2-(2H-1,2,3 -triazol-2-yl)benzamide,
   N-{(2S)-1-[5-(5-fluoropyridin-2-yl)-1,2,4-oxadiazol-3-yl]propan-2-yl}-N,5-dimethyl-2-(pyrimidin-2-yl)benzamide,
   N-ethyl-N-{(2S)-1-[5-(5-fluoropyridin-2-yl)-1,2,4-oxadiazol-3-yl]propan-2-yl}-5-methyl-2-(2H-1,2,3-triazol-2-yl)benzamide,
   N-ethyl-N-{(2S)-1-[5-(5-fluoropyridin-2-yl)-1,2,4-oxadiazol-3-yl]propan-2-yl}-5-methyl-2-(pyrimidin-2-yl)benzamide,
   N-ethyl-N-{(2S)-1-[5-(4-fluorophenyl)-1,2,4-oxadiazol-3-yl]propan-2-yl}-6-methyl-3-(2H-1,2,3-triazol-2-yl)pyridine-2-carboxamide,
   N-ethyl-5-fluoro-N- {(2S)-1-[5-(4-fluorophenyl)-1,2,4-oxadiazol-3-yl]propan-2-yl}-2-(2H-1,2,3-triazol-2-yl)benzamide,
   N-ethyl-N-{(2S)-1-[5-(4-fluorophenyl)-1,2,4-oxadiazol-3-yl]propan-2-yl}-2-(2H-1,2,3-triazol-2-yl)benzamide,
   N-ethyl-N-{(2S)-1-[5-(4-fluorophenyl)-1,2,4-oxadiazol-3-yl]propan-2-yl}-6-methyl-3-(pyrimidin-2-yl)pyridine-2-carboxamide,
   N-ethyl-5-fluoro-N-{(2S)-1-[5-(4-fluorophenyl)-1,2,4-oxadiazol-3-yl]propan-2-yl}-2-(pyrimidin-2-yl)benzamide,
   N-ethyl-N-{(2S)-1-[5-(4-fluorophenyl)-1,2,4-oxadiazol-3-yl]propan-2-yl}-2-(pyrimidin-2-yl)benzamide,
   N-ethyl-N-{(2S)-1-[5-(4-fluorophenyl)-1,2,4-oxadiazol-3-yl]propan-2-yl}-5-methyl-2-(pyrimidin-2-yl)benzamide,
   N-ethyl-N-{(2S)-1-[5-(5-fluoropyridin-2-yl)-1,2,4-oxadiazol-3-yl]propan-2-yl}-2-(2H-1,2,3-triazol-2-yl)benzamide,
   N-ethyl-5-fluoro-N-{(2S)-1-[5-(5-fluoropyridin-2-yl)-1,2,4-oxadiazol-3-yl]propan-2-yl}-2-(2H-1,2,3-triazol-2-yl)benzamide,
   N-ethyl-N-{(2S)-1-[5-(5-fluoropyridin-2-yl)-1,2,4-oxadiazol-3-yl]propan-2-yl}-6-methyl-3-(2H-1,2,3-triazol-2-yl)pyridine-2-carboxamide,
   N-{(2S)-1-[5-(4-fluorophenyl)-1,2,4-oxadiazol-3-yl]propan-2-yl}-N,5-dimethyl-2-(2H-1,2,3-triazol-2-yl)benzamide,
   N-{(2S)-1-[5-(4-fluorophenyl)-1,2,4-oxadiazol-3-yl]propan-2-yl}-N,5-dimethyl-2-(pyrimidin-2-yl)benzamide,
   N-ethyl-N-{(2S)-1-[5-(4-fluorophenyl)-1,2,4-oxadiazol-3-yl]propan-2-yl}-5-methyl-2-(2H-1,2,3-triazol-2-yl)benzamide,
   N-ethyl-N-{(2S)-1-[5-(4-fluorophenyl)-2H-tetrazol-2-yl]propan-2-yl}-5-methyl-2-(2H-1,2,3-triazol-2-yl)benzamide,
   N-{(2S)-1-[5-(4-fluorophenyl)-2H-tetrazol-2-yl]propan-2-yl}-N-(2-methoxyethyl)-5-methyl-2-(2H-1,2,3-triazol-2-yl)benzamide,
   N-{(2S)-1-[5-(4-fluorophenyl)-2H-tetrazol-2-yl]propan-2-yl}-N-(2-hydroxyethyl)-5-methyl-2-(2H-1,2,3-triazol-2-yl)benzamide,
   N-ethyl-N-{(2S)-1-[5-(4-fluorophenyl)-2H-tetrazol-2-yl]propan-2-yl}-6-methyl-3-(2H-1,2,3-triazol-2-yl)pyridine-2-carboxamide,
   N-ethyl-5-fluoro-N-{(2S)-1-[5-(4-fluorophenyl)-2H-tetrazol-2-yl]propan-2-yl}-2-(2H-1,2,3-triazol-2-yl)benzamide,
   N-ethyl-N-{(2S)-1-[5-(4-fluorophenyl)-2H-tetrazol-2-yl]propan-2-yl}-2-(2H-1,2,3-triazol-2-yl)benzamide,
   N-ethyl-N-{(2S)-1-[5-(4-fluorophenyl)-2H-tetrazol-2-yl]propan-2-yl}-5-methyl-2-(pyrimidin-2-yl)benzamide,
   N-ethyl-N-{(2S)-1-[5-(4-fluorophenyl)-2H-tetrazol-2-yl]propan-2-yl}-6-methyl-3-(pyrimidin-2-yl)pyridine-2-carboxamide,
   N-ethyl-5-fluoro-N-{(2S)-1-[5-(4-fluorophenyl)-2H-tetrazol-2-yl]propan-2-yl}-2-(pyrimidin-2-yl)benzamide,
   N-ethyl-N-{(2S)-1-[4-(4-fluorophenyl)-1H-1,2,3-triazol-1-yl]propan-2-yl}-5-methyl-2-(2H-1,2,3-triazol-2-yl)benzamide,
   N-{(2S)-1-[4-(4-fluorophenyl)-1H-1,2,3-triazol-1-yl]propan-2-yl}-N-(2-hydroxyethyl)-5-methyl-2-(2H-1,2,3-triazol-2-yl)benzamide,
   N-ethyl-N-{1-[1-(5-fluoropyridin-2-yl)-1H-pyrazol-4-yl]propan-2-yl}-5-methyl-2-(2H-1,2,3-triazol-2-yl)benzamide,
   N-ethyl-N-{1-[1-(5-fluoropyridin-2-yl)-1H-pyrazol-3-yl]propan-2-yl)-5-methyl-2-(2H-1,2,3-triazol-2-yl)benzamide,
   N-ethyl-N-{(2S)-1-[5-(4-fluorophenyl)-1,3,4-oxadiazol-2-yl]propan-2-yl}-5-methyl-2-(2H-1,2,3-triazol-2-yl)benzamide,
   N-ethyl-N-{(2S)-1-[4-(5-fluoropyridin-2-yl)-1H-1,2,3-triazol-1-yl]propan-2-yl}-5-methyl-2-(2H-1,2,3-triazol-2-yl)benzamide,
   N-ethyl-N-{(2S)-1-[4-(4-fluorophenyl)-1H-1,2,3-triazol-1-yl]propan-2-yl}-6-methyl-3-(2H-1,2,3-triazol-2-yl)pyridine-2-carboxamide,
   N-ethyl-5-fluoro-N-{(2S)-1-[4-(4-fluorophenyl)-1H-1,2,3-triazol-1-yl]propan-2-yl}-2-(2H-1,2,3-triazol-2-yl)benzamide,
   N-ethyl-N-{(2S)-1-[5-(5-fluoropyridin-2-yl)-2H-tetrazol-2-yl]propan-2-yl}-5-methyl-2-(2H-1,2,3-triazol-2-yl)benzamide,
   N-ethyl-5-fluoro-N-{(2S)-1-[5-(5-fluoropyridin-2-yl)-2H-tetrazol-2-yl]propan-2-yl}-2-(2H-1,2,3-triazol-2-yl)benzamide,
   N-ethyl-N-{(2S)-1-[5-(5-fluoropyridin-2-yl)-2H-tetrazol-2-yl]propan-2-yl}-5-methyl-2-(pyrimidin-2-yl)benzamide,
   N-[2-(dimethylamino)ethyl]-N-{(2S)-1-[4-(4-fluorophenyl)-1H-1,2,3-triazol-1-yl]propan-2-yl 5-methyl-2-(2H-1,2,3-triazol-2-yl)benzamide,
   N-{(2S)-1-[4-(4-fluorophenyl)-1H-1,2,3-triazol-1-yl]propan-2-yl}-5-methyl-N-[2-(methylsulfony)ethyl]-2-(2H-1,2,3-triazol-2-yl)benzamide,
   N-{(2S)-1-[4-(4-chlorophenyl)-1H-1,2,3-triazol-1-yl]propan-2-yl}-N-ethyl-5-methyl-2-(2H-1,2,3-triazol-2-yl)benzamide,
   N-ethyl-N-{(2S)-1-[4-(3-hydroxyphenyl)-1H-1,2,3-triazol-1-yl]propan-2-yl}-5-methyl-2-(2H-1,2,3-triazol-2-yl)benzamide,
   N-ethyl-5-methyl-N-{(2S)-1-[4-(pyridin-2-yl)-1H-1,2,3-triazol-1-yl]propan-2-yl}-2-(2H-1,2,3-triazol-2-yl)benzamide,
   N-ethyl-5-methyl-N-[(2S)-1-(4-phenyl-1H-1,2,3-triazol-1-yl)propan-2-yl]-2-(2H-1,2,3-triazol-2-yl)benzamide,
   N-ethyl-5-methyl-N-{(2S)-1-[4-(3-methylphenyl)-1H-1,2,3-triazol-1-yl]propan-2-yl} -2-(2H-1,2,3-triazol-2-yl)benzamide,
   N-ethyl-N-{(2S)-1-[4-(3-fluorophenyl)-1H-1,2,3-triazol-1-yl]propan-2-yl}-5-methyl-2-(2H-1,2,3-triazol-2-yl)benzamide,
   N-ethyl-N-{(2S)-1-[4-(4-fluoro-3-methoxyphenyl)-1H-1,2,3-triazol-1-yl]propan-2-yl}-5-methyl-2-(2H-1,2,3-triazol-2-yl)benzamide,
   N-ethyl-N-{(2S)-1-[4-(4-hydroxyphenyl)-1H-1,2,3-triazol-1-yl]propan-2-yl}-5-methyl-2-(2H-1,2,3-triazol-2-yl)benzamide,
   N-ethyl-N-{(2S)-1-[4-(3-methoxyphenyl)-1H-1,2,3-triazol-1-yl]propan-2-yl}-5-methyl-2-(2H-1,2,3-triazol-2-yl)benzamide, and
   N-{(2S)-1-[4-(4-fluorophenyl)-1H-pyrazol-1-yl]propan-2-yl]-N-(2-hydroxypropyl)-5-methyl-2-(2H-1,2,3-triazol-2-yl)benzamide.
(24) A pharmaceutical composition containing the compound or a pharmaceutically acceptable salt thereof according to any one of (1) to (23), as an active ingredient.
(25) A therapeutic or preventive drug for sleep disorder, depression, anxiety disorder, panic disorder, schizophrenia, drug dependence, Alzheimer's disease, Parkinson's disease, Huntington's disease, eating disorder, headache, migraine, pain, gastrointestinal disease, epilepsy, inflammation, immunological diseases, endocrine related disease or hypertension, containing the compound or a pharmaceutically acceptable salt thereof according to any one of (1) to (23), as an active ingredient.

### Advantageous Effects of Invention

It is revealed that the branched chain alkyl heteroaromatic ring derivative of the present invention shows an affinity to OX receptors and antagonistic activities against stimulation to the receptors by a physiological ligand.

### Description of Embodiments

The terms used in the present specification mean as follows.

The "halogen atom" refers to a fluorine atom, a chlorine atom, a bromine atom and an iodine atom.

The "C₁₋₆ alkyl group" means a linear or branched chain alkyl group having 1 to 6 carbon atoms and examples include groups such as methyl, ethyl, n-propyl, isopropyl, n-butyl, isobutyl, sec-butyl, tert-butyl, n-pentyl, isopentyl, neopentyl, tert-pentyl, 1-ethylpropyl, n-hexyl, isohexyl and neohexyl.

The C₁₋₃ alkyl group" means a linear or branched chain alkyl group having 1 to 3 carbon atoms and examples include groups such as methyl, ethyl, n-propyl and isopropyl.

The "C₁₋₆ alkoxy group" means a linear or branched chain alkoxyl group having 1 to 6 carbon atoms and examples include groups such as methoxy, ethoxy, n-propoxy, isopropoxy, n-butoxy, isobutoxy, sec-butoxy, tert-butoxy, n-pentyloxy, isopentyloxy, neopentyloxy, tert-pentyloxy, 1-ethylpropoxy and n-hexyloxy.

The "C₁₋₆ alkylsulfonyl group" means a sulfonyl group substituted with the above "C₁₋₆ alkyl group" and examples include groups such as methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, isopentylsulfonyl, neopentylsulfonyl, tert-pentylsulfonyl and n-hexylsulfonyl.

The "di C₁₋₆ alkylamino group" means an amino group having 2 of the above "C₁₋₆ alkyl group" as substituents, which are the same or different, and examples include dimethylamino, diethylamino, di(n-propyl)amino, di(isopropyl)amino, ethylmethylamino, methyl(n-propyl)amino and methyl (isopropyl)amino.

The "sleep disorder" used in the present specification refers to disorders at the disturbance of falling asleep, sleep, phase or awakening, where in including insomnia.

Further, the classification of insomnia includes disturbance of falling asleep, arousal during sleep, early-morning awakening and disturbance of deep sleep.

The "pharmaceutically acceptable salt" used in the present specification means a pharmaceutically acceptable acid addition salt and examples of the acid to be used include salts with an inorganic acid such as sulfuric acid, hydrochloric acid, hydrobromic acid, phosphoric acid and nitric acid; and salts with an organic acid such as acetic acid, benzoic acid, oxalic acid, lactic acid, malic acid, tartaric acid, fumaric acid, maleic acid, citric acid, malonic acid, mandelic acid, gluconic acid, galactaric acid, glucoheptonic acid, glycolic acid, glutamic acid, methanesulfonic acid, ethanesulfonic acid, benzenesulfonic acid, p-toluenesulfonic acid, camphor sulfonic acid and naphthalene-2-sulfonic acid. The conversion from a free compound to the above salt can be carried out by a conventional method.

Preferable embodiments of the compound of the present invention are described below.

Compounds, wherein Y is any of the structures in the following formula group (a1), are preferable, 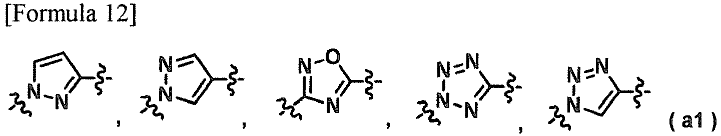 and compounds, wherein Y is any of the structures in the following formula group (a2), are further preferable.

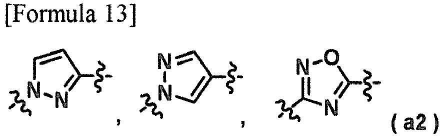

In another embodiment, compounds, wherein Y is the structure of the following formula (a3), are preferable.

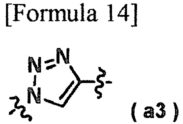

Compounds, wherein R¹ is a hydrogen atom, a fluorine atom or a methyl group, are preferable.

Compounds, wherein R² is a methyl group or an ethyl group (the ethyl group may optionally be substituted with one substituent selected from the group consisting of a hydroxyl group and a methoxy group), are preferable, with compounds, wherein R² is an ethyl group, being more preferable.

Compounds, wherein R³ is a 1,2,3-triazol-2-yl group or a pyrimidin-2-yl group, are preferable.

Compounds, wherein R⁴ is a fluorine atom, are preferable.

Compounds, wherein R⁵ is a hydrogen atom or a fluorine atom, are preferable.

Compounds, wherein R⁶ is a C₁₋₃ alkyl group, are preferable, with compounds, wherein R⁶ is a methyl group, being more preferable. Also, compounds, wherein the configuration of R⁶ substitution position is (S)-enantiomer, are preferable.

Examples of the preferable compound among the compounds of the present invention include:
N-ethyl-N-{(2S)-1-[3-(5-fluoropyridin-2-yl)-1H-pyrazol-1-yl]propan-2-yl}-5-methyl-2-(2H-1,2,3-triazol-2-yl)benzamide,
N-ethyl-N-{(2R)-1-[3-(5-fluoropyridin-2-yl)-1H-pyrazol-1-yl]propan-2-yl}-5-methyl-2-(2H-1,2,3-triazol-2-yl)benzamide,
N-ethyl-N-{(2S)-1-[3-(4-fluorophenyl)-1H-pyrazol-1-yl]propan-2-yl}-6-methyl-3-(2H-1,2,3-triazol-2-yl)pyridine-2-carboxamide,
N-{(2S)-1-[3-(5-fluoropyridin-2-yl)-1H-pyrazol-1-yl]propan-2-yl}-N-methyl-2-(pyrimidin-2-yl)benzamide,
5-fluoro-N-{{(2S)-1-[3-(5-fluoropyridin-2-yl)-1H-pyrazol-1-yl]propan-2-yl}-N-methyl-2-(pyrimidin-2-yl)benzamide,
N-{(2S)-1-[3-(5-fluoropyridin-2-yl)-1H-pyrazol-1-yl]propan-2-yl}-N,5-dimethyl-2-(pyrimidin-2-yl)benzamide,
N-ethyl-N-{(2S)-1-[3-(5-fluoropyridin-2-yl)-1H-pyrazol-1-yl]propan-2-yl}-2-(pyrimidin-2-yl)benzamide,
N-ethyl-5-fluoro-N-{(2S)-1-[3-(5-fluoropyridin-2-yl)-1H-pyrazol-1-yl]propan-2-yl}-2-(pyrimidin-2-yl)benzamide,
N-ethyl-N-{(2S)-1-[3-(4-fluorophenyl)-1H-pyrazol-1-yl]propan-2-yl}-2-(pyrimidin-2-yl)benzamide,
N-ethyl-5-fluoro-N-{(2S)-1-[3-(4-fluorophenyl)-1H-pyrazol-1-yl]propan-2-yl}-2-(pyrimidin-2-yl)benzamide,
N-ethyl-2-fluoro-N-{(2S)-1-[3-(4-fluorophenyl)-1H-pyrazol-1-yl]propan-2-yl}-6-(pyrimidin-2-yl)benzamide,
N-ethyl-4-fluoro-N-{(2S)-1-[3-(4-fluorophenyl)-1H-pyrazol-1-yl]propan-2-yl}-2-(pyrimidin-2-yl)benzamide,
N-ethyl-N-{(2S)-1-[3-(4-fluorophenyl)-1H-pyrazol-1-yl]propan-2-yl}-6-methyl-3-(pyrimidin-2-yl)pyridine-2-carboxamide,
N-{(2S)-1-[3-(3,4-difluorophenyl)-1H-pyrazol-1-yl]propan-2-yl}-N-ethyl-6-methyl-3-(2H-1,2,3-triazol-2-yl)pyridine-2-carboxamide,
N-{(2S)-1-[3-(3,4-difluorophenyl)-1H-pyrazol-1-yl]propan-2-yl}-N-ethyl-5-fluoro-2-(pyrimidin-2-yl)benzamide,
N-ethyl-N-{(2S)-1-[3-(5-fluoropyridin-2-yl)-1H-pyrazol-1-yl]propan-2-yl}-2-(2H-1,2,3-triazol-2-yl)benzamide,
N-{(2S)-1-[3-(5-fluoropyridin-2-yl)-1H-pyrazol-1-yl]propan-2-yl}-N,5-dimethyl-2-(2H-1,2,3-triazol-2-yl)benzamide,
N-{(2S)-1-[3-(5-fluoropyridin-2-yl)-1H-pyrazol-1-yl]propan-2-yl}-N-methyl-2-(2H-1,2,3-triazol-2-yl)benzamide,
N-ethyl-N-{(2S)-1-[4-(5-fluoropyridin-2-yl)-1H-pyrazol-1-yl]propan-2-yl}-2-(2H-1,2,3-triazol-2-yl)benzamide,
N-{(2S)-1-[4-(5-fluoropyridin-2-yl)-1H-pyrazol-1-yl]propan-2-yl}-N,5-dimethyl-2-(2H-1,2,3-triazol-2-yl)benzamide,
N-{(2S)-1-[3-(5-fluoropyridin-2-yl)-1H-pyrazol-1-yl]butan-2-yl}-N,5-dimethyl-2-(2H-1,2,3-triazol-2-yl)benzamide,
N-ethyl-N-{(2S)-1-[4-(5-fluoropyridin-2-yl)-1H-pyrazol-1-yl]propan-2-yl}-5-methyl-2-(2H-1,2,3-triazol-2-yl)benzamide,
N-ethyl-N-{(2S)-1-[4-(4-fluorophenyl)-1H-pyrazol-1-yl]propan-2-yl}-6-methyl-3-(2H-1,2,3-triazol-2-yl)pyridine-2-carboxamide,
N-{(2S)-1-[4-(3,4-difluorophenyl)-1H-pyrazol-1-yl]propan-2-yl}-N-ethyl-6-methyl-3-(2H-1,2,3-triazol-2-yl)pyridine-2-carboxamide,
N-{(2S)-1-[4-(4-fluorophenyl)-1H-pyrazol-1-yl]propan-2-yl}-N,6-dimethyl-3-(2H-1,2,3-triazol-2-yl)pyridine-2-carboxamide,
N-{(2S)-1-[4-(3,4-difluorophenyl)-1H-pyrazol-1-yl]propan-2-yl}-N,6-dimethyl-3-(2H-1,2,3-triazol-2-yl)pyridine-2-carboxamide,
N-ethyl-5-fluoro-N-{(2S)-1-[4-(4-fluorophenyl)-1H-pyrazol-1-yl]propan-2-yl}-2-(pyrimidin-2-yl)benzamide,
N-{(2S)-1-[4-(3,4-difluorophenyl)-1H-pyrazol-1-yl]propan-2-yl}-N-ethyl-5-fluoro-2-(pyrimidin-2-yl)benzamide,
5-fluoro-N-{(2S)-1-[4-(4-fluorophenyl)-1H-pyrazol-1-yl]propan-2-yl}-N-methyl-2-(pyrimidin-2-yl)benzamide,
N-{(2S)-1-[4-(3,4-difluorophenyl)-1H-pyrazol-1-yl]propan-2-yl}-5-fluoro-N-methyl-2-(pyrimidin-2-yl)benzamide,
N-ethyl-5-fluoro-N-{(2S)-1-[4-(5-fluoropyridin-2-yl)-1H-pyrazol-1-yl]propan-2-yl}-2-(pyrimidin-2-yl)benzamide,
N-ethyl-N-{(2S)-1-[4-(5-fluoropyridin-2-yl)-1H-pyrazol-1-yl]propan-2-yl}-5-methyl-2-(pyrimidin-2-yl)benzamide,
N-ethyl-5-fluoro-N-{(2S)-1-[4-(5-fluoropyridin-2-yl)-1H-pyrazol-1-yl]propan-2-yl}-2-(2H-1,2,3-triazol-2-yl)benzamide,
5-chloro-N-ethyl-N-{(2S)-1-[4-(5-fluoropyridin-2-yl)-1H-pyrazol-1-yl]propan-2-yl}-2-(pyrimidin-2-yl)benzamide,
N-{(2S)-1-[4-(5-fluoropyridin-2-yl)-1H-pyrazol-1-yl]propan-2-yl}-N-(2-methoxyethyl)-5-methyl-2-(2H-1,2,3-triazol-2-yl)benzamide,
N-{(2S)-1-[3-(4-fluorophenyl)-1H-pyrazol-1-yl]propan-2-yl}-N-(2-methoxyethyl)-5-methyl-2-(2H-1,2,3-triazol-2-yl)benzamide,
N-{(2S)-1-[3-(5-fluoropyridin-2-yl)-1H-pyrazol-1-yl]propan-2-yl}-N-(2-methoxyethyl)-5-methyl-2-(2H-1,2,3-triazol-2-yl)benzamide,
N-{(2S)-1-[4-(5-fluoropyridin-2-yl)-1H-pyrazol-1-yl]propan-2-yl}-N-(2-hydroxyethyl)-5-methyl-2-(2H-1,2,3-triazol-2-yl)benzamide,
N-{(2S)-1-[3-(4-fluorophenyl)-1H-pyrazol-1-yl]propan-2-yl}-N-(2-hydroxyethyl)-5-methyl-2-(2H-1,2,3-triazol-2-yl)benzamide,
N-{(2S)-1-[4-(4-fluorophenyl)-1H-pyrazol-1-yl]propan-2-yl}-N-(2-hydroxyethyl)-5-methyl-2-(2H-1,2,3-triazol-2-yl)benzamide,
N-{(2S)-1-[3-(5-fluoropyridin-2-yl)-1H-pyrazol-1-yl]propan-2-yl}-N-(2-hydroxyethyl)-5-methyl-2-(2H-1,2,3-triazol-2-yl)benzamide,
N-{(2S)-1-[5-(5-fluoropyridin-2-yl)-1,2,4-oxadiazol-3-yl]propan-2-yl}-N,5-dimethyl-2-(2H-1,2,3-triazol-2-yl)benzamide,
N-{(2S)-1-[5-(5-fluoropyridin-2-yl)-1,2,4-oxadiazol-3-yl]propan-2-yl}-N,5-dimethyl-2-(pyrimidin-2-yl)benzamide,
N-ethyl-N-{(2S)-1-[5-(5-fluoropyridin-2-yl)-1,2,4-oxadiazol-3-yl]propan-2-yl}-5-methyl-2-(2H-1,2,3-triazol-2-yl)benzamide,
N-ethyl-N-{(2S)-1-[5-(5-fluoropyridin-2-yl)-1,2,4-oxadiazol-3-yl]propan-2-yl}-5-methyl-2-(pyrimidin-2-yl)benzamide,
N-ethyl-N-{(2S)-1-[5-(4-fluorophenyl)-1,2,4-oxadiazol-3-yl]propan-2-yl}-6-methyl-3-(2H-1,2,3-triazol-2-yl)pyridine-2-carboxamide,
N-ethyl-5-fluoro-N-{(2S)-1-[5-(4-fluorophenyl)-1,2,4-oxadiazol-3-yl]propan-2-yl}-2-(2H-1,2,3-triazol-2-yl)benzamide,
N-ethyl-N-{(2S)-1-[5-(4-fluorophenyl)-1,2,4-oxadiazol-3-yl]propan-2-yl}-2-(2H-1,2,3-triazol-2-yl)benzamide,
N-ethyl-N-{(2S)-1-[5-(4-fluorophenyl)-1,2,4-oxadiazol-3-yl]propan-2-yl}-6-methyl-3-(pyrimidin-2-yl)pyridine-2-carboxamide,
N-ethyl-5-fluoro-N-{(2S)-1-[5-(4-fluorophenyl)-1,2,4-oxadiazol-3-yl]propan-2-yl}-2-(pyrimidin-2-yl)benzamide,
N-ethyl-N-{(2S)-1-[5-(4-fluorophenyl)-1,2,4-oxadiazol-3-yl]propan-2-yl}-2-(pyrimidin-2-yl)benzamide,
N-ethyl-N-{(2S)-1-[5-(4-fluorophenyl)-1,2,4-oxadiazol-3-yl]propan-2-yl}-5-methyl-2-(pyrimidin-2-yl)benzamide,
N-ethyl-N-{(2S)-1-[5-(5-fluoropyridin-2-yl)-1,2,4-oxadiazol-3-yl]propan-2-yl}-2-(2H-1,2,3-triazol-2-yl)benzamide,
N-ethyl-5-fluoro-N-{(2S)-1-[5-(5-fluoropyridin-2-yl)-1,2,4-oxadiazol-3-yl]propan-2-yl}-2-(2H-1,2,3-triazol-2-yl)benzamide,
N-ethyl-N-{(2S)-1-[5-(5-fluoropyridin-2-yl)-1,2,4-oxadiazol-3-yl]propan-2-yl}-6-methyl-3-(2H-1,2,3-triazol-2-yl)pyridine-2-carboxamide,
N-{(2S)-1-[5-(4-fluorophenyl)-1,2,4-oxadiazol-3-yl]propan-2-yl}-N,5-dimethyl-2-(2H-1,2,3-triazol-2-yl)benzamide,
N-{(2S)-1-[5-(4-fluorophenyl)-1,2,4-oxadiazol-3-yl]propan-2-yl}-N,5-dimethyl-2-(pyrimidin-2-yl)benzamide,
N-ethyl-N-{(2S)-1-[5-(4-fluorophenyl)-1,2,4-oxadiazol-3-yl]propan-2-yl}-5-methyl-2-(2H-1,2,3-triazol-2-yl)benzamide,
N-ethyl-N-{(2S)-1-[5-(4-fluorophenyl)-2H-tetrazol-2-yl]propan-2-yl}-5-methyl-2-(2H-1,2,3-triazol-2-yl)benzamide,
N-{(2S)-1-[5-(4-fluorophenyl)-2H-tetrazol-2-yl]propan-2-yl}-N-(2-methoxyethyl)-5-methyl-2-(2H-1,2,3-triazol-2-yl)benzamide,
N-{(2S)-1-[5-(4-fluorophenyl)-2H-tetrazol-2-yl]propan-2-yl}-N-(2-hydroxyethyl)-5-methyl-2-(2H-1,2,3-triazol-2-yl)benzamide,
N-ethyl-N-{(2S)-1-[5-(4-fluorophenyl)-2H-tetrazol-2-yl]propan-2-yl}-6-methyl-3-(2H-1,2,3-triazol-2-yl)pyridine-2-carboxamide,
N-ethyl-5-fluoro-N-{(2S)-1-[5-(4-fluorophenyl)-2H-tetrazol-2-yl]propan-2-yl}-2-(2H-1,2,3-triazol-2-yl)benzamide,
N-ethyl-N-{(2S)-1-[5-(4-fluorophenyl)-2H-tetrazol-2-yl]propan-2-yl}-2-(2H-1,2,3-triazol-2-yl)benzamide,
N-ethyl-N-{(2S)-1-[5-(4-fluorophenyl)-2H-tetrazol-2-yl]propan-2-yl}-5-methyl-2-(pyrimidin-2-yl)benzamide,
N-ethyl-N-{(2S)-1-[5-(4-fluorophenyl)-2H-tetrazol-2-yl]propan-2-yl}-6-methyl-3-(pyrimidin-2-yl)pyridine-2-carboxamide,
N-ethyl-5-fluoro-N-{(2S)-1-[5-(4-fluorophenyl)-2H-tetrazol-2-yl]propan-2-yl}-2-(pyrimidin-2-yl)benzamide,
N-ethyl-N-{(2S)-1-[4-(4-fluorophenyl)-1H-1,2,3-triazol-1-yl]propan-2-yl}-5-methyl-2-(2H-1,2,3-triazol-2-yl)benzamide,
N-{(2S)-1-[4-(4-fluorophenyl)-1H-1,2,3-triazol-1-yl]propan-2-yl}-N-(2-hydroxyethyl)-5-methyl-2-(2H-1,2,3-triazol-2-yl)benzamide,
N-ethyl-N-{1-[1-(5-fluoropyridin-2-yl)-1H-pyrazol-4-yl]propan-2-yl}-5-methyl-2-(2H-1,2,3-triazol-2-yl)benzamide,
N-ethyl-N-{1-[1-(5-fluoropyridin-2-yl)-1H-pyrazol-3-yl]propan-2-yl}-5-methyl-2-(2H-1,2,3-triazol-2-yl)benzamide,
N-ethyl-N-{(2S)-1-[5-(4-fluorophenyl)-1,3,4-oxadiazol-2-yl]propan-2-yl}-5-methyl-2-(2H-1,2,3-triazol-2-yl)benzamide,
N-ethyl-N-{(2S)-1-[4-(5-fluoropyridin-2-yl)-1H-1,2,3-triazol-1-yl]propan-2-yl}-5-methyl-2-(2H-1,2,3-triazol-2-yl)benzamide,
N-ethyl-N-{(2S)-1-[4-(4-fluorophenyl)-1H-1,2,3-triazol-1-yl]propan-2-yl}-6-methyl-3-(2H-1,2,3-triazol-2-yl)pyridine-2-carboxamide,
N-ethyl-5-fluoro-N-{(2S)-1-[4-(4-fluorophenyl)-1H-1,2,3-triazol-1-yl]propan-2-yl}-2-(2H-1,2,3-triazol-2-yl)benzamide,
N-ethyl-N-{(2S)-1-[5-(5-fluoropyridin-2-yl)-2H-tetrazol-2-yl]propan-2-yl}-5-methyl-2-(2H-1,2,3-triazol-2-yl)benzamide,
N-ethyl-5-fluoro-N-{(2S)-1-[5-(5-fluoropyridin-2-yl)-2H-tetrazol-2-yl]propan-2-yl}-2-(2H-1,2,3-triazol-2-yl)benzamide,
N-ethyl-N-{(2S)-1-[5-(5-fluoropyridin-2-yl)-2H-tetrazol-2-yl]propan-2-yl}-5-methyl-2-(pyrimidin-2-yl)benzamide,
N-[2-(dimethylamino)ethyl]-N-{(2S)-1-[4-(4-fluorophenyl)-1H-1,2,3-triazol-1-yl]propan-2-yl}-5-methyl-2-(2H-1,2,3-triazol-2-yl)benzamide,
N-{(2S)-1-[4-(4-fluorophenyl)-1H-1,2,3-triazol-1-yl]propan-2-yl}-5-methyl-N-[2-(methylsulfony)ethyl]-2-(2H-1,2,3-triazol-2-yl)benzamide,
N-{(2S)-1-[4-(4-chlorophenyl)-1H-1,2,3-triazol-1-yl]propan-2-yl}-N-ethyl-5-methyl-2-(2H-1,2,3-triazol-2-yl)benzamide,
N-ethyl-N-{(2S)-1-[4-(3-hydroxyphenyl)-1H-1,2,3-triazol-1-yl]propan-2-yl}-5-methyl-2-(2H-1,2,3-triazol-2-yl)benzamide,
N-ethyl-5-methyl-N-{(2S)-1-[4-(pyridin-2-yl)-1H-1,2,3-triazol-1-yl]propan-2-yl}-2-(2H-1,2,3-triazol-2-yl)benzamide,
N-ethyl-5-methyl-N-[(2S)-1-(4-phenyl-1H-1,2,3-triazol-1-yl)propan-2-yl]-2-(2H-1,2,3-triazol-2-yl)benzamide,
N-ethyl-5-methyl-N-{(2S)-1-[4-(3-methylphenyl)-1H-1,2,3-triazol-1-yl]propan-2-yl}-2-(2H-1,2,3-triazol-2-yl)benzamide,
N-ethyl-N-{(2S)-1-[4-(3-fluorophenyl)-1H-1,2,3-triazol-1-yl]propan-2-yl}-5-methyl-2-(2H-1,2,3-triazol-2-yl)benzamide,
N-ethyl-N-{(2S)-1-[4-(4-fluoro-3-methoxyphenyl)-1H-1,2,3-triazol-1-yl]propan-2-yl}-5-methyl-2-(2H-1,2,3-triazol-2-yl)benzamide,
N-ethyl-N-{(2S)-1-[4-(4-hydroxyphenyl)-1H-1,2,3-triazol-1-yl]propan-2-yl}-5-methyl-2-(2H-1,2,3-triazol-2-yl)benzamide,
N-ethyl-N-{(2S)-1-[4-(3-methoxyphenyl)-1H-1,2,3-triazol-1-yl]propan-2-yl}-5-methyl-2-(2H-1,2,3-triazol-2-yl)benzamide, and
N-{(2S)-1-[4-(4-fluorophenyl)-1H-pyrazol-1-yl]propan-2-yl}-N-(2-hydroxypropyl)-5-methyl-2-(2H-1,2,3-triazol-2-yl)benzamide,
or pharmaceutically acceptable salts thereof.

Additionally, when the compound of the present invention forms a hydrate or a solvate, they are also encompassed in the scope of the present invention. Similarly, pharmaceutically acceptable salts of the hydrates or solvates of the compound of the present invention are also encompassed in the scope of the present invention.

The compound of the present invention encompasses all of the enantiomers, diastereomers, equilibrium compounds, mixtures thereof in any ratio, racemic compounds, and the like.

The compound according to the present invention also includes those wherein at least one hydrogen atom, carbon atom, nitrogen atom, oxygen atom and halogen atom is substituted with a radioactive isotope or a stable isotope. These labelled compounds are useful for the studies on metabolism and pharmacokinetics and for biological analysis, or the like, as a receptor ligand, or the like.

The compound according to the present invention can be administered orally or parenterally. Dosage form thereof may be tablets, capsules, granules, powders, dusts, troches, ointments, creams, plasters, emulsions, suspensions, suppositories, injections, or the like, and any of which can be produced by a routine pharmaceutical preparation technique (for example, methods stipulated in The Japanese Pharmacopoeia Fifteenth Edition, or the like). These dosage forms can suitably be selected in accordance with patient's symptoms, age, body weight and purpose of treatment.

These pharmaceutical preparations can be produced by adding pharmacologically acceptable carriers, more specifically, excipients (for example, crystalline cellulose, starch, lactose, mannitol), binders (for example, hydroxypropylcellulose, polyvinylpyrrolidone), lubricants (for example, magnesium stearate, talc), disintegrators (for example, carboxymethyl cellulose calcium) and other pharmacologically acceptable various additives, to a composition containing the compound of the present invention.

The compound of the present invention can be orally or parenterally administered to an adult patient in a single dose of 0.001 to 500 mg once or in several divided times a day. Additionally, the dose can suitably be increased or reduced depending on the disease type to be treated, patient's age, body weight, symptoms, and the like.

Typical production methods of the compounds (I) and (Ia) of the present invention are shown below in Schemes A to J.
The following methods are examples of the production method of the compounds of the present invention, and the present invention is not limited thereto. Additionally, in the following examples of the production method, the compounds may form a salt unless the reactions are affected. 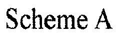 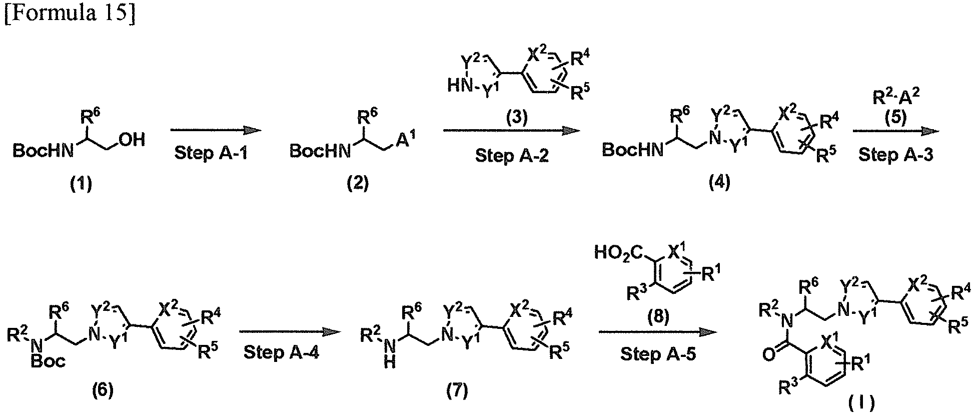 wherein A¹ and A² represent a halogen atom, a methanesulfonyloxy group, a p-toluenesulfonyloxy group or a trifluoromethanesulfonyloxy group, and other symbols are as defined above.)
Step A-1: The compound (2) can be obtained by converting the hydroxy group of the compound (1) to a general leaving group. Examples of the reaction in Step A-1 include chlorination, bromination, iodization, methanesulfonyloxylation and p-toluenesulfonyloxylation. An example of the chlorination reaction incudes a method wherein a leaving group is obtained using, for example, methanesulfonyl chloride, or the like, followed by substitution with a chlorine atom. A method which uses carbon tetrachloride and triphenyl phosphine and a method which uses thionyl chloride or phosphorus oxychloride are further included. During these procedures, a chloride such as sodium chloride or potassium chloride may be added. An example of the bromination reaction includes a method wherein, for example, carbon tetrabromide and triphenyl phosphine are used. An example of the iodization reaction includes a method wherein, for example, iodine, triphenyl phosphine and imidazole are used. The methanesulfonylation and p-toluenesulfonylation of the compound (1) can be achieved using, for example, methanesulfonyl chloride, p-toluenesulfonyl chloride, or the like, respectively. During these reactions, a suitable base may be added. Examples of the base to be added include organic bases such as triethylamine and diisopropylethylamine or inorganic bases such as potassium carbonate. Examples of the reaction solvent include ether solvents such as tetrahydrofuran, aprotic polar solvents such as N,N-dimethylformamide, halogen solvents such as chloroform, acetonitrile or mixed solvents thereof, and therein the reactions can be carried out under the temperature condition of about -80°C to about the boiling point of such a solvent.
Step A-2: The compound (4) can be obtained by reacting the compound (2) and the compound (3). The reaction in Step A-2 proceeds in an alcohol solvent such as ethanol, an ether solvent such as tetrahydrofuran, an aprotic polar solvent such as N,N-dimethylformamide, a halogen solvent such as chloroform, dimethyl sulfoxide, acetonitrile or a mixed solvent thereof, in the presence of an inorganic base such as sodium hydride, sodium hydroxide, sodium carbonate, potassium carbonate or cesium carbonate, an alkali metal such as sodium ethoxide or potassium tert-butoxide, or an organic base such as a lower alkoxide of the alkaline earth metal, under the temperature condition of about -80°C to about the boiling point of such a solvent.
Step A-3: The compound (6) can be obtained by the alkylation reaction of the compound (4). The reaction in Step A-3 can be carried out by a general method of amide alkylation. Examples of the base to be used in the present reaction include inorganic bases such as sodium hydride and sodium hydroxide, and metal lower alkoxides such as sodium ethoxide and tert-butoxy potassium. Examples of the solvent to be used in the present reaction include ether solvents such as tetrahydrofuran and 1,4-dioxane, aprotic polar solvents such as N,N-dimethylformamide, dimethyl sulfoxide and acetonitrile, aromatic hydrocarbon solvents such as toluene, or mixed solvents thereof. The present reaction can be carried out usually at 0°C to 150°C, preferably 20°C to 100°C.
Step A-4: The compound (7) can be obtained by deprotecting the tert-butoxycarbonyl group of the compound (6) in the presence of an acid such as hydrochloric acid, sulfuric acid, trifluoroacetic acid, p-toluenesulfonic acid or methanesulfonic acid. The comprehensive overview of the reaction in Step A-3 can be found in Protective Groups in Organic Chemistry, written by J. F.W. McOmie and Protective Groups in Organic Synthesis written by T. W. Greene and P. G. M. Wuts.
Step A-5: The compound (I) of the present invention can be obtained by the condensation reaction of the amine compound (7) and the carboxylic acid compound (8). The reaction in Step A-5 can be carried out by a general amidation method of carboxylic acid and amine. Examples include a method wherein carboxylic acid is converted to a carboxylic acid halide such as carboxylic acid chloride or carboxylic acid bromide and subsequently reacted with amine and a method wherein carboxylic acid is reacted with amine in the presence of a dehydration condensation agent. These reactions can be carried out in the presence or absence of a base in a solvent. Examples of the halogenating agent to be used in the present reaction can include thionyl chloride, oxalyl chloride, phosphorus oxychloride or phosphorus oxybromide. Also, examples of the dehydration condensation agent to be used in the present reaction include 1-ethyl-3-(3-dimethylaminopropyl) carbodiimide-hydrochloride (EDC-HCl), O-(7-azabenzotriazol-1-yl)-N,N,N',N'-tetramethyluronium-hexafluorophosphate (HATU), propane phosphonic acid anhydride, dicyclohexyl carbodiimide (DDC), diphenylphosphoryl azide (DPPA) and carbonyldiimidazole (CDI), and an activator such as 1-hydroxybenzotriazole or hydroxysuccinimide may be used as necessary. Examples of the solvent to be used in the present reaction include ether solvents such as tetrahydrofuran and 1,4-dioxane, aprotic polar solvents such as N,N-dimethylformamide and acetonitrile, halogen solvents such as dichloromethane and chloroform, aromatic hydrocarbon solvents such as toluene, ethyl acetate or mixed solvents thereof. Examples of the base to be used in the present reaction include organic amines such as pyridine, triethylamine and diisopropylethylamine and inorganic bases such as potassium carbonate, sodium carbonate and sodium hydrogen carbonate. The present reaction can be carried out usually at 0°C to 150°C, preferably 25°C to 80°C. 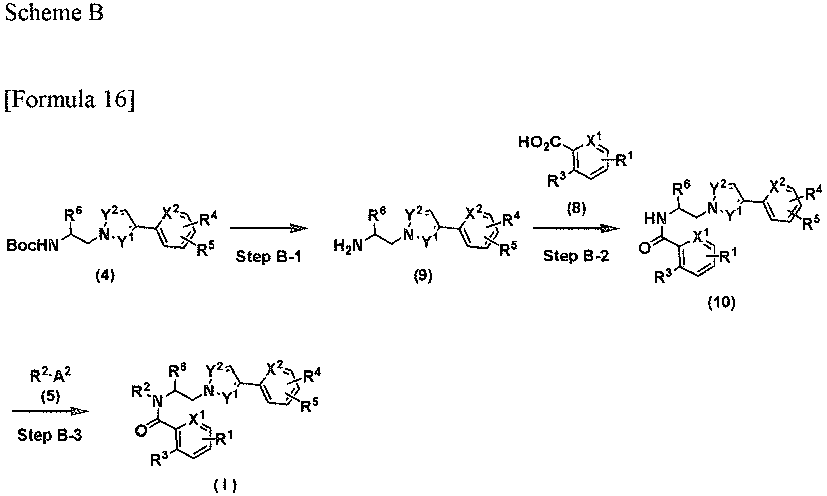
wherein the symbols are as defined above.

The compound (I) of the present invention can alternatively be produced by the method shown in Scheme B.
Step B-1: The compound (9) can be obtained by deprotecting the tert-butoxycarbonyl group of the compound (4) in the presence of an acid. The reaction in Step B-1 can be carried out in accordance with the same reaction conditions as in Step A-4.
Step B-2: The compound (10) can be obtained by the condensation reaction of the carboxylic acid compound (8) and the amine compound (9). The reaction in Step B-2 can be carried out in accordance with the same reaction conditions as in Step A-5.
Step B-3: The compound (I) of the present invention can be obtained by the alkylation reaction of the compound (10). The reaction in Step B-3 can be carried out in accordance with the same reaction conditions as in Step A-3. 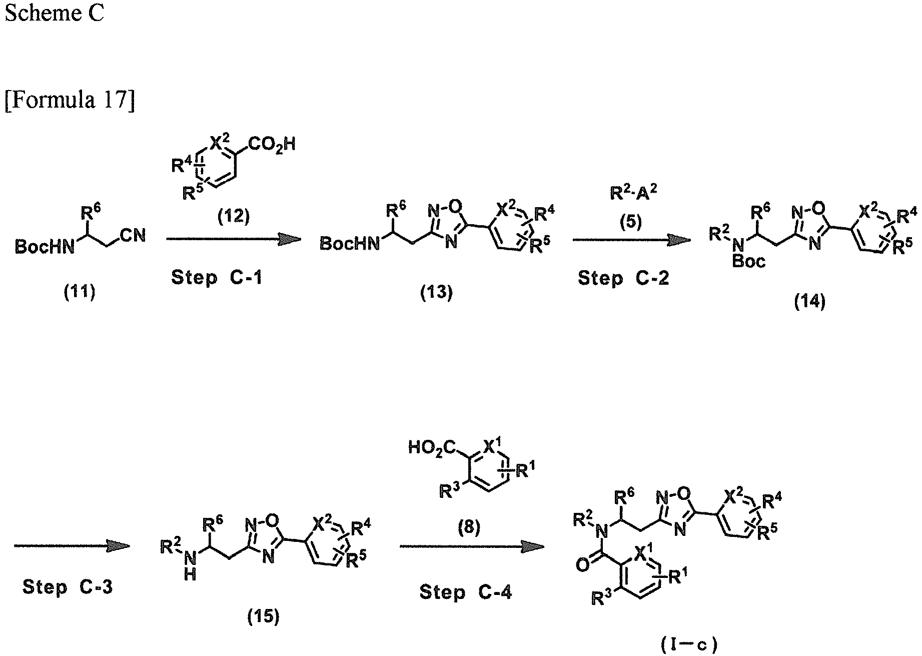
wherein the symbols are as defined above.

The compound of the present invention represented by the formula (I-c) can be produced by the method shown in Scheme C.
Step C-1: The compound (13) can be obtained by the amidoximation reaction of the compound (11), followed by the oxadiazole cyclization reaction. The reaction in Step C-1 can be carried out under the conditions in which the cyano compound (11) is first amidoximated by being treated with hydroxyl amine or hydrochloride thereof in an alcohol solvent such as methanol or ethanol, and subsequently reacted with the carboxylic acid compound (12) and a dehydration condensation agent such as 1-ethyl-3-(3-dimethylaminopropyl)carbodiimide-hydrochloride, dicyclohexylcarbodiimide or carbonyldiimidazole in an ether solvent such as tetrahydrofuran or 1,4-dioxane, an aprotic polar solvent such as N,N-dimethylformamide, a halogen solvent such as dichloromethane or chloroform, an aromatic hydrocarbon solvent such as toluene, ethyl acetate, acetonitrile or a mixed solvent thereof.
Step C-2: The compound (14) can be obtained by the alkylation reaction of the compound (13). The reaction in Step C-2 can be carried out in accordance with the same reaction conditions as in Step A-3.
Step C-3: The compound (15) can be obtained by reacting the compound (14) with an acid. The reaction in Step C-3 can be carried out in accordance with the same reaction conditions as in Step A-4.
Step C-4: The compound (I-c) of the present invention can be obtained by the condensation reaction of the compound (15) and the compound (8). The reaction in Step C-4 can be carried out in accordance with the same reaction conditions as in Step A-5. 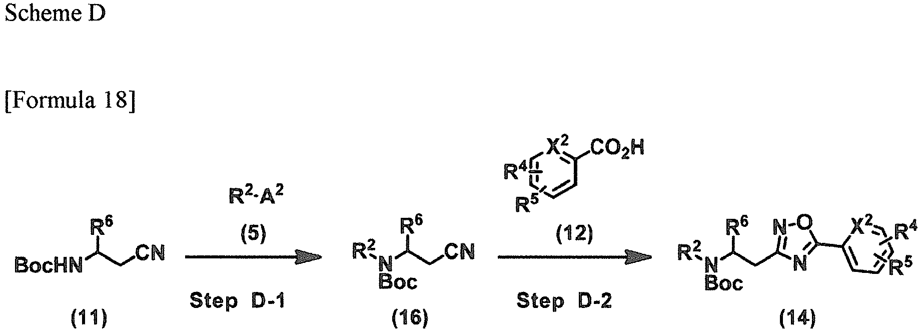
wherein the symbols are as defined above.

The intermediate (14) can alternatively be produced by the method shown in Scheme D.
Step D-1: The compound (16) can be obtained by the alkylation reaction of the compound (11). The reaction in Step D-1 can be carried out in accordance with the same reaction conditions as in Step A-3.
Step D-2: The compound (14) can be obtained by the amidoximation reaction of the compound (16), followed by the oxadiazole cyclization reaction. The reaction in Step D-2 can be carried out in accordance with the same reaction conditions as in Step C-1. 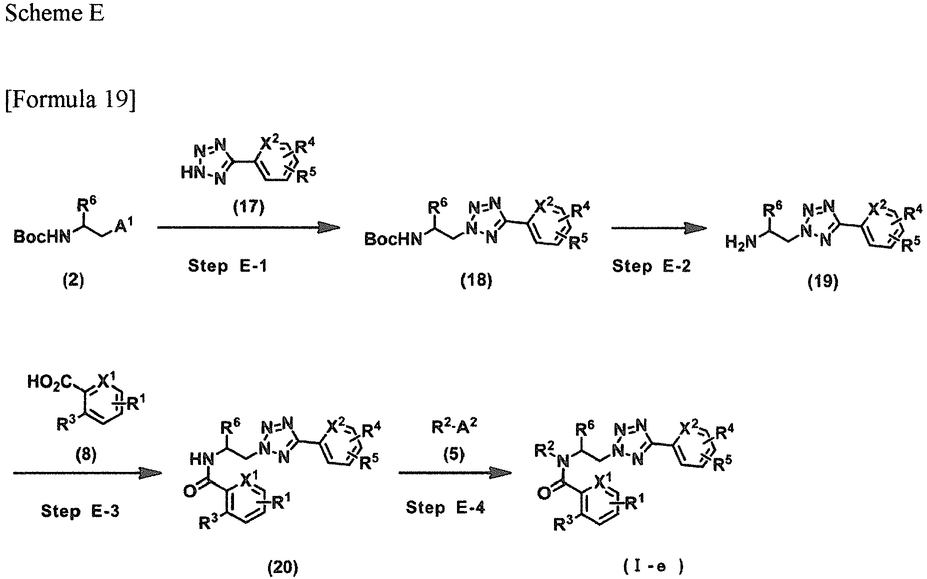
wherein the symbols are as defined above.

The compound of the present invention represented by the formula (I-e) can be produced by the method shown in Scheme E.
Step E-1: The compound (18) can be obtained by reacting the compound (2) and the compound (17). The reactions in Step E-1 can be carried out in accordance with the same reaction conditions as in Step A-2.
Step E-2: The compound (19) can be obtained by reacting the compound (18) with an acid. The reaction in Step E-2 can be carried out in accordance with the same reaction conditions as in Step A-4.
Step E-3: The compound (20) can be obtained by the condensation reaction of the compound (19) and the compound (8). The reaction in Step E-3 can be carried out in accordance with the same reaction conditions as in Step A-5.
Step E-4: The compound (I-e) of the present invention can be obtained by the alkylation reaction of the compound (20). The reaction in Step E-1 can be carried out in accordance with the same reaction conditions as in Step A-3. 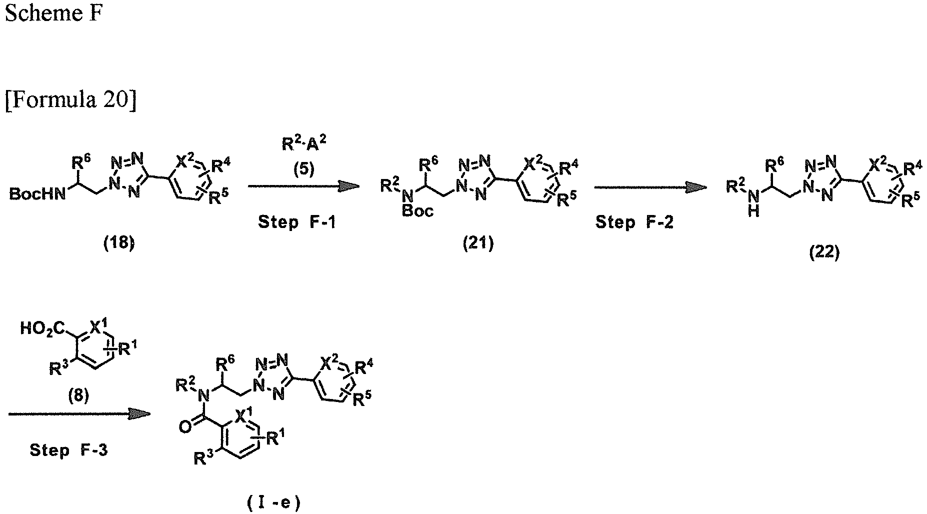
wherein the symbols are as defined above.

The compound of the present invention represented by the formula (I-e) can be produced by the method shown in Scheme F.
Step F-1: The compound (21) can be obtained by the alkylation reaction of the compound (18). The reaction in Step F-1 can be carried out in accordance with the same reaction conditions as in Step A-3.
Step F-2: The compound (22) can be obtained by reacting the compound (21) with an acid. The reaction in Step F-2 can be carried out in accordance with the same reaction conditions as in Step A-4.
Step F-3: The compound (I-e) of the present invention can be obtained by the condensation reaction of the compound (22) and the compound (8). The reaction in Step F-3 can be carried out in accordance with the same reaction conditions as in Step A-5. 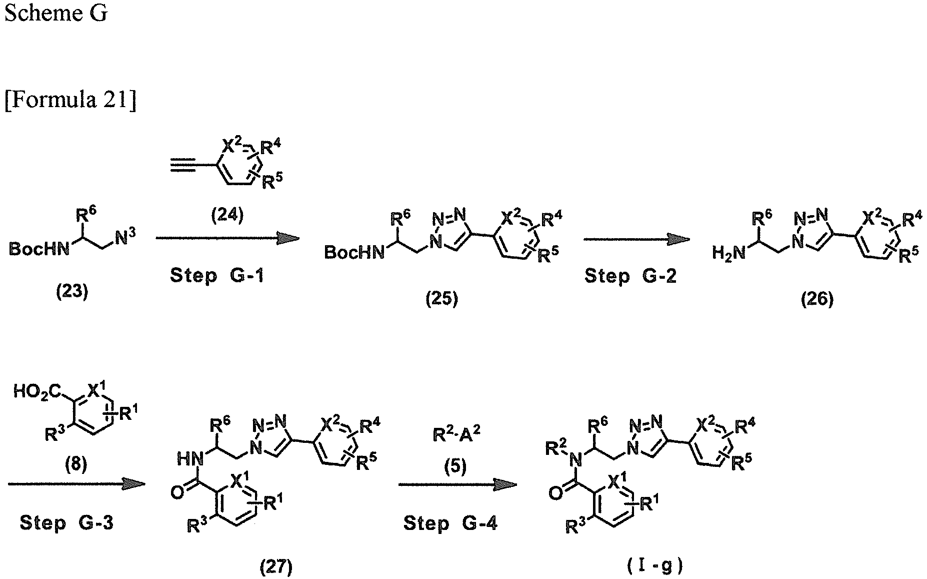
wherein the symbols are as defined above.

The compound of the present invention represented by the formula (I-g) can be produced by the method shown in Scheme G.
Step G-1: The compound (25) can be obtained by the 1,3-dipolar cycloaddition reaction of the compound (23) and the compound (24). The reaction in Step G-1 can be carried out by a method in which treatment is performed with a copper catalyst in the presence of a reducing agent. Examples of the reducing agent to be used in the present reaction include (L)-ascorbic acid and sodium ascorbate. Examples of the copper catalyst to be used in the present reaction include copper sulfate, iodination copper, copper bromide and copper chloride. When a copper halide is used as the copper catalyst, a base is required and triethylamine, diisopropylethylamine, or the like, can be used. Examples of the solvent to be used in the present reactions include alcohol solvents such as methanol and ethanol, ether solvents such as tetrahydrofuran and 1,4-dioxane, aprotic polar solvents such as N,N-dimethylformamide, dimethyl sulfoxide and acetonitrile, aromatic hydrocarbon solvents such as toluene, water or mixed solvents thereof. The present reaction can be carried out usually at 0°C to 150°C, preferably 25°C to 100°C.
Step G-2: The compound (26) can be obtained by reacting the compound (25) with an acid. The reaction in Step G-2 can be carried out in accordance with the same reaction conditions as in Step A-4.
Step G-3: The compound (27) can be obtained by the condensation reaction of the compound (26) and the compound (8). The reactions in Step G-3 can be carried out in accordance with the same reaction conditions as in Step A-5.
Step G-4: The compound (I-g) of the present invention can be obtained by the alkylation reaction of the compound (27). The reactions in Step G-4 can be carried out in accordance with the same reaction conditions as in Step A-3. 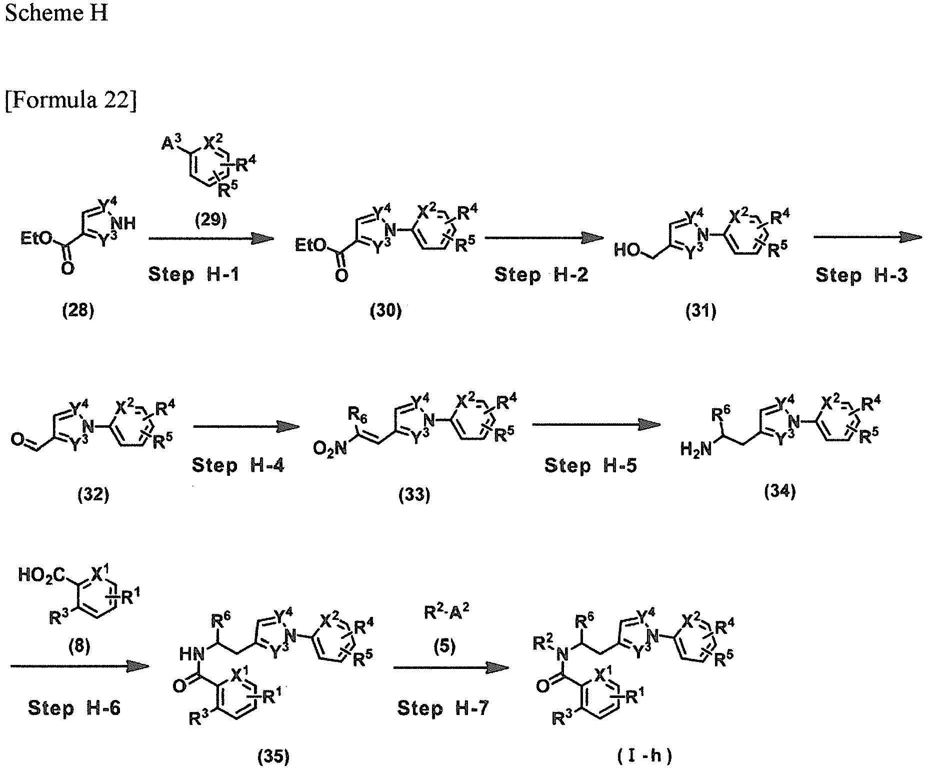
wherein either one of Y³ and Y⁴ represents a nitrogen atom, and the other represents CH, A³ represents a halogen atom, a methanesulfonyloxy group, a p-toluenesulfonyloxy group or a trifluoromethanesulfonyloxy group. Other symbols are as defined above.)

The compound of the present invention represented by the formula (I-h) can be produced by the method shown in Scheme H.
Step H-1: The compound (30) can be obtained by the coupling reaction of the compound (28) and the compound (29). The reaction in Step H-1 can be carried out by a general method in which the nitrogen atom of the azole compound is substituted with an aromatic ring using a catalyst and a ligand in the presence of a base. Examples include the method described in Synlett, 2003, 15, 2428-2439 or a method in accordance therewith. Examples of the catalyst to be used in the present reaction include copper catalyst such as copper (O), copper (I) iodine, copper (I) chloride and copper (I) oxide. Examples of the ligand to be used in the present reaction include N,N'-dimethylethylenediamine, N,N'-dimethylcyclohexane-1,2-diamine, 2-aminopyridine, 1,10-phenanthroline and 2-hydroxybenzaldehyde oxime. Examples of the base to be used in the present reaction include potassium carbonate, potassium phosphate, potassium hydroxide, potassium tert-butoxide, cesium carbonate, sodium carbonate, sodium bicarbonate, sodium acetate, sodium methoxide and tetrabutyl ammonium hydroxide. Examples of the solvent to be used in the present reaction include alcohol solvents such as methanol and ethanol, ether solvents such as tetrahydrofuran and 1,4-dioxane, aprotic polar solvents such as N,N-dimethylformamide, dimethyl sulfoxide and acetonitrile, halogen solvents such as dichloromethane and chloroform, aromatic hydrocarbon solvents such as toluene, water or mixed solvents thereof. The present reaction can be carried out usually at 0°C to 150°C, preferably 25°C to 100°C.
Step H-2: The compound (31) can be obtained by the reduction reaction of the ester of the compound (30). The reaction in Step H-2 can be carried out under the conditions in which the compound (30) is reacted with a reducing agent such as lithium aluminium hydride, diisobutyl aluminium hydride, sodium borohydride or lithium borohydride in an alcohol solvent such as methanol or ethanol, an ether solvent such as tetrahydrofuran or 1,4-dioxane, an aromatic hydrocarbon solvent such as toluene or a mixed solvent thereof. The present reaction can be carried out at -80°C to 150°C, preferably 0°C to 25°C.
Step H-3: The compound (32) can be obtained by the oxidation reaction of the hydroxyl group of the compound (31). The reaction in Step H-3 can be carried out under the conditions in which the compound (31) is reacted with a hypervalent iodine compound such as Dess-Martin reagent or 2-iodoxybenzoic acid, chromate such as pyridinium chlorochromate or pyridinium dichromate, an oxidizing agent such as tetrapropylammonium perruthenate or manganese dioxide in a halogen solvent such as dichloromethane or chloroform, dimethyl sulfoxide or acetonitrile. The present reaction can be carried out at 0°C to 150°C, preferably 25°C to 80°C.
Step H-4: The compound (33) can be obtained by the condensation reaction of the compound (32) and nitroalkane (R⁶-CH₂-NO₂). The reaction in Step H-4 can be carried out under the conditions in which the compound (32) is reacted with nitroalkane (R⁶-CH₂-NO₂) in a halogen solvent such as chloroform, an ether solvent such as tetrahydrofuran or 1,4-dioxane, an alcohol solvent such as methanol or ethanol, or an aprotic solvent such as dimethylformamide, or without a solvent, in the presence of an organic base such as triethylamine, diisopropylethylamine or ethanolamine, or an inorganic base such as sodium hydroxide. The present reactions can be carried out at 0°C to 50°C.
Step H-5: The compound (34) can be obtained by the double bond of the compound (33) and the reduction reaction of the nitro group. The reaction in Step H-5 can be carried out under the conditions in which the compound (33) is reduced in an alcohol solvent such as methanol or ethanol, an ether solvent such as tetrahydrofuran or 1,4-dioxane, ethyl acetate or a mixed solvent thereof, in the presence of a metal catalyst such as palladium or platinum in a hydrogen atmosphere. The present reaction can be carried out at 25°C to 80°C. Alternatively, the reaction can also be carried out in the same reaction conditions as in Step H-2.
Step H-6: The compound (35) can be obtained by the condensation reaction of the compound (34) and the compound (8). The reaction in Step H-6 can be carried out in accordance with the same reaction conditions as in Step A-5.
Step H-7: The compound (I-h) of the present invention can be obtained by the alkylation reaction of the compound (35). The reaction in Step H-7 can be carried out in accordance with the same reaction conditions as in Step A-3. 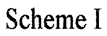 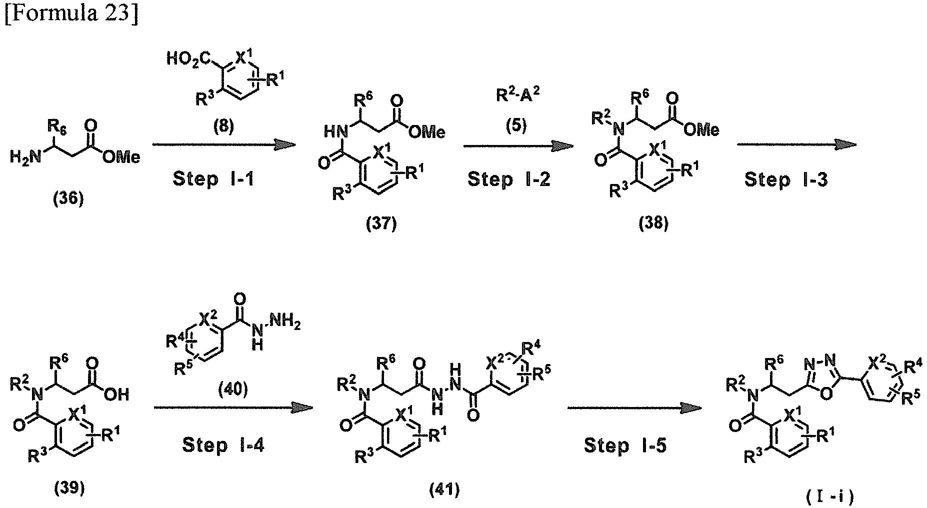
wherein the symbols are as defined above.

The compound of the present invention represented by the formula (I-i) can be produced by the method shown in Scheme I.
Step I-1: The compound (37) can be obtained by the condensation reaction of the compound (36) and the compound (8). The reaction in Step I-1 can be carried out in accordance with the same reaction conditions as in Step A-5.
Step I-2: The compound (38) can be obtained by the alkylation reaction of the compound (37). The reaction in Step I-2 can be carried out in accordance with the same reaction conditions as in Step A-3.
Step I-3: The compound (39) can be obtained by the hydrolysis reaction of the compound (38). The reaction in Step I-3 can be carried out under the reaction conditions described in Protective Groups in Organic Chemistry, written by J. F. W. McOmie and Protective Groups in Organic Synthesis written by T. W. Greene and P. G. M. Wuts. Examples include hydrolysis methods which use a mineral acid such as hydrochloric acid or sulfuric acid, or an inorganic base such as sodium hydroxide or potassium hydroxide. Examples of the solvent to be used in the present reaction include alcohol solvents such as methanol and ethanol, ether solvents such as tetrahydrofuran and 1,4-dioxane, water or mixed solvents thereof. The present reaction can be carried out at 0°C to 100°C.
Step I-4: The compound (41) can be obtained by the condensation reaction of the compound (39) and the compound (40). The reaction in Step I-4 can be carried out in accordance with the same reaction conditions as in Step A-5.
Step I-5: The compound (I-i) of the present invention can be obtained by the cyclodehydration reaction of the compound (41). The reaction in Step I-5 can be carried out under the conditions in which the compound (41) is reacted with a dehydrating agent such as phosphorus oxychloride, sulfuric acid or Burgess reagent in an ether solvent such as tetrahydrofuran or 1,4-dioxane, an aprotic polar solvent such as N,N-dimethylformamide or acetonitrile or a mixed solvent thereof. The present reaction can be carried out at 25°C to 100°C. 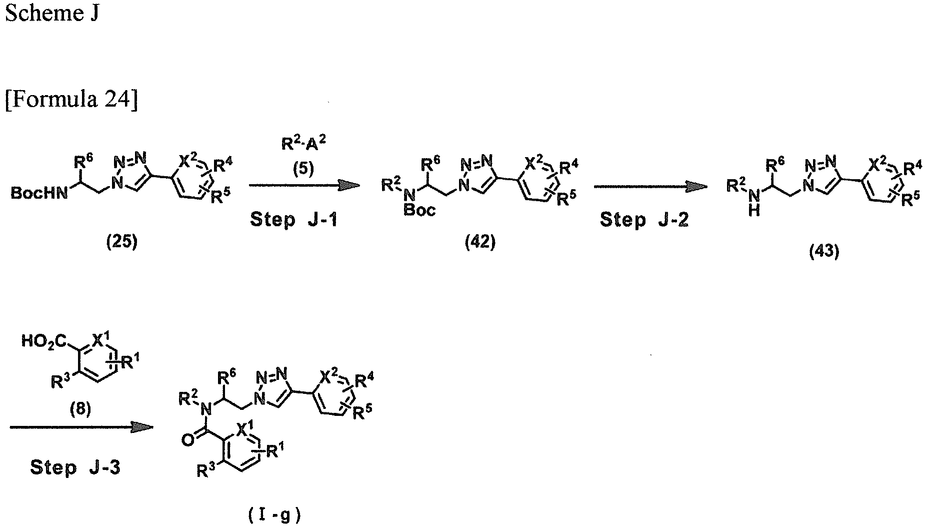
wherein the symbols are as defined above.

The compound of the present invention represented by the formula (I-g) can be produced by the method shown in Scheme J.
Step J-1: The compound (42) can be obtained by the alkylation reaction of the compound (25). The reaction in Step J-1 can be carried out in accordance with the same reaction conditions as in Step A-3.
Step J-2: The compound (43) can be obtained by reacting the compound (42) with an acid. The reaction in Step J-2 can be carried out in accordance with the same reaction conditions as in Step A-4.
Step J-3: The compound (I-g) of the present invention can be obtained by the condensation reaction of the compound (43) and the compound (8). The reaction in Step J-3 can be carried out in accordance with the same reaction condition as in Step A-5. 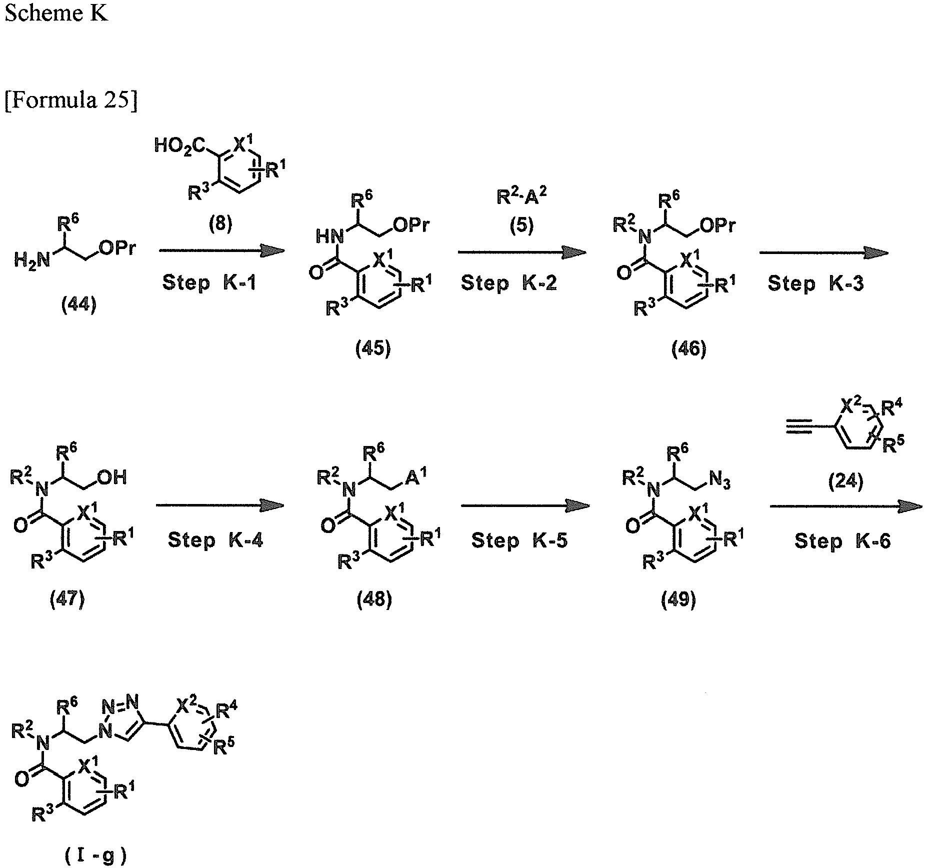
wherein Pr represents a commonly used protective group of a hydroxy group described in Protective Groups in Organic Chemistry, written by J. F. W. McOmie and Protective Groups in Organic Synthesis written by T. W. Greene and P. G. M. Wuts. Other symbols are as defined above.

The compound of the present invention represented by the formula (I-g) can be produced by the method shown in Scheme K.
Step K-1: The compound (45) can be obtained by the condensation reaction of the compound (44) and the compound (8). The reaction in Step K-1 can be carried out in accordance with the same reaction conditions as in Step A-5.
Step K-2: The compound (46) can be obtained by the alkylation reaction of the compound (45). The reaction in Step K-2 can be carried out in accordance with the same reaction conditions as in Step A-3.
Step K-3: The compound (47) can be obtained by removing the protective group of the hydroxy group of the compound (46). The comprehensive overview of the reaction in Step K-3 can be found in Protective Groups in Organic Chemistry, written by J. F. W. McOmie and Protective Groups in Organic Synthesis written by T. W. Greene and P. G. M. Wuts. When Pr of the compound (46) is an acetal protective group such as a methoxymethyl group, the compound (47) can be obtained by reacting the compound (46) with an acid such as hydrochloride.
Step K-4: The compound (48) can be obtained by converting the hydroxy group of the compound (47) to a general leaving group. The reaction in Step K-4 can be carried out in accordance with the same reaction conditions as in Step A-1.
Step K-5: The compound (49) can be obtained by substituting the leaving group of the compound (48) with an azide ion. The reaction in Step K-5 can use an aprotic polar solvent such as N,N-dimethylformamide or dimethyl sulfoxide, water, tetrahydrofuran or a mixed solvent thereof as a solvent. The reaction can be carried out at a reaction temperature of about 0°C to the boiling point of the reaction solvent, with a temperature from 50°C to 80°C being preferable.
Step K-6: The compound (I-g) of the present invention can be obtained by the 1,3-dipolar cycloaddition reaction of the compound (49) and the compound (24). The reaction in Step K-6 can be carried out in accordance with the same reaction conditions as in Step G-1. 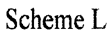 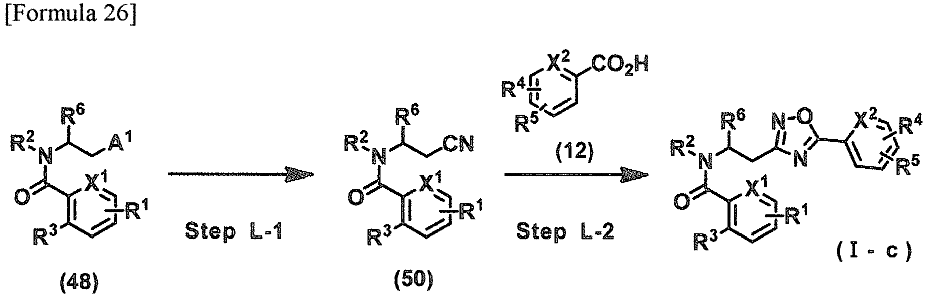
wherein the symbols are as defined above.

The compound of the present invention represented by the formula (I-c) can be produced by the method shown in Scheme L.
Step L-1: The compound (50) can be obtained by substituting the leaving group of the compound (48) with an inorganic cyanide. The reaction in Step L-1 can be carried out by using sodium cyanide or potassium cyanide in an aprotic polar solvent such as N,N-dimethylformamide, an alcohol such as MeOH, water, tetrahydrofuran or a mixed solvent thereof. The reaction is carried out at a reaction temperature of about room temperature to the boiling point of the reaction solvent, with a temperature from 50°C to 100°C being preferable.
Step L-2: The compound (I-c) of the present invention can be obtained by the amidoximation reaction of the compound (50), followed by the oxadiazole cyclization reaction. The reaction in Step L-2 can be carried out in accordance with the same reaction conditions as in Step C-1. 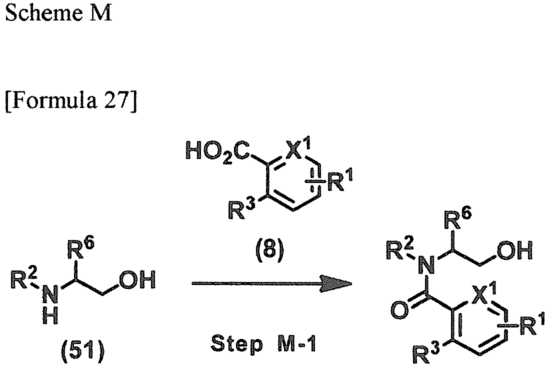
wherein the symbols are as defined above.

The intermediate (47) can alternatively be produced by the method shown in Scheme M.
Step M-1: The compound (47) can be obtained by the condensation reaction of the compound (51) and the compound (8). The reactions in Step M-1 can be carried out in accordance with the same reaction conditions as in Step A-5. 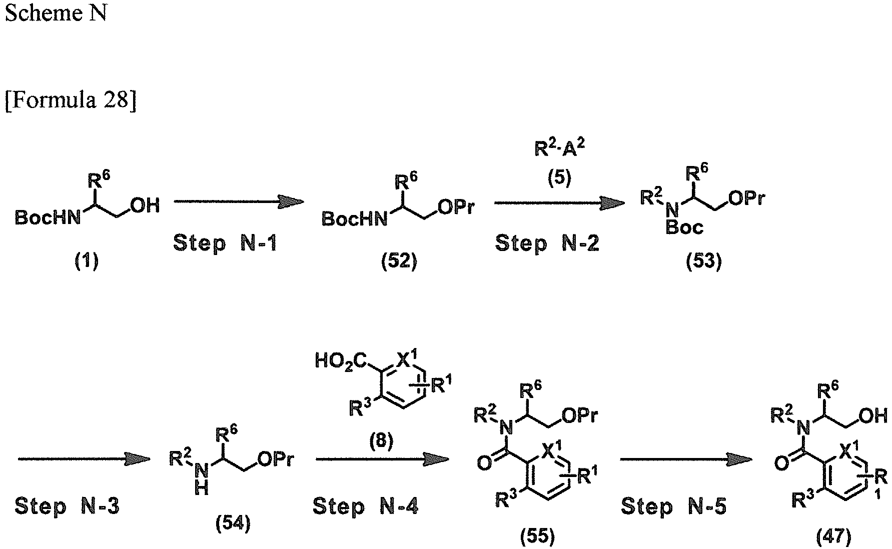
wherein the symbols are as defined above.

The intermediate (47) can alternatively be produced by the method shown in Scheme N.
Step N-1: The compound (52) can be obtained by protecting the hydroxy group of the compound (1). The comprehensive overview of the reaction in Step N-1 can be found in Protective Groups in Organic Chemistry, written by J. F. W. McOmie and Protective Groups in Organic Synthesis written by T. W. Greene and P. G. M. Wuts. For example, when the protective group of the compound (52) is an acyl protective group such as a benzoyl group, the compound (52) can be obtained by reacting an acyl halide such as benzoyl chloride, benzoic anhydride or acetic anhydride with an organic base such as pyridine or triethylamine.
Step N-2: The compound (53) can be obtained by the alkylation reaction of the compound (52). The reaction in Step N-2 can be carried out in accordance with the same reaction conditions as in Step A-3.
Step N-3: The compound (54) can be obtained by reacting the compound (53) with an acid. The reaction in Step N-3 can be carried out in accordance with the same reaction conditions as in Step A-4.
Step N-4: The compound (55) can be obtained by the condensation reaction of the compound (54) and the compound (8). The reaction in Step N-4 can be carried out in accordance with the same reaction conditions as in Step A-5.
Step N-5: The compound (47) can be obtained by removing the protective group of the hydroxy group of the compound (55). The comprehensive overview of the reaction in Step N-5 can be found in Protective Groups in Organic Chemistry, written by J. F. W. McOmie and Protective Groups in Organic Synthesis written by T. W. Greene and P. G. M. Wuts. For example, when Pr of the compound (55) is an acyl protective group such as a benzoyl group, the compound (47) can be obtained by reacting the compound (55) with a base such as potassium hydroxide or sodium hydroxide.

### Examples

Hereinafter, the present invention is further described in details with reference to Reference Examples, Examples and Test Examples, but is not limited thereto, and changes may be made without departing from the scope of the present invention.

In Reference Examples and Examples below, the purification by column chromatography was performed using a Biotage SNAPCartridge KP-Sil for the "KP-Sil", a Biotage SNAPCartridge HP-Sil for the "HP-Sil", a Biotage SNAPCartridge SNAP Ultra for the "SNAP Ultra", a Biotage SNAPCartridge KP-NH for the "KP-NH", a Grace Reveleris Silica Flash Cartridge for the "Grace", and a Grace Reveleris Amino Flash Cartridge for the "Grace NH".

For the aftertreatment operation in the following Reference Examples and Examples, a Biotage ISOLUTE Phase Separator was used for the "ISOLUTE Phase Separator".

In Reference Examples and Examples below, the purification by preparative high performance liquid chromatography (HPLC) was carried out under the following conditions. However, for the case of a compound having a basic functional group and when trifluoroacetic acid is used in the present operation, a neutralization operation, or the like, may sometimes be carried out to obtain a free form.
Device: a Gilson preparative HPLC system
Column: Shiseido Capcelpak C18 MGII 5 µm 20 x 150 mm
Solvent: Liquid A; 0.1% trifluoroacetic acid containing water, Liquid B; 0.1% trifluoroacetic acid containing acetonitrile
Gradient: 0 min. (Liquid A/Liquid B = 90/10), 22 min. (Liquid A/Liquid B = 20/80), 25 min. (Liquid A/Liquid B = 10/90)
Flow rate: 20 mL/min., Detection method: UV 254 nm

In Reference Examples and Examples below, high performance liquid chromatography mass spectrum (HPLC) were measured by either one of the following 2 conditions.
Condition 1
   Measurement Instrument: a MicroMass Platform LC and an Agilent Agilent 1100
   Column: Waters SunFire C18 2.5 µm 4.6 x 50 mm
   Solvent: 0.1% trifluoroacetic acid containing water, Liquid B; 0.1% trifluoroacetic acid containing acetonitrile
Gradient: 0 min. (Liquid A/Liquid B = 90/10), 0.5 min. (Liquid A/Liquid B = 90/10), 5.5 min. (Liquid A/Liquid B = 20/80), 6.0 min. (Liquid A/Liquid B = 1/99), 6.3 min. (Liquid A/Liquid B = 1/99).
   Flow rate: 1 mL/min., Detection method: 254 nm
   Ionization method: Electron Spray method (ESI: Electron Spray Ionization)
Condition 2
   Measurement Instrument: an Agilent Agilent 2900 and Agilent 6150
   Column: Waters Acquity CSH C18 1.7 µm 2.1 x 50 mm
   Solvent: Liquid A; 0.1% formic acid containing water, Liquid B; 0.1% formic acid containing acetonitrile
   Gradient: 0 min. (Liquid A/Liquid B = 80/20), 1.2 to 1.4 min. (Liquid A/Liquid B = 1/99) Flow rate: 0.8 mL/min, Detection method: UV 254 nm
   Ionization method: Electron Spray method (ESI: Electron Spray Ionization)

In Reference Examples and Examples below, the mass spectrum (MS) was measured under the following conditions.
MS Measurement Instrument: Shimadzu LCMS-2010EV or MicroMass Platform LC
In Reference Examples and Examples below, the compounds were named in accordance with ACD/Name (ACD/Labs 12.01, Advanced Chemistry Development Inc.).

In Reference Examples and Examples, the following terms and reagents are shown as follows.
MgSO₄ (magnesium sulfate), Na₂SO₄ (anhydrous sodium sulfate), Na₂CO₃ (sodium carbonate), Cs₂CO₃ (cesium carbonate), KOH (potassium hydroxide), NaHCO₃ (sodium bicarbonate), NaOH (sodium hydroxide), NH₄Cl (ammonium chloride), DMF (N,N-dimethylformamide), EtOAc (ethyl acetate), CHCl₃ (chloroform), THF (tetrahydrofuran), Et₂O (diethyl ether), MeOH (methanol), EtOH (ethanol), H₂O (water), HATU [O-(7-azabenzotriazol-1-yl)-N,N,N',N'-tetramethyluronium hexafluorophosphate], Pd(PPh₃)₄ [tetrakistriphenylphosphinepalladium (O)], Pd/C (palladium on carbon), brine (saturated saline), Boc (tert-butoxycarbonyl), Ms (methanesulfonyl), DIPEA (N,N-diisopropylethylamine), MeI (methyl iodide), EtI (ethyl iodide), CuI (copper (I) iodide), NaH (sodium hydride), LAH (LiAlH₄, lithium aluminium hydride), NaBH₄ (sodium borohydride), HCl (hydrogen chloride).

### Reference Example 1: 5-Fluoro-2-[1-(tetrahydro-2H-pyran-2-yl)-1H-pyrazol-5-yl]pyridine

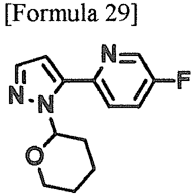

To a mixed solution of 1-(tetrahydro-2H-pyran-2-yl)-5-(4,4,5,5-tetramethyl-1,3,2-dioxaborolan-2-yl)-1H-pyrazole (100.5 g, 361.3 mmol), 2-bromo-5-fluoropyridine (56.5 g, 328.5 mmol), and Pd(PPh₃)₄ (37.96 g, 32.85 mmol) in ethanol (300 mL) and toluene (300 mL), a 2M aqueous solution of Na₂CO₃ (492.7 mL, 985.4 mmol) was added, and the resulting mixture was heated to reflux for 2 hours. The reaction mixture was allowed to cool to room temperature, then water and EtOAc were added thereto, and the resulting mixture was stirred at room temperature for 30 minutes, followed by extraction with EtOAc. The organic layer was washed with brine and dried over MgSO₄, then the drying agent was filtered off, then NH silica gel (400 g) was added to the resulting organic layer, and the resulting mixture was stirred for 15 hours. The mixture was filtered through acid silica gel (eluted with n-hexane:EtOAc = 1:1 → EtOAc) and the solvent was distilled off under reduced pressure to obtain the title compound (100 g) (pale yellow oil).
MS (ESI pos.) m/z: 248 [M+H]+]

### Reference Example 2: 5-Fluoro-2-(1H-pyrazol-3-yl)pyridine

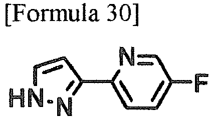

To a solution of 5-fluoro-2-[1-(tetrahydro-2H-pyran-2-yl)-1H-pyrazol-5-yl]pyridine obtained in Reference Example 1 (81.2 g, 328.5 mmol) in methanol (250 mL), a 4M solution of HCl-EtOAc (246.3 mL, 985.4 mmol) was added, and the resulting mixture was stirred at room temperature for 16 hours. The solvent was distilled off under reduced pressure, then EtOAc (500 mL) was added to the residue, and the resulting mixture was heated to reflux for 1 hour. The reaction mixture was allowed to cool to room temperature, then ice-cooled, and then the precipitate was filtered out and dried to obtain a hydrochloride (colorless solid) of the title compound. Water (700 mL) and EtOAc (350 mL) were added to the obtained hydrochloride, and the resulting mixture was stirred for 30 minutes and then separated. The obtained organic layer was extracted with 1.2M hydrochloric acid (100 mL) three times. The aqueous layers were combined and the pH was adjusted to 12 with an 8M aqueous solution of NaOH, and then the organic layer was extracted with chloroform. The extracted organic layer was passed through an ISOLUTE Phase Separator, and the solvent was distilled off under reduced pressure. Diisopropyl ether (300 mL) was added to the obtained residue, and the resulting mixture was heated to reflux for 2 hours. The reaction mixture was allowed to cool to room temperature, then ice-cooled, and then the precipitate was filtered out and dried to obtain the title compound (44.9 g) (pale pink solid).
MS (ESI pos.) m/z: 164 [M+H]+

### Reference Example 3: 5-Fluoro-2-(1H-pyrazol-4-yl)pyridine

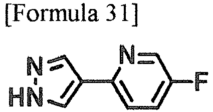

To a solution of tert-butyl 4-(4,4,5,5-tetramethyl-1,3,2-dioxaborolan-2-yl)-1H-pyrazole-1-carboxylate (15.4 g, 52.5 mmol) and 2-bromo-5-fluoropyridine (8.40 g, 47.7 mmol) in 1,4-dioxane (100 mL), Pd(PPh₃)₄ (5.52 g, 4.77 mmol) and a 2M aqueous solution of Na₂CO₃ (71.6 mL, 143.2 mmol) were added, then the resulting mixture was stirred at 100°C for 3 hours and then at room temperature for 72 hours. Water was added to the reaction mixture, followed by extraction with EtOAc. The organic layer was washed with brine, dried over MgSO₄, then the drying agent was filtered off, and then the solvent was distilled off under reduced pressure. A small amount of EtOAc was added to the obtained residue and the resulting mixture was filtered out and dried to obtain the title compound (4.9 g) (colorless solid).
MS (ESI pos.) m/z: 164 [M+H]+

### Reference Example 4: 4-(3,4-Difluorophenyl)-1H-pyrazole

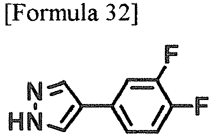

To a mixed solution of tert-butyl 4-(4,4,5,5-tetramethyl-1,3,2-dioxaborolan-2-yl-1H-pyrazole-1-carboxylate (2.0 g, 6.80 mmol) and 4-bromo-1,2-difluorobenzene (1.19 g, 6.18 mmol) in ethanol (10 mL) and toluene (10 mL), Pd(PPh₃)₄ (0.71 g, 0.62 mmol) and a 2M aqueous solution of Na₂CO₃ (9.26 mL, 18.51 mmol) were added, and the resulting mixture was stirred at 100°C for 30 minutes and then at room temperature for 16 hours. Water was added to the reaction mixture, followed by extraction with EtOAc. The organic layer was washed with brine, dried over MgSO₄, then the drying agent was filtered off, and then the solvent was distilled off under reduced pressure. The obtained residue was purified by column chromatography (SNAP Ultra 100 g, hexane/EtOAc = 88/12 to 0/100) to obtain the title compound (0.55 g) (pale yellow solid).
MS (ESI neg.) m/z: 179 [M-H]-

### Reference Example 5: (2S)-2-[(tert-Butoxycarbonyl)amino]propyl methanesulfonate

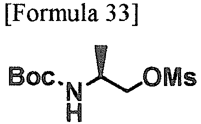

To a solution of tert-butyl [(2S)-1-hydroxypropan-2-yl]carbamate (1.5 g, 8.6 mmol) in chloroform (20 mL), triethylamine (1.78 mL, 12.8 mmol) and methanesulfonyl chloride (0.80 mL, 10.3 mmol) were added under ice-cooling, and the resulting mixture was stirred for 1 hour. Water was added to the reaction mixture, followed by extraction with chloroform. The organic layer was washed with brine, and then was passed through an ISOLUTE Phase Separator. The solvent was distilled off under reduced pressure to obtain the title compound (2.1 g) (yellow solid).
MS (ESI pos.) m/z: 276 [M+Na]+

### Reference Example 6: tert-Butyl {(2S)-1-[3-(5-fluoropyridin-2-yl)-1H-pyrazol-1-yl]propan-2-yl}carbamate

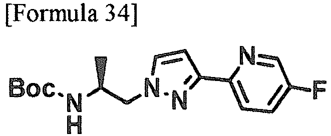

To a solution of 5-fluoro-2-(1H-pyrazol-3-yl)pyridine obtained in Reference Example 2 (0.30 g, 1.6 mmol) in DMF (10 mL), Cs₂CO₃ (1.20 g, 7.36 mmol) and a solution of (2S)-2-[(tert-butoxycarbonyl)amino]propyl methanesulfonate obtained in Reference Example 5 (0.93 g, 3.68 mmol) in DMF were added, and the resulting mixture was heated to 90°C and stirred for 24 hours. The reaction mixture was allowed to cool, then water was added thereto, followed by extraction with EtOAc. The organic layer was washed with water and brine, dried over MgSO₄, then the drying agent was filtered off, and then the solvent was distilled off under reduced pressure. The obtained residue was purified by column chromatography (KP-NH 55g, hexane/EtOAc = 90/10 to 50/50) to obtain the title compound (0.2 g) (colorless solid).
MS (ESI pos.) m/z: 321 [M+H]+

### Reference Example 7: tert-Butyl ethyl {(2S)-1-[3-(5-fluoropyridin-2-yl)-1H-pyrazol-1-yl]propan-2-yl}carbamate

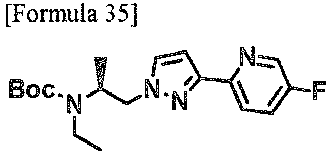

To a solution of tert-butyl {(2S)-1-[3-(5-fluoropyridin-2-yl)-1H-pyrazol-1-yl]propan-2-yl}carbamate obtained in Reference Example 6 (0.20 g, 0.62 mmol) in DMF (5 mL), 60% NaH (0.037 g, 0.94 mmol) was added, the resulting mixture was stirred for 30 minutes, then EtI (0.06 mL, 0.75 mmol) was added thereto, and the resulting mixture was stirred for 16 hours. Water was added to the reaction mixture, followed by extraction with EtOAc. The organic layer was washed with water and brine, dried over MgSO₄, then the drying agent was filtered off. The solvent was distilled off under reduced pressure to obtain the title compound (0.25 g) (yellow oil).
MS (ESI pos.) m/z: 349 [M+H]+

### Reference Example 8: (2S)-N-Ethyl-1-[3-(5-fluoropyridin-2-yl)-1H-pyrazol-1-yl]propan-2-amine dihydrochloride

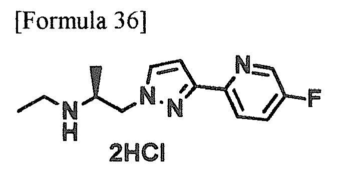

To a solution oftert-butyl ethyl {(2S)-1-[3-(5-fluoropyridin-2-yl)-1H-pyrazol-1-yl]propan-2-yl}carbamate obtained in Reference Example 7 (0.22 g, 0.63 mmol) in chloroform (5 mL), a 4M solution of HCl-EtOAc (3.0 mL, 12.0 mmol) was added, and the resulting mixture was stirred at room temperature for 24 hours. The reaction mixture was concentrated under reduced pressure to obtain the title compound (0.14 g) (yellow solid).
MS (ESI pos.) m/z: 249 [M+H]+

### Reference Example 9: (2S)-1-[3-(5-Fluoropyridin-2-yl)-1H-pyrazol-1-yl]propan-2-amine dihydrochloride

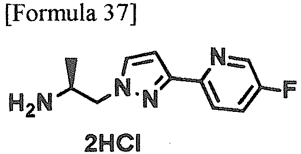

By using tert-butyl {(2S)-1-[3-(5-fluoropyridin-2-yl)-1H-pyrazol-1-yl]propan-2-yl}carbamate obtained in Reference Example 6 (1.5 g, 4.68 mmol) as the raw material, the same procedure as in Reference Example 8 was carried out to obtain the title compound (1.2 g) (colorless and amorphous).
MS (ESI pos.) m/z: 221 [M+H]+

### Reference Example: 10 N-{(2S)-1-[3-(5-Fluoropyridin-2-yl)-1H-pyrazol-1-yl]propan-2-yl}-2-(2H-1,2,3-triazol-2-yl)benzamide

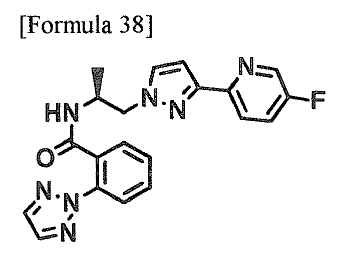

To a solution of (2S)-1-[3-(5-fluoropyridin-2-yl)-1H-pyrazol-1-yl]propan-2-amine dihydrochloride obtained in Reference Example 9 (0.36 g, 1.23 mmol) and 2-(2H-1,2,3-triazol-2-yl)benzoic acid (0.28 g, 1.47 mmol) in DMF (5 mL), HATU (0.707 g, 1.84 mmol) and DIPEA (1.93 mL, 11.1 mmol) were added, and the resulting mixture was stirred for 24 hours. Water was added to the reaction mixture, followed by extraction with EtOAc. The organic layer was washed with water and brine, dried over MgSO₄, then the drying agent was filtered off, and then the solvent was distilled off under reduced pressure. The obtained residue was purified by column chromatography (KP-Sil 50 g, CHCl₃/MeOH = 100/0 to 90/10) to obtain the title compound (0.16 g) (colorless solid).
MS (ESI pos.) m/z: 392 [M+H]+

### Reference Example 11: N-{(2S)-1-[3-(5-Fluoropyridin-2-yl)-1H-pyrazol-1-yl]propan-2-yl}-5-methyl-2-(2H-1,2,3-triazol-2-yl)benzamide

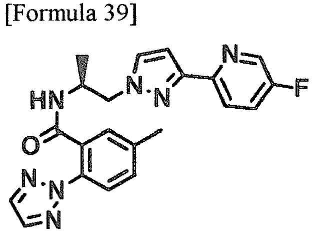

By using (2S)-1-[3-(5-fluoropyridin-2-yl)-1H-pyrazol-1-yl]propan-2-amine dihydrochloride obtained in Reference Example 9 (0.18 g, 0.61 mmol) and 5-methyl-2-(2H-1,2,3-triazol-2-yl)benzoic acid (0.15 g, 0.74 mmol) as the raw materials, the same procedure as in Reference Example 10 was carried out to obtain the title compound (0.13 g) (colorless solid).
MS (ESI pos.) m/z: 406 [M+H]+

### Reference Example 12: tert-Butyl {(2S)-1-[4-(5-fluoropyridin-2-yl)-1H-pyrazol-1-yl]propan-2-yl}carbamate

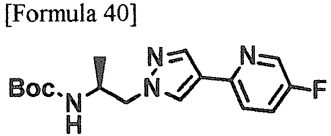

By using (2S)-2-[(tert-butoxycarbonyl)amino]propyl methane sulfonate obtained in Reference Example 5 (3.26 g, 12.87 mmol) and 5-fluoro-2-(1H-pyrazole-4-yl)pyridine (1.40 g, 8.58 mmol) obtained in Reference Example 3 as the raw materials, the same procedure as in Reference Example 6 was carried out to obtain the title compound (2.70 g) (pale yellow oil).
MS (ESI pos.) m/z: 321 [M+H]+

### Reference Example 13: (2S)-1-[4-(5-Fluoropyridin-2-yl)-1H-pyrazol-1-yl]propan-2-amine dihydrochloride

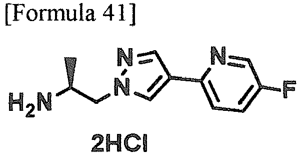

By using tert-butyl {(2S)-1-[4-(5-fluoropyridin-2-yl)-1H-pyrazol-1-yl]propan-2-yl}carbamate obtained in Reference Example 12 (2.70 g, 8.43 mmol) as the raw material, the same procedure as in Reference Example 8 was carried out to obtain the title compound (1.6 g) (colorless solid).
MS (ESI pos.) m/z: 221 [M+H]+

### Reference Example 14: N-{(2S)-1-[4-(5-Fluoropyridin-2-yl)-1H-pyrazol-1-yl]propan-2-yl}-2-(2H-1,2,3-triazol-2-yl)benzamide

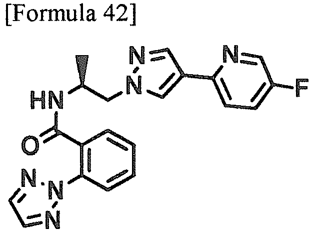

By using (2S)-1-[4-(5-fluoropyridin-2-yl)-1H-pyrazol-1-yl]propan-2-amine dihydrochloride obtained in Reference Example 13 (0.30 g, 1.07 mmol) and 2-(2H-1,2,3-triazol-2-yl)benzoic acid (0.22 g, 1.18 mmol) as the raw materials, the same procedure as in Reference Example 10 was carried out to obtain the title compound (0.24 g) (colorless and amorphous).
MS (ESI pos.) m/z: 392 [M+H]+

### Reference Example 15: N-{(2S)-1-[4-(5-Fluoropyridin-2-yl)-1H-pyrazol-1-yl]propan-2-yl}-5-methyl-2-(2H-1,2,3-triazol-2-yl)benzamide

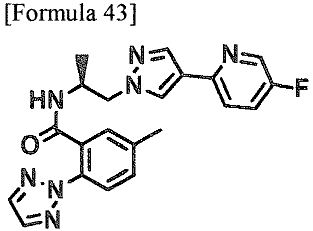

By using (2S)-1-[4-(5-fluoropyridin-2-yl)-1H-pyrazol-1-yl]propan-2-amine dihydrochloride obtained in Reference Example 13 (0.30 g, 1.07 mmol) and 5-methyl-2-(2H-1,2,3-triazol-2-yl)benzoic acid (0.24 g, 1.18 mmol) as the raw materials, the same procedure as in Reference Example 10 was carried out to obtain the title compound (0.28 g) (colorless solid).
MS (ESI pos.) m/z: 406 [M+H]+

### Reference Example 16: tert-Butyl {(2R)-1-[3-(5-fluoropyridin-2-yl)-1H-pyrazol-1-yl]propan-2-yl}carbamate

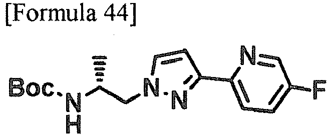

To a solution of tert-butyl [(2R)-1-hydroxypropan-2-yl]carbamate (4.3 g, 24.5 mmol) in chloroform (50 mL), triethylamine (6.16 mL, 44.2 mmol) and methanesulfonyl chloride (2.28 mL, 29.5 mmol) were added under ice-cooling, and the resulting mixture was stirred for 1 hour. Water was added to the reaction mixture, followed by extraction with chloroform. The organic layer was washed with brine, then passed through an ISOLUTE Phase Separator, and the solvent was distilled off under reduced pressure. A solution of the obtained residue in DMF and Cs₂CO₃ (7.79 g, 23.9 mmol) were added to a solution of 5-fluoro-2-(1H-pyrazol-3-yl)pyridine obtained in Reference Example 2 (1.30 g, 7.97 mmol) in DMF (50 mL), then the resulting mixture was stirred at 90°C for 2 hours. The reaction mixture was allowed to cool to room temperature, then water was added thereto, followed by extraction with EtOAc. The organic layer was washed with water and brine, dried over MgSO₄, then the drying agent was filtered off, and then the solvent was distilled off under reduced pressure. The obtained residue was purified by column chromatography (HP-Sil 50 g, hexane/EtOAc = 90/10 to 50/50) to obtain the title compound (0.30 g) (colorless solid).
MS (ESI pos.) m/z: 321 [M+H]+

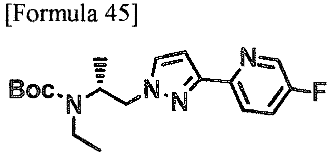

### Reference Example 17: tert-Butyl ethyl{(2R)-1-[3-(5-Fluoropyridin-2-yl)-1H-pyrazol-1-yl]propan-2-yl}carbamate

By using tert-butyl {(2R)-1-[3-(5-fluoropyridin-2-yl)-1H-pyrazol-1-yl]propan-2-yl}carbamate obtained in Reference Example 16 (0.30 g, 0.94 mmol) as the raw material, the same procedure as in Reference Example 7 was carried out to obtain the title compound (0.26 g) (pale yellow oil).
MS (ESI pos.) m/z: 349 [M+H]+

### Reference Example 18: (2R)-N-Ethyl-1-[3-(5-fluoropyridin-2-yl)-1H-pyrazol-1-yl]propan-2-amine dihydrochloride

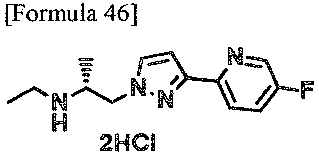

By using tert-butyl ethyl{(2R)-1-[3-(5-fluoropyridin-2-yl)-1H-pyrazol-1-yl]propan-2-yl}carbamate obtained in Reference Example 17 (0.26 g, 0.75 mmol) as the raw material, the same procedure as in Reference Example 8 was carried out to obtain the title compound (0.17 g) (colorless solid).
MS (ESI pos.) m/z: 249 [M+H]+

### Reference Example 19: tert-Butyl {(2S)-1-[3-(5-fluoropyridin-2-yl)-1H-pyrazol-1-yl}-3-methylbutan-2-yl]carbamate

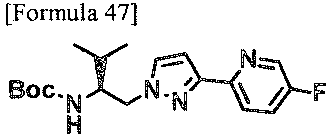

By using tert-butyl [(2S)-1-hydroxy-3-methylbutan-2-yl]carbamate (0.5 g, 2.46 mmol) as the raw material, the same procedure as in Reference Example 16 was carried out to obtain the title compound (0.09 g) (colorless solid).
MS (ESI pos.) m/z: 349 [M+H]+

### Reference Example 20: (2S)-1-[3-(5-Fluoropyridin-2-yl)-1H-pyrazol-1-yl]-3-methylbutan-2-amine dihydrochloride

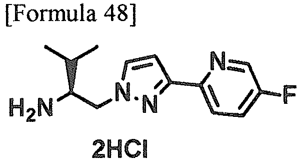

By using tert-butyl {(2S)-1-[3-(5-fluoropyridin-2-yl)-1H-pyrazol-1-yI}-3-methylbutan-2-yl]carbamate obtained in Reference Example 19 (0.09 g, 0.26 mmol) as the raw material, the same procedure as in Reference Example 8 was carried out to obtain the title compound (0.075 g) (colorless oil).
MS (ESI pos.) m/z: 249 [M+H]+

### Reference Example 21 N-{(2S)-1-[3-(5-Fluoropyridin-2-yl)-1H-pyrazol-1-yl]-3-methylbutan-2-yl}-5-methyl-2-(2H-1,2,3-triazol-2-yl)benzamide

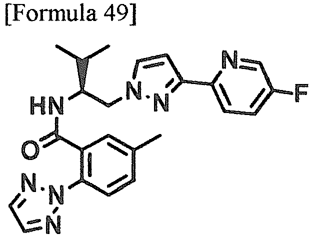

By using (2S)-1-[3-(5-fluoropyridin-2-yl)-1H-pyrazol-1-yl]-3-methylbutan-2-amine dihydrochloride obtained in Reference Example 20 (0.075 g, 0.23 mmol) and 5-methyl-2-(2H-1,2,3-triazol-2-yl)benzoic acid (0.057 g, 0.28 mmol) as the raw materials, the same procedure as in Reference Example 10 was carried out to obtain the title compound (0.05 g) (colorless solid).
MS (ESI pos.) m/z: 434 [M+H]+

### Reference Example 22: tert-Butyl {(2S)-1-[3-(5-fluoropyridin-2-yl)-1H-pyrazol-1-yl]butan-2-yl}carbamate

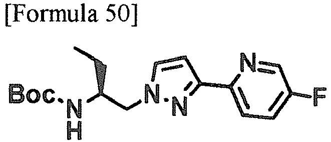

By using tert-butyl [(2S)-1-hydroxybutan-2-yl]carbamate (0.50 g, 2.64 mmol) as the raw material, the same procedure as in Reference Example 16 was carried out to obtain the title compound (0.14 g) (colorless solid).
MS (ESI pos.) m/z: 335 [M+H]+

### Reference Example 23: (2S)-1-[3-(5-Fluoropyridin-2-yl)-1H-pyrazol-1-yl]butan-2-amine dihydrochloride

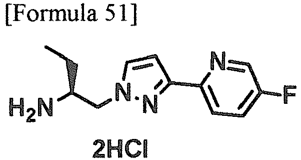

By using tert-butyl {(2S)-1-[3-(5-fluoropyridin-2-yl)-1H-pyrazol-1-yl]butan-2-yl}carbamate obtained in Reference Example 22 (0.14 g, 0.42 mmol) as the raw material, the same procedure as in Reference Example 8 was carried out to obtain the title compound (0.06 g) (colorless oil).
MS (ESI pos.) m/z: 235 [M+H]+

### Reference Example 24: N-{(2S)-1-[3-(5-Fluoropyridin-2-yl)-1H-pyrazol-1-yl]butan-2-yl}-5-methyl-2-(2H-1,2,3-triazol-2-yl)benzamide

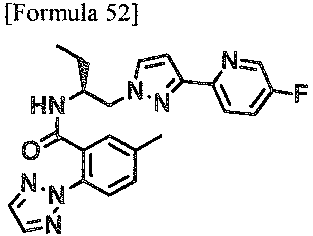

By using (2S)-1-[3-(5-fluoropyridin-2-yl)-1H-pyrazol-1-yl]butan-2-amine dihydrochloride obtained in Reference Example 23 (0.06 g, 0.20 mmol) and 5-methyl-2-(2H-1,2,3-triazol-2-yl)benzoic acid (0.048 g, 0.23 mmol) as the raw materials, the same procedure as in Reference Example 10 was carried out to obtain the title compound (0.034 g) (colorless solid). MS (ESI pos.) m/z: 420 [M+H]+

### Reference Example 25: tert-Butyl {(2S)-1-[3-(4-fluorophenyl)-1H-pyrazol-1-yl]propan-2-yl}carbamate

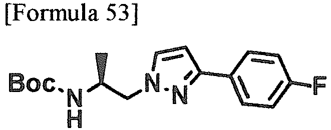

By using (2S)-2-[(tert-butoxycarbonyl)amino]propyl methane sulfonate obtained in Reference Example 5 (3.62 g, 14.3 mmol) and 3-(4-fluorophenyl)-1H-pyrazole (1.17 g, 7.2 mmol) as the raw materials, the same procedure as in Reference Example 6 was carried out to obtain the title compound (0.67 g) (colorless solid).
MS (ESI pos.) m/z: 320 [M+H]+

### Reference Example 26: tert-Butyl ethyl{(2S)-1-[3-(4-fluorophenyl)-1H-pyrazol-1-yl]propan-2-yl}carbamate

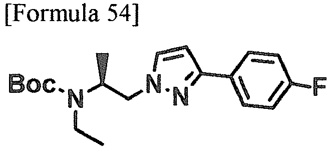

By using tert-butyl {(2S)-1-[3-(4-Fluorophenyl)-1H-pyrazol-1-yl]propan-2-yl}carbamate obtained in Reference Example 25 (0.67 g, 2.10 mmol) as the raw material, the same procedure as in Reference Example 7 was carried out to obtain the title compound (0.35 g) (colorless oil).
MS (ESI pos.) m/z: 348 [M+H]+

### Reference Example 27: (2S)-N-Ethyl-1-[3-(4-fluorophenyl)-1H-pyrazol-1-yl]propan-2-amine hydrochloride

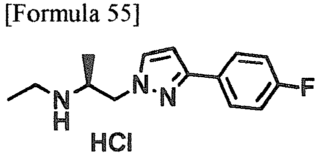

By using tert-butyl ethyl{(2S)-1-[3-(4-fluorophenyl)-1H-pyrazol-1-yl]propan-2-yl}carbamate obtained in Reference Example 26 (0.35 g, 1.01 mmol) as the raw material, the same procedure as in Reference Example 8 was carried out to obtain the title compound (0.26 g) (colorless solid).
MS (ESI pos.) m/z: 248 [M+H]+

### Reference Example 28: tert-Butyl {(2S)-1-[3-(5-fluoropyridin-2-yl)-1H-pyrazol-1-yl]propan-2-yl}methyl carbamate

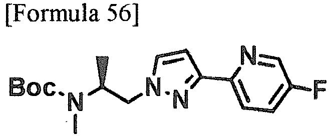

By using tert-butyl {(2S)-1-[3-(5-fluoropyridin-2-yl)-1H-pyrazol-1-yl]propan-2-yl}carbamate obtained in Reference Example 6 (0.63 g, 1.97 mmol) as the raw material, the same procedure as in Reference Example 7 was carried out by using MeI (0.19 mL, 2.94 mmol) to obtain the title compound (0.74 g) (pale yellow oil).
MS (ESI pos.) m/z: 335 [M+H]+

### Reference Example 29: (2S)-1-[3-(5-Fluoropyridin-2-yl)-1H-pyrazol-1-yl]-N-methylpropan-2-amine dihydrochloride

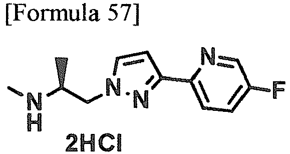

By using tert-butyl {(2S)-1-[3-(5-fluoropyridin-2-yl)-1H-pyrazol-1-yl]propan-2-yl}methyl carbamate obtained in Reference Example 28 (0.66 g, 1.97 mmol) as the raw material, the same procedure as in Reference Example 8 was carried out to obtain the title compound (0.46 g) (yellow oil).
MS (ESI pos.) m/z: 235 [M+H]+

### Reference Example 30: tert-Butyl {(2R)-1-[3-(4-fluorophenyl)-1H-pyrazol-1-yl]propan-2-yl}carbamate

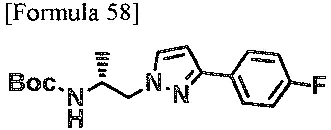

By using tert-butyl [(2R)-1-hydroxypropan-2-yl]carbamate (4.3 g, 24.5 mmol) as the raw material, the same procedure as in Reference Example 16 was carried out to obtain the title compound (1.0 g) (colorless solid).
MS (ESI pos.) m/z: 320 [M+H]+

### Reference Example 31: tert-Butyl ethyl{(2R)-1-[3-(4-fluorophenyl)-1H-pyrazol-1-yl)propan-2-yl}carbamate

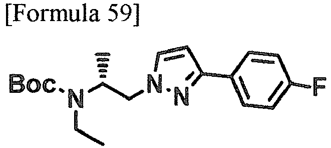

By using tert-butyl {(2R)-1-[3-(4-fluorophenyl)-1H-pyrazol-1-yl]propan-2-yl}carbamate obtained in Reference Example 30 (1.0 g, 3.13 mmol) as the raw material, the same procedure as in Reference Example 7 was carried out to obtain the title compound (0.65 g) (colorless oil).
MS (ESI pos.) m/z: 348 [M+H]+

### Reference Example 32: (2R)-N-Ethyl-1-[3-(4-fluorophenyl)-1H-pyrazol-1-yl]propan-2-amine hydrochloride

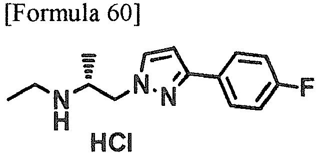

By using tert-butyl ethyl{(2R)-1-[3-(4-fluorophenyl)-1H-pyrazol-1-yl]propan-2-yl}carbamate obtained in Reference Example 31 (0.65 g, 1.87 mmol) as the raw material, the same procedure as in Reference Example 8 was carried out to obtain the title compound (0.5 g) (colorless solid).
MS (ESI pos.) m/z: 248 [M+H]+

### Reference Example 33: tert-Butyl {(2S)-1-[3-(3,4-difluorophenyl)-1H-pyrazol-1-yl]propan-2-yl}carbamate

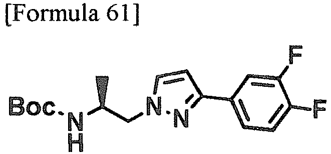

By using (2S)-2-[(tert-butoxycarbonyl)amino]propyl methane sulfonate obtained in Reference Example 5 (0.45 g, 1.77 mmol) and 3-(3,4-difluorophenyl)-1H-pyrazole (0.35 g, 1.95 mmol) as the raw materials, the same procedure as in Reference Example 6 was carried out to obtain the title compound (0.30 g) (colorless solid).
MS (ESI pos.) m/z: 338 [M+H]+

### Reference Example 34: tert-Butyl {(2S)-1-[3-(3,4-difluorophenyl)-1H-pyrazol-1-yl]propan-2-yl}ethyl carbamate

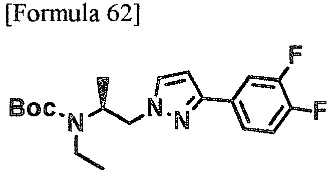

By using tert-butyl {(2S)-1-[3-(3,4-difluorophenyl)-1H-pyrazol-1-yl]propan-2-yl}carbamate obtained in Reference Example 33 (0.30 g, 0.89 mmol) as the raw material, the same procedure as in Reference Example 7 was carried out to obtain the title compound (0.34 g) (colorless oil).
MS (ESI pos.) m/z: 366 [M+H]+

### Reference Example 35: (2S)-1-[3-(3,4-Difluorophenyl)-1H-pyrazol-1-yl]-N-ethylpropan-2-amine hydrochloride

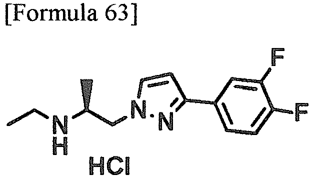

By using tert-butyl {(2S)-1-[3-(3,4-difluorophenyl)-1H-pyrazol-1-yl]propan-2-yl}ethyl carbamate obtained in Reference Example 34 (0.33 g, 0.89 mmol) as the raw material, the same procedure as in Reference Example 8 was carried out to obtain the title compound (0.19 g) (colorless solid).
MS (ESI pos.) m/z: 266 [M+H]+

### Reference Example 36: tert-Butyl {(2S)-1-[4-(4-fluorophenyl)-1H-pyrazol-1-yl]propan-2-yl}carbamate

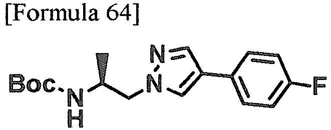

By using (2S)-2-[(tert-butoxycarbonyl)amino]propyl methane sulfonate obtained in Reference Example 5 (0.41 g, 2.54 mmol) and 4-(4-fluorophenyl)-1H-pyrazole (0.97 g, 3.81 mmol) as the raw materials, the same procedure as in Reference Example 6 was carried out to obtain the title compound (0.68 g) (colorless solid).
MS (ESI pos.) m/z: 320 [M+H]+

### Reference Example 37: (2S)-1-[4-(4-Fluorophenyl)-1H-pyrazol-1-yl]propan-2-amine hydrochloride

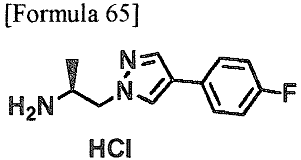

By using tert-butyl {(2S)-1-[4-(4-fluorophenyl)-1H-pyrazol-1-yl]propan-2-yl}carbamate obtained in Reference Example 36 (0.68 g, 2.11 mmol) as the raw material, the same procedure as in Reference Example 8 was carried out to obtain the title compound (0.54 g) (pale yellow and amorphous).
MS (ESI pos.) m/z: 220 [M+H]+

### Reference Example 38: N-{(2S)-1-[4-(4-Fluorophenyl)-1H-pyrazol-1-yl]propan-2-yl}-6-methyl-3-(2H-1,2,3-triazol-2-yl)pyridine-2-carboxamide

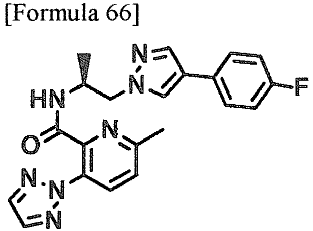

By using (2S)-1-[4-(4-fluorophenyl)-1H-pyrazol-1-yl]propan-2-amine hydrochloride obtained in Reference Example 37 (0.27 g, 1.05 mmol) and 6-methyl-3-(2H-1,2,3-triazol-2-yl)pyridine-2-carboxylic acid (0.21 g, 1.05 mmol) as the raw material, the same procedure as in Reference Example 10 was carried out to obtain the title compound (0.26 g) (pale yellow solid).
MS (ESI pos.) m/z: 406 [M+H]+

### Reference Example 39: tert-Butyl {(2S)-1-[4-(3,4-difluorophenyl)-1H-pyrazol-1-yl]propan-2-yl}carbamate

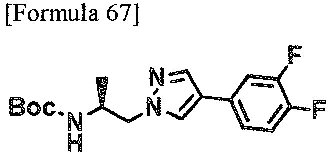

By using (2S)-2-[(tert-butoxycarbonyl)amino]propyl methane sulfonate obtained in Reference Example 5 (1.16 g, 4.59 mmol) and 4-(3,4-difluorophenyl)-1H-pyrazole obtained in Reference Example 4 (0.55 g, 3.06 mmol) as the raw materials, the same procedure as in Reference Example 6 was carried out to obtain the title compound (0.91 g) (colorless solid). MS (ESI pos.) m/z: 338 [M+H]+

### Reference Example 40: (2S)-1-[4-(3,4-Difluorophenyl)-1H-pyrazol-1-yl]propan-2-amine hydrochloride

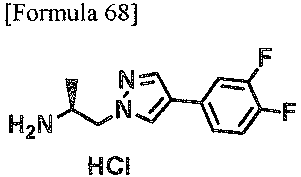

By using tert-butyl {(2S)-1-[4-(3,4-difluorophenyl)-1H-pyrazol-1-yl]propan-2-yl}carbamate obtained in Reference Example 39 (0.91 g, 2.69 mmol) as the raw material, the same procedure as in Reference Example 8 was carried out to obtain the title compound (0.73 g) (colorless and amorphous).
MS (ESI pos.) m/z: 238 [M+H]+

### Reference Example 41: N-{(2S)-1-[4-(3,4-Difluorophenyl)-1H-pyrazol-1-yl]propan-2-yl}-6-methyl-3-(2H-1,2,3-triazol-2-yl)pyridine-2-carboxamide

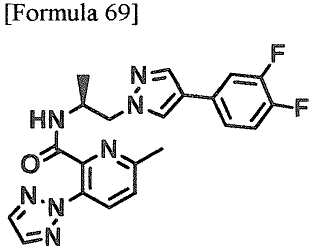

By using (2S)-1-[4-(3,4-difluorophenyl)-1H-pyrazol-1-yl]propan-2-amine hydrochloride obtained in Reference Example 40 (0.37 g, 1.34 mmol) and 6-methyl-3-(2H-1,2,3-triazol-2-yl)pyridine-2-carboxylic acid (0.27 g, 1.34 mmol) as the raw materials, the same procedure as in Reference Example 10 was carried out to obtain the title compound (0.35 g) (pale yellow solid).
MS (ESI pos.) m/z: 424 [M+H]+

### Reference Example 42: 5-Fluoro-N-{(2S)-1-[4-(4-fluorophenyl)-1H-pyrazol-1-yl]propan-2-yl}-2-(pyrimidin-2-yl)benzamide

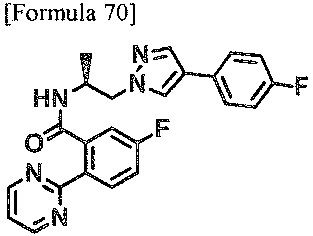

By using (2S)-1-[4-(4-fluorophenyl)-1H-pyrazol-1-yl]propan-2-amine hydrochloride obtained in Reference Example 37 (0.27 g, 1.05 mmol) and 5-fluoro-2-(pyrimidin-2-yl)benzoic acid (0.29 g, 1.33 mmol) as the raw materials, the same procedure as in Reference Example 10 was carried out to obtain the title compound (0.30 g) (brown and amorphous).
MS (ESI pos.) m/z: 420 [M+H]+

### Reference Example 43: N-{(2S)-1-[4-(3,4-Difluorophenyl)-1H-pyrazol-1-yl]propan-2-yl}-5-fluoro-2-(pyrimidin-2-yl)benzamide

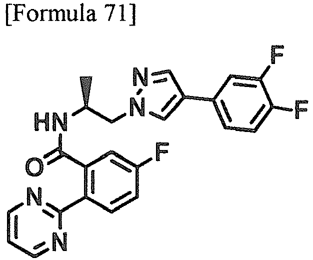

By using (2S)-1-[4-(3,4-difluorophenyl)-1H-pyrazol-1-yl]propan-2-amine hydrochloride obtained in Reference Example 40 (0.37 g, 1.34 mmol) and 5-fluoro-2-(pyrimidin-2-yl)benzoic acid (0.37 g, 1.70 mmol) as the raw materials, the same procedure as in Reference Example 10 was carried out to obtain the title compound (0.43 g) (brown and amorphous).
MS (ESI pos.) m/z: 460 [M+Na]+

### Reference Example 44 tert-Butyl [(2S)-1-cyanopropan-2-yl]carbamate

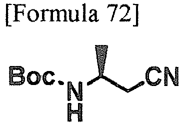

A solution of sodium cyanide (0.54 g, 11.05 mmol) in DMF (10 mL) was stirred at 35°C for 30 minutes, then tetrabutylammonium bromide (0.28 g, 8.56 mmol) was added, and then the resulting mixture was stirred for 2 hours. To the reaction mixture, a solution of (2S)-2-[(tert-butoxycarbonyl)amino]propyl methane sulfonate obtained in Reference Example 5 (2.17 g, 8.56 mmol) in DMF (2 mL) was added, then the resulting mixture was stirred at 35°C for 4 hours and then at room temperature for 12 hours. Water was added to the reaction mixture, followed by extraction with EtOAc. The organic layer was washed with brine, and then the solvent was distilled off under reduced pressure. The obtained residue was purified by column chromatography (SNAP Ultra 50g, hexane/EtOAc = 95/5 to 60/40) to obtain the title compound (0.91 g) (colorless solid).
MS (ESI pos.) m/z: 207 [M+Na]+

### Reference Example 45: tert-Butyl {(2S)-1-[5-(5-fluoropyridin-2-yl)-1,2,4-oxadiazol-3-yl]propan-2-yl }carbamate

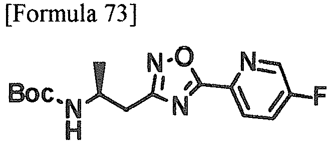

To a solution of tert-butyl [(2S)-1-cyanopropan-2-yl]carbamate obtained in Reference Example 44 (0.91 g, 4.95 mmol) in EtOH (5 mL), a 50% aqueous solution of hydroxylamine (0.65 mL, 9.90 mmol) was added, then the mixture was heated to 90°C and stirred for 14 hours. Water was added to the reaction mixture, followed by extraction with EtOAc, then the organic layer was dried over MgSO₄, then the drying agent was filtered off, and the solvent was distilled off under reduced pressure. To a solution of 5-fluoropyridine-2-carboxylic acid hydrochloride (0.97 g, 5.45 mmol) in DMF (10 mL), 1,1'-carbonyldiimidazole (0.96 g, 5.94 mmol) was added, then the resulting mixture was stirred at room temperature for 1 hour. To the reaction solution, a solution of the residue obtained in the previous process in DMF (3 mL) was added, then the mixture was heated to 90°C and stirred for 7 hours and then at room temperature for 60 hours. Water was added to the reaction mixture, followed by extraction with EtOAc. The solvent was distilled off under reduced pressure, and the obtained residue was purified by column chromatography (SNAP Ultra 50g, hexane/EtOAc = 98/2 to 30/70) to obtain the title compound (1.16 g) (colorless solid).
MS (ESI pos.) m/z: 345 [M+Na]+

### Reference Example 46: tert-Butyl {(2S)-1-[5-(5-fluoropyridin-2-yl)-1,2,4-oxadiazol-3-yl]propan-2-yl}methyl carbamate

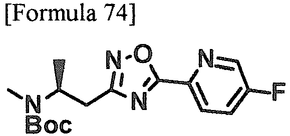

By using tert-butyl {(2S)-1-[5-(5-fluoropyridin-2-yl)-1,2,4-oxadiazol-3-yl]propan-2-yl}carbamate obtained in Reference Example 45 (0.58 g, 1.80 mmol) as the raw material, the same procedure as in Reference Example 7 was carried out by using MeI (0.134 mL, 2.16 mmol) to obtain the title compound (0.34 g) (light brown oil).
MS (ESI pos.) m/z: 359 [M+Na]+

### Reference Example 47: (2S)-1-[5-(5-Fluoropyridin-2-yl)-1,2,4-oxadiazol-3-yl]-N-methylpropan-2-amine

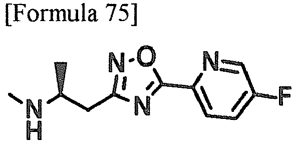

To a solution of tert-butyl {(2S)-1-[5-(5-fluoropyridin-2-yl)-1,2,4-oxadiazol-3-yl]propan-2-yl}methyl carbamate obtained in Reference Example 46 (0.34 g, 1.00 mmol) in chloroform (3 mL), trifluoroacetic acid (1.2 mL, 16.2 mmol) was added, then the resulting mixture was stirred at room temperature for 16 hours. A saturated aqueous solution of NaHCO₃ was added to the reaction mixture, followed by extraction with CHCl₃ and a 10% solution of MeOH/CHCl₃. The solvent was distilled off under reduced pressure to obtain the title compound (0.24 g) (yellow solid).
MS (ESI pos.) m/z: 237 [M+H]+

### Reference Example 48: tert-Butyl ethyl{(2S)-1-[5-(5-fluoropyridin-2-yl)-1,2,4-oxadiazol-3-yl]propan-2-yl}carbamate

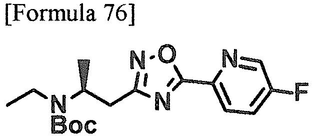

By using tert-butyl {(2S)-1-[5-(5-fluoropyridin-2-yl)-1,2,4-oxadiazol-3-yl]propan-2-yl}carbamate obtained in Reference Example 45 (0.58 g, 1.80 mmol) as the raw material, the same procedure as in Reference Example 7 was carried out to obtain the title compound (0.22 g) (yellow and amorphous).
MS (ESI pos.) m/z: 373 [M+Na]+

### Reference Example 49: tert-Butyl {(2S)-1-[3-(5-fluoropyridin-2-yl)-1H-pyrazol-1-yl]propan-2-yl}carbamate

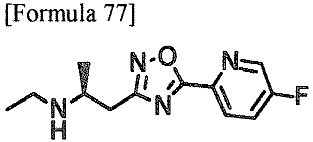

By using tert-butyl ethyl{(2S)-1-[5-(5-fluoropyridin-2-yl)-1,2,4-oxadiazol-3-yl]propan-2-yl}carbamate obtained in Reference Example 48 (0.22 g, 0.63 mmol) as the raw material, the same procedure as in Reference Example 47 was carried out to obtain the title compound (0.075 g) (yellow solid).
MS (ESI pos.) m/z: 251 [M+H]+

### Reference Example 50: tert-Butyl [(2S)-1-cyanopropan-2-yl]ethyl carbamate

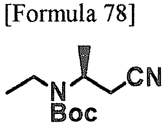

By using tert-butyl [(2S)-1-cyanopropan-2-yl]carbamate obtained in Reference Example 44 (1.7 g, 9.23 mmol) as the raw material, the same procedure as in Reference Example 7 was carried out to obtain the title compound (0.94 g) (pale yellow oil).
MS (ESI pos.) m/z: 213 [M+H]+

### Reference Example 51: (tert-Butyl ethyl{(2S)-1-[5-(4-fluorophenyl)-1,2,4-oxadiazol-3-yl]propan-2-yl}carbamate

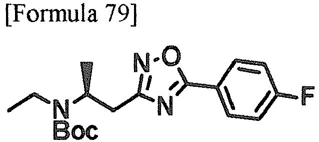

By using tert-butyl [(2S)-1-cyanopropan-2-yl]ethyl carbamate obtained in Reference Example 50 (0.45 g, 2.12 mmol) and 4-fluorobenzoic acid (0.33 g, 2.33 mmol) as the raw materials, the same procedure as in Reference Example 45 was carried out to obtain the title compound (0.35 g) (colorless oil).
MS (ESI pos.) m/z: 372 [M+Na]+

### Reference Example 52: (2S)-N-Ethyl-1-[5-(4-fluorophenyl)-1,2,4-oxadiazol-3-yl]propan-2-amine

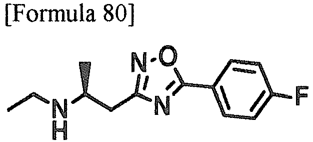

By using (tert-butyl ethyl{(2S)-1-[5-(4-fluorophenyl)-1,2,4-oxadiazol-3-yl]propan-2-yl}carbamate obtained in Reference Example 51 (0.35 g, 0.99 mmol) as the raw material, the same procedure as in Reference Example 47 was carried out to obtain the title compound (0.24 g) (yellow oil).
MS (ESI pos.) m/z: 250 [M+H]+

### Reference Example 53: tert-Butyl [(2S)-1-cyanopropan-2-yl]methyl carbamate

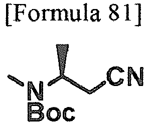

By using tert-butyl [(2S)-1-cyanopropan-2-yl]carbamate obtained in Reference Example 44 (0.35 g, 1.90 mmol) as the raw material, the same procedure as in Reference Example 7 was carried out by using MeI (0.142 mL, 2.28 mmol) to obtain the title compound (0.12 g) (pale yellow oil).
MS (ESI pos.) m/z: 221 [M+Na]+

### Reference Example 54: tert-Butyl {(2S)-1-[5-(4-fluorophenyl)-1,2,4-oxadiazol-3-yl]propan-2-yl}methyl carbamate

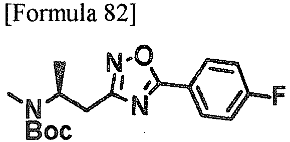

By using tert-butyl [(2S)-1-cyanopropan-2-yl]methyl carbamate obtained in Reference Example 53 (0.12 g, 0.59 mmol) and 4-fluorobenzoic acid (0.091 g, 0.65 mmol) as the raw materials, the same procedure as in Reference Example 45 was carried out to obtain the title compound (0.076 g) (colorless oil).
MS (ESI pos.) m/z: 358 [M+Na]+

### Reference Example 55: (2S)-1-[5-(4-Fluorophenyl)-1,2,4-oxadiazol-3-yl]-N-methylpropan-2-amine

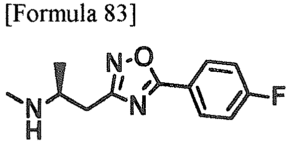

By using tert-butyl {(2S)-1-[5-(4-fluorophenyl)-1,2,4-oxadiazol-3-yl]propan-2-yl}methyl carbamate obtained in Reference Example 54 (0.076 g, 0.23 mmol) as the raw material, the same procedure as in Reference Example 47 was carried out to obtain the title compound (0.039 g) (colorless oil).
MS (ESI pos.) m/z: 236 [M+H]+

### Reference Example 56: Ethyl 1-(5-fluoropyridin-2-yl)-1H-pyrazole-4-carboxylate

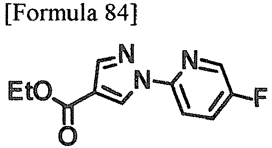

To a solution of ethyl 1H-pyrazole-4-carboxylate (1.0 g, 7.14 mmol) in DMF (10 mL), 60% NaH (0.29 g, 7.13 mmol) was gradually added under ice-cooling, and the resulting mixture was returned to room temperature and stirred for 1 hour. The resulting mixture was ice-cooled again, then 2,5-difluoropyridine (0.68 g, 5.94 mmol) was added dropwise thereto, then the resulting mixture was stirred at room temperature for 66 hours and then heated to 80°C and stirred for 5 hours. Water (80 mL) was added to the reaction mixture, and the precipitate was filtered out. The obtained precipitate was purified by column chromatography (Grace 12g, hexane/EtOAc = 92/8 to 35/65) to obtain the title compound (0.58 g) (colorless solid).
MS (ESI pos.) m/z: 258 [M+Na]+

### Reference Example 57: [1-(5-Fluoropyridin-2-yl)-1H-pyrazol-4-yl]methanol

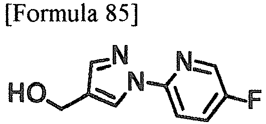

To a solution of ethyl 1-(5-fluoropyridin-2-yl)-1H-pyrazole-4-carboxylate obtained in Reference Example 56 (0.67 g, 2.83 mmol) in toluene (12 mL), a 1.02M solution of diisobutylaluminium hydride-hexane (6.10 mL, 6.22 mmol) was added dropwise at -60°C or below, and then the resulting mixture was gradually heated. The resulting mixture was stirred for 3 hours, and then a 1M aqueous solution of HCl (20 mL) was added thereto at -48°C. After extraction with EtOAc, the solvent was distilled off under reduced pressure. The obtained residue was purified by column chromatography (Grace 12g, hexane/EtOAc = 92/8 to 35/65) to obtain the title compound (0.40 g) (colorless solid).
MS (ESI pos.) m/z: 216 [M+Na]+

### Reference Example 58: 1-(5-Fluoropyridin-2-yl)-1H-pyrazole-4-carbaldehyde

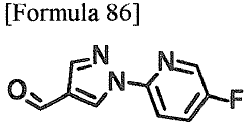

A solution of [1-(5-fluoropyridin-2-yl)-1H-pyrazol-4-yl]methanol obtained in Reference Example 57 (0.40 g, 2.07 mmol) and MnO₂ (1.80 g, 20.7 mmol) in chloroform (10 mL) was stirred at room temperature for 5.5 hours and then at 60°C for 5 hours. The reaction mixture was filtered through Celite® and washed with chloroform, and then the filtrate was concentrated under reduced pressure to obtain the title compound (0.36 g) (colorless solid).
MS (ESI pos.) m/z: 192 [M+H]+

### Reference Example 59: 5-Fluoro-2-{4-[(1E)-2-nitropropan-1-en-1-yl]-1H-pyrazol-1-yl}pyridine

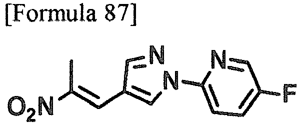

A suspension of 1-(5-fluoropyridin-2-yl)-1H-pyrazole-4-carbaldehyde obtained in Reference Example 58 (0.26 g, 1.34 mmol), nitroethane (0.16 g, 2.13 mmol), and formic acid:2-aminoethanol (1:1) (2.0 g, 18.7 mmol) was stirred at room temperature for 19 hours. Water was added to the reaction mixture, followed by extraction with EtOAc. The organic layer was concentrated under reduced pressure and then purified by column chromatography (Grace 12g, hexane/EtOAc = 92/8 to 35/65) to obtain the title compound (0.32 g) (light brown solid).
MS (ESI pos.) m/z: 249 [M+H]+

### Reference Example 60: 1-[1-(5-Fluoropyridin-2-yl)-1H-pyrazol-4-yl]propan-2-amine

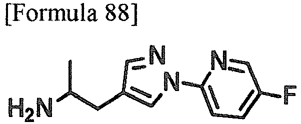

A solution of 5-fluoro-2-{4-[(1E)-2-nitropropan-1-en-1-yl]-1H-pyrazol-1-yl}pyridine obtained in Reference Example 59 (0.15 g, 0.60 mmol) and 10% Pd/C (0.15 g) in THF-MeOH (10 mL - 5 mL) was stirred at room temperature for 14 hours under a hydrogen atmosphere. The reaction mixture was filtered through Celite® and then the obtained filtrate was concentrated under reduced pressure. The obtained residue was purified by column chromatography (SNAP KP-NH 28g, hexane/EtOAc = 75/25 to 0/100) to obtain the title compound (0.04 g) (pale yellow oil).
MS (ESI pos.) m/z: 221 [M+H]+

### Reference Example 61: N-{1-[1-(5-Fluoropyridin-2-yl)-1H-pyrazol-4-yl]propan-2-yl}-5-methyl-2-(2H-1,2,3-triazol-2-yl)benzamide

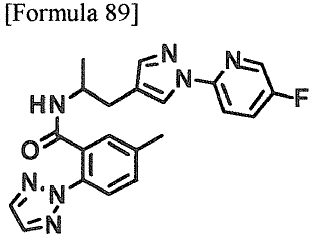

By using 1-[1-(5-fluoropyridin-2-yl)-1H-pyrazol-4-yl]propan-2-amine obtained in Reference Example 60 (0.043 g, 0.21 mmol) and 5-methyl-2-(2H-1,2,3-triazol-2-yl)benzoic acid (0.039 g, 0.18 mmol) as the raw materials, the same procedure as in Reference Example 10 was carried out to obtain the title compound (0.005 g) (colorless solid).
MS (ESI pos.) m/z: 406 [M+H]+

### Reference Example 62: Ethyl 1-(5-fluoropyridin-2-yl)-1H-pyrazole-3-carboxylate

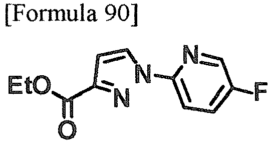

By using ethyl 1H-pyrazole-3-carboxylate (0.50 g, 3.57 mmol) and 2,5-difluoropyridine (0.49 g, 4.28 mmol) as the raw materials, the same procedure as in Reference Example 56 was carried out to obtain the title compound (0.15 g) (colorless solid).
MS (ESI pos.) m/z: 236 [M+H]+

### Reference Example 63: [1-(5-Fluoropyridin-2-yl)-1H-pyrazol-3-yl]methanol

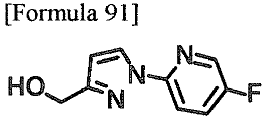

By using ethyl 1-(5-fluoropyridin-2-yl)-1H-pyrazole-3-carboxylate obtained in Reference Example 62 (10.0 g, 42.5 mmol) as the raw material, the same procedure as in Reference Example 57 was carried out to obtain the title compound (8.0 g) (pale yellow solid).
MS (ESI pos.) m/z: 194 [M+H]+

### Reference Example 64: 1-(5-Fluoropyridin-2-yl)-1H-pyrazole-3-carbaldehyde

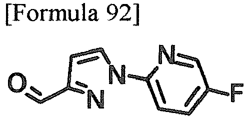

By using [1-(5-fluoropyridin-2-yl)-1H-pyrazol-3-yl]methanol obtained in Reference Example 63 (8.0 g, 34.0 mmol) as the raw material, the same procedure as in Reference Example 58 was carried out to obtain the title compound (5.3 g) (light brown solid). MS (ESI pos.) m/z: 192 [M+H]+

### Reference Example 65: 5-Fluoro-2-{3-[(1E)-2-nitropropan-1-en-1-yl]-1H-pyrazol-1-yl}pyridine

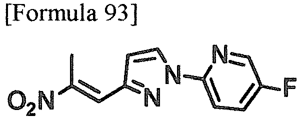

By using 1-(5-fluoropyridin-2-yl)-1H-pyrazole-3-carbaldehyde obtained in Reference Example 64 (0.10 g, 0.52 mmol) as the raw material, the same procedure as in Reference Example 59 was carried out to obtain the title compound (0.13 g) (yellow solid).
MS (ESI pos.) m/z: 249 [M+H]+

### Reference Example 66: 1-[1-(5-Fluoropyridin-2-yl)-1H-pyrazol-3-yl]propan-2-amine

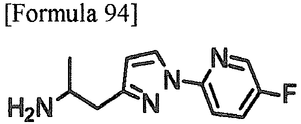

A solution of 5-fluoro-2-{3-[(1E)-2-nitropropan-1-en-1-yl]-1H-pyrazol-1-yl}pyridine obtained in Reference Example 65 (0.13 g, 0.54 mmol) in THF (2 mL) was added dropwise to a solution of LAH (0.12 g, 3.22 mmol) in THF (3 mL) under ice-cooling. The resulting mixture was stirred per se for 1 hour under ice-cooling, and then heated to room temperature and stirred for 1 hour. Na₂SO₄•10H₂O was added to the reaction mixture, then the reaction was stopped, Na₂SO₄ was added thereto, and then the resulting mixture was stirred. Insolubles were separated by filtration through Celite®, then the filtrate was concentrated under reduced pressure. The obtained residue was purified by HPLC to obtain the title compound (0.0068 g) (colorless oil).
MS (ESI pos.) m/z: 221 [M+H]+

### Reference Example 67: N-{1-[1-(5-Fluoropyridin-2-yl)-1H-pyrazol-3-yl]propan-2-yl}-5-methyl-2-(2H-1,2,3-triazol-2-yl)benzamide

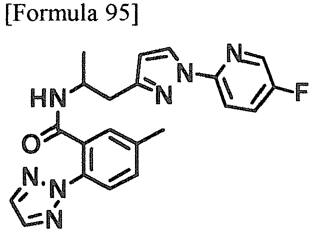

By using 1-[1-(5-fluoropyridin-2-yl)-1H-pyrazol-3-yl]propan-2-amine obtained in Reference Example 66 (0.0068 g, 0.031 mmol) and 5-methyl-2-(2H-1,2,3-triazol-2-yl)benzoic acid (0.0069 g, 0.034 mmol) as the raw materials, the same procedure as in Reference Example 10 was carried out to obtain the title compound (0.0054 g) (colorless and amorphous).
MS (ESI pos.) m/z: 428 [M+Na]+

### Reference Example 68: Methyl (3S)-3-{[5-methyl-2-(2H-1,2,3-triazol-2-yl)benzoyl]amino}butanoate

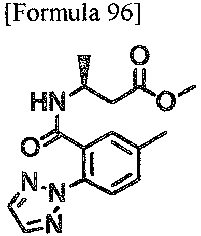

To a suspension of (3 S)-3-aminobutanoic acid (0.20 g, 1.94 mmol) in MeOH (1 mL)-THF (1 mL), a solution of 2.0M (diazomethyl)(trimethyl)silane-Et₂O (1.0 mL, 2.0 mmol) was added dropwise, and the mixture was stirred at room temperature for 3 hours. The solvent was distilled off under reduced pressure, then by using the obtained residue and 5-methyl-2-(2H-1,2,3-triazol-2-yl)benzoic acid (0.26 g, 1.29 mmol) as the raw materials, the same procedure as in Reference Example 10 was carried out to obtain the title compound (0.029 g) (colorless solid).
MS (ESI pos.) m/z: 303 [M+H]+

### Reference Example 69: Ethyl (3S)-3-{ethyl[5-methyl-2-(2H-1,2,3-triazol-2-yl)benzoyl]amino}butanoate

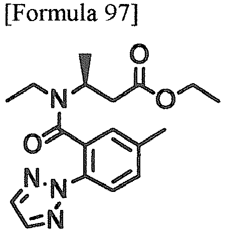

By using methyl (3S)-3-{[5-methyl-2-(2H-1,2,3-triazol-2-yl)benzoyl]amino}butanoate obtained in Reference Example 68 (0.067 g, 0.22 mmol) as the raw material, the same procedure as in Reference Example 7 was carried out to obtain the title compound (0.047 g).
MS (ESI pos.) m/z: 345 [M+H]+

### Reference Example 70: (3S)-3-{Ethyl[5-methyl-2-(2H-1,2,3-triazol-2-yl)benzoyl]amino}butanoic acid

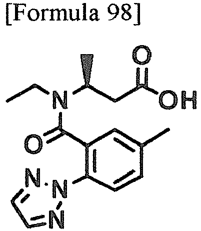

A solution of ethyl (3S)-3-{ethyl[5-methyl-2-(2H-1,2,3-triazol-2-yl)benzoyl]amino}butanoate obtained in Reference Example 69 (0.044 g, 0.13 mmol) and a 2M aqueous solution of NaOH (0.32 mL, 0.64 mmol) in EtOH (2 mL) was stirred at 60°C for 2 hours. EtOAc (30 mL) was added to the reaction mixture, and the resulting mixture was washed with water (30 mL) twice. A 1M aqueous solution of HCl was added to the aqueous layer for adjustment of pH to about 1, and then the resulting mixture was extracted with CHCl₃ (40 mL) three times. The obtained organic layer was allowed to pass through an ISOLUTE Phase Separator, and then the solvent was distilled off under reduced pressure to obtain the title compound (0.029 g) (colorless oil).
MS (ESI pos.) m/z: 317 [M+H]+

### Reference Example 71: N-Ethyl-N-{(2S)-4-[2-(4-fluorobenzoyl)hydrazinyl]-4-oxobutan-2-yl}-5-methyl-2-(2H-1,2,3-triazol-2-yl)benzamide

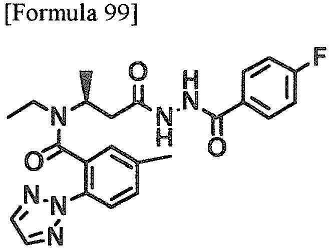

By using (3S)-3-{ethyl[5-methyl-2-(2H-1,2,3-triazol-2-yl)benzoyl]amino}butanoic acid obtained in Reference Example 70 (0.028 g, 0.089 mmol) and 4-fluorobenzohydrazide (0.022 g, 0.14 mmol) as the raw materials, the same procedure as in Reference Example 10 was carried out to obtain the title compound (0.027 g) (colorless solid).
MS (ESI pos.) m/z:475 [M+Na]+

### Reference Example 72: tert-Butyl {(2S)-1-[5-(4-fluorophenyl)-2H-tetrazol-2-yl)propan-2-yl}carbamate

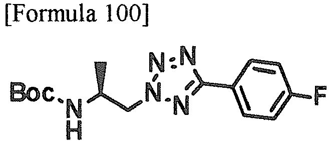

By using (2S)-2-[(tert-butoxycarbonyl)amino]propyl methane sulfonate obtained in Reference Example 5 (2.89 g, 11.4 mmol) and 5-(4-fluorophenyl)-2H-tetrazole (1.86 g, 11.4 mmol) as the raw materials, the same procedure as in Reference Example 6 was carried out to obtain the title compound (0.54 g) (pale yellow oil).
MS (ESI pos.) m/z: 322 [M+H]+

### Reference Example 73: (2S)-1-[5-(4-Fluorophenyl)-2H-tetrazol-2-yl]propan-2-amine

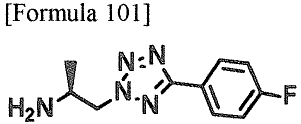

To tert-butyl {(2S)-1-[5-(4-fluorophenyl)-2H-tetrazol-2-yl]propan-2-yl}carbamate obtained in Reference Example 72 (0.5 g, 1.6 mmol), a 4M solution of HCl-dioxane (2.0 mL, 8.0 mmol) was added, and the mixture was stirred at room temperature for 3 hours. The reaction mixture was concentrated under reduced pressure, then chloroform and a saturated aqueous solution of NaHCO₃ were added to the obtained residue for extraction. The obtained organic layer was washed with water and then dried over Na₂SO₄. The drying agent was filtered off, then the solvent was distilled off under reduced pressure to obtain the title compound (0.34 g) (pale yellow oil).
MS (ESI pos.) m/z: 222 [M+H]+

### Reference Example 74: N-{(2S)-1-[5-(4-Fluorophenyl)-2H-tetrazol-2-yl]propan-2-yl}-5-methyl-2-(2H-1,2,3-triazol-2-yl)benzamide

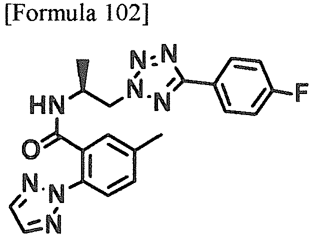

By using (2S)-1-[5-(4-fluorophenyl)-2H-tetrazol-2-yl]propan-2-amine obtained in Reference Example 73 (0.11 g, 0.49 mmol) and 5-methyl-2-(2H-1,2,3-triazol-2-yl)benzoic acid (0.10 g, 0.49 mmol) as the raw materials, the same procedure as in Reference Example 10 was carried out to obtain the title compound (0.18 g) (pale yellow oil).
MS (ESI pos.) m/z: 407 [M+H]+

### Reference Example 75: tert-Butyl ethyl{(2S)-1-[5-(4-fluorophenyl)-2H-tetrazol-2-yl]propan-2-yl}carbamate

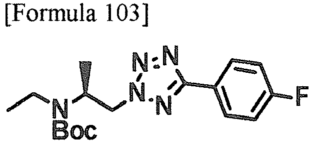

By using tert-butyl {(2S)-1-[5-(4-fluorophenyl)-2H-tetrazol-2-yl]propan-2-yl}carbamate obtained in Reference Example 72 (0.50 g, 1.56 mmol) as the raw material, the same procedure as in Reference Example 7 was carried out to obtain the title compound (0.26 g) (pale yellow solid).
MS (ESI pos.) m/z: 350 [M+H]+

### Reference Example 76: (2S)-N-Ethyl-1-[5-(4-fluorophenyl)-2H-tetrazol-2-yl]propan-2-amine

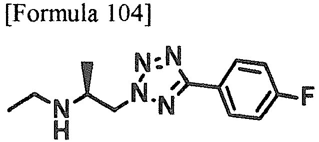

By using tert-butyl ethyl{(2S)-1-[5-(4-fluorophenyl)-2H-tetrazol-2-yl]propan-2-yl}carbamate obtained in Reference Example 75 (0.26 g, 0.74 mmol) as the raw material, the same procedure as in Reference Example 73 was carried out to obtain the title compound (0.17 g) (pale yellow oil).
MS (ESI pos.) m/z: 250 [M+H]+

### Reference Example 77: tert-Butyl [(2S)-1-azidepropan-2-yl]carbamate

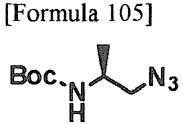

A solution of (2S)-2-[(tert-butoxycarbonyl)amino]propyl methane sulfonate obtained in Reference Example 5 (4.3 g, 17.1 mmol) and sodium azide (3.34 g, 51.4 mmol) in DMF (20 mL) was stirred at 80°C for 3 hours. The reaction mixture was allowed to cool to room temperature, then water was added thereto, followed by extraction with EtOAc. The obtained organic layer was washed with water, and then dried over Na₂SO₄. The drying agent was filtered off, then the solvent was distilled off under reduced pressure, and then the obtained residue was purified by column chromatography (SNAP Ultra 50g, hexane/EtOAc = 100/0 to 0/100) to obtain the title compound (1.8 g) (pale yellow oil).
1H NMR (600 MHz, CHLOROFORM-d) δ ppm : 1.04 - 1.27 (m, 3 H), 1.44 (s, 9 H), 3.14 - 3.50 (m, 2 H), 3.84 (br. s., 1 H), 4.53 (br. s., 1 H)

### Reference Example 78: tert-Butyl {(2S)-1-[4-(4-fluorophenyl)-1H-1,2,3-triazol-1-yl]propan-2-yl}carbamate

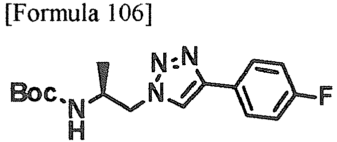

To a solution of tert-butyl [(2S)-1-azidepropan-2-yl]carbamate obtained in Reference Example 77 (0.80 g, 4.00 mmol) in DMF/4-methylpiperidine (5 mL/5 mL), 1-ethynyl-4-fluorobenzene (0.48 g, 4.00 mmol), CuI (0.038 g, 0.20 mmol), and L-ascorbic acid (0.14 g, 0.80 mmol) were added, and the resulting mixture was stirred at 80°C for 3 hours. The reaction mixture was allowed to cool to room temperature, and then filtered through Celite®. Water was added to the filtrate, followed by extraction with EtOAc, and the organic layer was washed with water and then dried over Na₂SO₄. The drying agent was filtered off, and then the residue obtained by concentration under reduced pressure was purified by column chromatography (SNAP Ultra 50g, hexane/EtOAc = 100/0 to 0/100) to obtain the title compound (0.82 g) (pale yellow oil).
MS (ESI pos.) m/z: 321 [M+H]+

### Reference Example 79: N-{(2S)-1-[4-(4-fluorophenyl)-1H-1,2,3-triazol-1-yl]propan-2-yl}-5-methyl-2-(2H-1,2,3-triazol-2-yl)benzamide

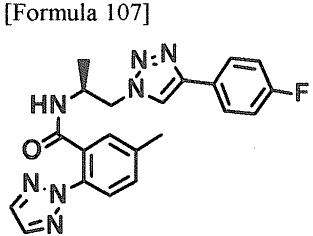

The Boc group was eliminated from tert-butyl {(2S)-1-[4-(4-fluorophenyl)-1H-1,2,3-triazol-1-yl)propan-2-yl}carbamate obtained in Reference Example 78 (0.79 g, 2.46 mmol) by carrying out the same procedure as in Reference Example 73 to obtain pale yellow oil. By using the obtained pale yellow oil and 5-methyl-2-(2H-1,2,3-triazol-2-yl)benzoic acid (0.50 g, 2.46 mmol) as the raw materials, the same procedure as in Reference Example 10 was carried out to obtain the title compound (0.65 g) (pale yellow oil).
MS (ESI pos.) m/z: 406 [M+H]+

### Reference Example 80: (2S)-1-[3-(4-Fluorophenyl)-1H-pyrazol-1-yl]propan-2-amine hydrochloride

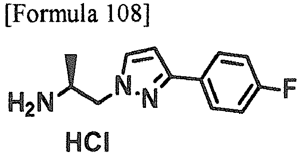

By using tert-butyl {(2S)-1-[3-(4-fluorophenyl)-1H-pyrazol-1-yl]propan-2-yl}carbamate obtained in Reference Example 25 (3.21 g, 10.1 mmol) as the raw material, the same procedure as in Reference Example 8 was carried out to obtain the title compound (2.58 g) (pale yellow and amorphous).
MS (ESI pos.) m/z: 220 [M+H]+

### Reference Example 81: 5-Fluoro-N-{(2S)-1-[4-(5-fluoropyridin-2-yl)-1H-pyrazol-1-yl]propan-2-yl}-2-(pyrimidin-2-yl)benzamide

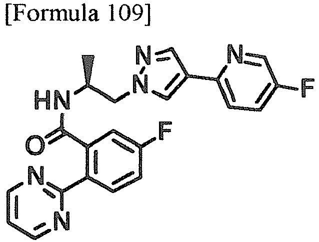

By using (2S)-1-[4-(5-fluoropyridin-2-yl)-1H-pyrazol-1-yl]propan-2-amine dihydrochloride obtained in Reference Example 13 (0.10 g, 0.36 mmol) and 5-fluoro-2-(pyrimidin-2-yl)benzoic acid (0.099 g, 0.46 mmol) as the raw materials, the same procedure as in Reference Example 10 was carried out to obtain the title compound (0.028 g) (colorless and amorphous).
MS (ESI pos.) m/z: 421 [M+H]+

Reference Examples 82 to 86 were obtained by the same procedure as in Reference Example 81. The structural formula, the names, and MS data of the obtained compounds are shown in Table 1.

**[Table 1]**

| Reference Example No. | Structural formula | Compound name | MS (ESI pos.) m/z |
|---|---|---|---|
| Reference Example 82 | 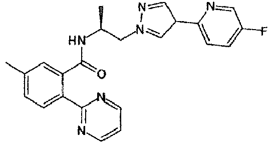 | N-{(2S)-1-[4-(5-Fluoropyridin-2-yl)-1H-pyrazol-1-yl]propan-2-yl}-5-methyl-2-(pyrimidin-2-yl) benzamide | 417(M+H)+ |
| Reference Example 83 | 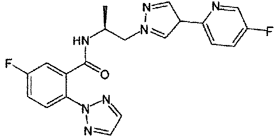 | 5-Fluoro-N-{(2S)-1-[4-(5-fluoropyridin-2-yl)-1H-pyrazol-1-yl]propan-2-yl}-2-(2H-1,2,3-triazol -2-yl)benzamide | 410(M+H)+ |
| Reference Example 84 | 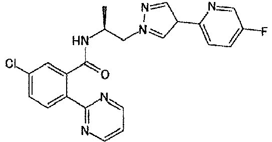 | 5-Chloro-N-{(2S)-1-[4-(5-fluoropyridin-2-yl)-1H-pyrazol-1-yl]propan-2-yl}-2-(pyrimidin-2-yl) benzamide | 459(M+Na)+ |
| Reference Example 85 | 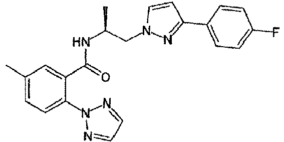 | N-{(2S)-1-[3-(4-Fluorophenyl)-1H-pyrazol-1-yl] propan-2-yl}-5-methyl-2-(2H-1,2,3-triazol-2-yl) benzamide | 405(M+H)+ |
| Reference Example 86 | 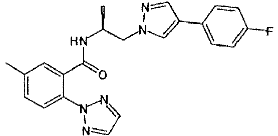 | N-{(2S)-1-[4-(4-Fluorophenyl)-1H-pyrazol-1 -yl] propan-2-yl}-5-methyl-2-(2H-1,2,3-triazol-2-yl) benzamide | 405(M+H)+ |

### Reference Example 87: tert-Butyl {(2S)-1-[4-(5-fluoropyridin-2-yl)-1H-1,2,3-triazol-1-yl]propan-2-yl}carbamic acid

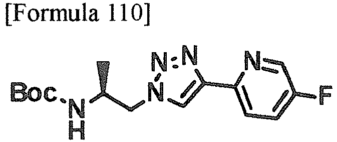

By using tert-butyl [(2S)-1-azidepropan-2-yl]carbamate obtained in Reference Example 77 (0.50 g, 2.48 mmol) and 2-ethynyl-5-fluoropyridine (0.30 g, 2.48 mmol) as the raw materials, the same procedure as in Reference Example 78 was carried out to obtain the title compound (0.58 g) (colorless solid).
MS (ESI pos.) m/z: 322 [M+H]+

### Reference Example 88: tert-Butyl ethyl{(2S)-1-[4-(5-fluoropyridin-2-yl)-1H-1,2,3-triazol-1-yl]propan-2-yl}carbamate

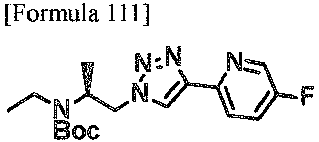

By using tert-butyl {(2S)-1-[4-(5-fluoropyridin-2-yl)-1H-1,2,3-triazol-1-yl]propan-2-yl}carbamic acid obtained in Reference Example 87 (0.30 g, 0.93 mmol) as the raw material, the same procedure as in Reference Example 7 was carried out to obtain the title compound (0.33 g) (colorless oil).
MS (ESI pos.) m/z: 350 [M+H]+

### Reference Example 89: (2S)-N-Ethyl-1-[4-(5-fluoropyridin-2-yl)-1H-1,2,3-triazol-1-yl]propan-2-amine

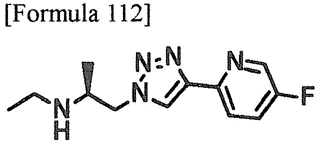

By using tert-butyl ethyl {(2S)-1-[4-(5-fluoropyridin-2-yl)-1H-1,2,3-triazol-1-yl]propan-2-yl}carbamate obtained in Reference Example 88 (0.33 g, 0.93 mmol) as the raw material, the same procedure as in Reference Example 73 was carried out to obtain the title compound (0.19 g) (colorless oil).
MS (ESI pos.) m/z: 250 [M+H]+

### Reference Example 90: 5-Fluoro-2-(2H-tetrazol-5-yl)pyridine

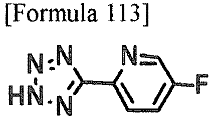

An aqueous solution (16 mL) of 5-fluoropyridine-2-carbonitrile (1.0 g, 8.19 mmol), zinc dibromide (1.84 g, 8.19 mmol), and sodium azide (0.59 g, 9.01 mmol) was stirred at 110°C for 15 hours. A 2M aqueous solution of HCl (20 mL) and EtOAc (70 mL) were added to the reaction solution, and the resulting mixture was intensely stirred at room temperature for 1 hour until the solid dissolved. After extraction with EtOAc, the organic layer was washed with brine and dried over Na₂SO₄. The drying agent was filtered off, and then the solvent was distilled off under reduced pressure. The obtained solid was washed with Et₂O and filtered out to obtain the title compound (1.17 g) (colorless solid).
MS (ESI pos.) m/z: 166 [M+H]+

### Reference Example 91: tert-Butyl {(2S)-1-[5-(5-fluoropyridin-2-yl)-2H-tetrazol-2-yl]propan-2-yl}carbamic acid

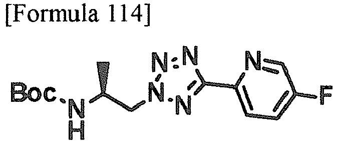

By using (2S)-2-[(tert-butoxycarbonyl)amino]propyl methane sulfonate obtained in Reference Example 5 (1.30 g, 5.14 mmol) and 5-fluoro-2-(2H-tetrazol-5-yl)pyridine obtained in Reference Example 90 (0.85 g, 5.14 mmol) as the raw materials, the same procedure as in Reference Example 6 was carried out to obtain the title compound (0.42 g) (colorless solid).
MS (ESI pos.) m/z: 345 [M+Na]+

### Reference Example 92: tert-Butyl ethyl{(2S)-1-[5-(5-fluoropyridin-2-yl)-2H-tetrazol-2-yl]propan-2-yl}carbamate

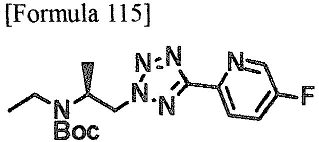

By using tert-butyl {(2S)-1-[5-(5-fluoropyridin-2-yl)-2H-tetrazol-2-yl]propan-2-yl}carbamic acid obtained in Reference Example 91 (0.42 g, 1.32 mmol) as the raw material, the same procedure as in Reference Example 7 was carried out to obtain the title compound (0.45 g) (colorless oil).
MS (ESI pos.) m/z: 373 [M+Na]+

### Reference Example 93: (2S)-N-Ethyl-1-[4-(5-fluoropyridin-2-yl)-1H-1,2,3-triazol-1-yl]propan-2-amine

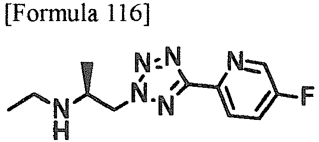

By using tert-butyl ethyl{(2S)-1-[5-(5-fluoropyridin-2-yl)-2H-tetrazol-2-yl]propan-2-yl}carbamate obtained in Reference Example 92 (0.45 g, 1.29 mmol) as the raw material, the same procedure as in Reference Example 73 was carried out to obtain the title compound (0.31 g) (colorless oil).
MS (ESI pos.) m/z: 251 [M+H]+

### Reference Example 94: tert-Butyl ethyl{(2S)-1-[4-(4-fluorophenyl)-1H-1,2,3-triazol-1-yl]propan-2-yl}carbamate

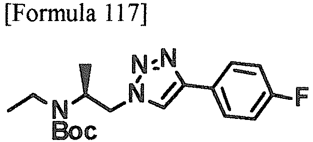

By using tert-butyl {(2S)-1-[4-(4-fluorophenyl)-1H-1,2,3-triazol-1-yl]propan-2-yl}carbamate obtained in Reference Example 78 (0.50 g, 1.56 mmol) as the raw material, the same procedure as in Reference Example 7 was carried out to obtain the title compound (0.54 g) (colorless oil).
MS (ESI pos.) m/z: 349 [M+H]+

### Reference Example 95: ((2S))-N-Ethyl-1-[4-(4-fluorophenyl)-1H-1,2,3-triazol-1-yl]propan-2-amine

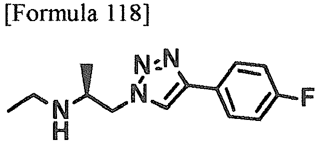

By using tert-butyl ethyl{(2S)-1-[4-(4-fluorophenyl)-1H-1,2,3-triazol-1-yl]propan-2-yl}carbamate obtained in Reference Example 94 (0.54 g, 1.56 mmol) as the raw material, the same procedure as in Reference Example 73 was carried out to obtain the title compound (0.39 g) (colorless oil).
MS (ESI pos.) m/z: 249 [M+H]+

### Reference Example 96: 2-[(2S)-1-Hydroxypropan-2-yl]-1H-isoindole-1,3(2H)-dione

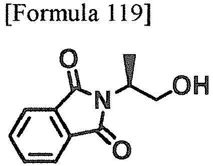

A solution of (2S)-2-aminopropan-1-ol (10 g, 133 mmol) and 2-benzofuran-1,3-dione (19.7 g, 133 mmol) in toluene (100 mL) was heated to reflux for 6 hours. A saturated aqueous solution of NaHCO₃ was added to the reaction mixture, followed by extraction with EtOAc. The organic layer was washed with water, dried over Na₂SO₄, and then the drying agent was filtered off. The solvent was distilled off under reduced pressure to obtain the title compound (24.3 g) (pale yellow solid).
MS (ESI pos.) m/z: 206 [M+H]+

### Reference Example 97: 2-[(2S)-1-(Methoxymethoxy)propan-2-yl]-1H-isoindole-1,3(2H)-dione

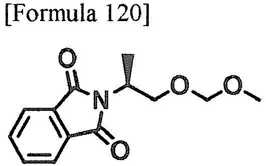

To a solution of 2-[(2S)-1-hydroxypropan-2-yl]-1H-isoindole-1,3(2H)-dione obtained in Reference Example 96 (16.7 g, 81.4 mmol) and DIPEA(21.3 mL, 122 mmol) in chloroform (50 mL), chloro(methoxy)methane (8.52 g, 106 mmol) was added at 0°C, and the mixture was heated to room temperature and stirred for 3 hours. Water was added to the reaction mixture, followed by extraction with EtOAc. The organic layer was washed with water, dried over Na₂SO₄, and then the drying agent was filtered off, The obtained residue was purified by column chromatography (SNAP Ultra 50g, hexane/EtOAc = 100/0 to 0/100) to obtain the title compound (9.3 g) (pale yellow oil).
MS (ESI pos.) m/z: 250 [M+H]+

### Reference Example 98: (2S)-1-(Methoxymethoxy)propan-2-amine

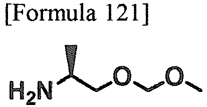

A solution of 2-[(2S)-1-(methoxymethoxy)propan-2-yl]-1H-isoindole-1,3(2H)-dione obtained in Reference Example 97 (9.7 g, 38.9 mmol) and hydrazine monohydrate (1.95 g, 38.9 mmol) in EtOH (50 mL) was stirred at 80°C for 2 hours. The reaction mixture was allowed to cool to room temperature, then the solvent was distilled off under reduced pressure to obtain pale yellow solid. Chloroform was added thereto, then the resulting mixture was stirred and filtered. The filtrate was concentrated under reduced pressure to obtain the title compound (2.3 g) (pale yellow oil).
1H NMR (200 MHz, CHLOROFORM-d) δ ppm 1.07 (d, J=6.15 Hz, 3 H) 3.01 - 3.54 (m, 6 H) 4.65 (s, 2 H)

### Reference Example 99: N-[(2S)-1-(Methoxymethoxy)propan-2-yl]-5-methyl-2-(2H-1,2,3-triazol-2-yl)benzamide

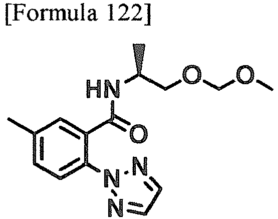

By using (2S)-1-(methoxymethoxy)propan-2-amine obtained in Reference Example 98 (0.92 g, 7.7 mmol) and 5-methyl-2-(2H-1,2,3-triazol-2-yl)benzoic acid (1.57 g, 7.7 mmol) as the raw materials, the same procedure as in Reference Example 10 was carried out to obtain the title compound (2.3 g) (pale yellow oil).
MS (ESI pos.) m/z: 305 [M+H]+

### Reference Example 100: N-Ethyl-N-[(2S)-1-(methoxymethoxy)propan-2-yl]-5-methyl-2-(2H-1,2,3-triazol-2-yl)benzamide

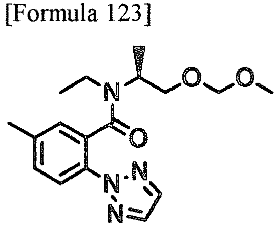

By using N-[(2S)-1-(methoxymethoxy)propan-2-yl]-5-methyl-2-(2H-1,2,3-triazol-2-yl)benzamide obtained in Reference Example 99 (2.28 g, 7.5 mmol) as the raw material, the same procedure as in Reference Example 7 was carried out by using EtI (0.90 mL, 11.3 mmol) to obtain the title compound (1.34 g) (pale yellow solid).
MS (ESI pos.) m/z: 333 [M+H]+

### Reference Example 101: (2S)-2-[(tert-Butoxycarbonyl)amino]propyl benzoate

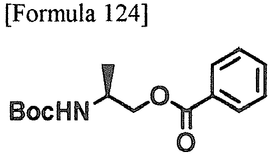

To a solution of tert-butyl [(2S)-1-hydroxypropan-2-yl]carbamate (2.0 g, 11.4 mmol) in chloroform (24 mL), triethylamine (2.1 mL, 14.8 mmol) and benzoyl chloride (1.6 mL, 13.7 mmol) were added under ice-cooling, and the resulting mixture was stirred for 4.5 hours under continuous ice-cooling. The reaction solution was washed with water twice after dilution with chloroform, and then the resultant was dried over MgSO₄. The drying agent was filtered off, the solvent was distilled off under reduced pressure, then the obtained solid was washed with hexane under stirring, and then filtered out and dried under reduced pressure to obtain the title compound (3.11 g) (colorless solid).
MS (ESI pos.) m/z: 302[M+Na]+

### Reference Example 102: (2S)-2-[(tert-Butoxycarbonyl)(ethyl)amino]propyl benzoate

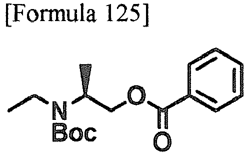

By using (2S)-2-[(tert-butoxycarbonyl)amino]propyl benzoate obtained in Reference Example 101 (1.50 g, 5.37 mmol) as the raw material, the same procedure as in Reference Example 7 was carried out by using EtI (0.48 mL, 5.91 mmol) to obtain the title compound (1.56 g) (colorless oil).
MS (ESI pos.) m/z: 330[M+Na]+

### Reference Example 103: (2S)-2-(Ethylamino)propyl benzoate

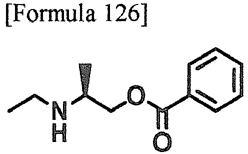

By using (2S)-2-[(tert-butoxycarbonyl)(ethyl)amino]propyl benzoate obtained in Reference Example 102 (0.50 g, 1.63 mmol) as the raw material, the same procedure as in Reference Example 73 was carried out to obtain the title compound (0.20 g) (colorless oil).
MS (ESI pos.) m/z: 208[M+H]+

### Reference Example 104: (2S)-2-{Ethyl[5-methyl-2-(2H-1,2,3-triazol-2-yl)benzoyl]amino}propyl benzoate

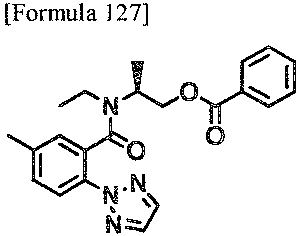

By using (2S)-2-(ethylamino)propyl benzoate obtained in Reference Example 103 (0.39 g, 1.94 mmol) and 5-methyl-2-(2H-1,2,3-triazol-2-yl)benzoic acid (0.44 g, 2.13 mmol) as the raw materials, the same procedure as in Reference Example 10 was carried out to obtain the title compound (0.56 g) (pale yellow solid).
MS (ESI pos.) m/z: 393 [M+H]+

### Reference Example 105-1: N-Ethyl-N-[(2S)-1-hydroxypropan-2-yl]-5-methyl-2-(2H-1,2,3-triazol-2-yl)benzamide

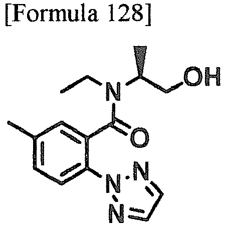

To N-ethyl-N-[(2S)-1-(methoxymethoxy)propan-2-yl]-5-methyl-2-(2H-1,2,3-triazol-2-yl)benzamide obtained in Reference Example 100 (1.34 g, 4.0 mmol), a 4M solution of HCl-dioxane (5.0 mL, 20.0 mmol) was added, and the resulting mixture was stirred at room temperature for 2 hours. The solvent was distilled off under reduced pressure, then a saturated aqueous solution of NaHCO₃ was added to the obtained residue, followed by extraction with chloroform. The organic layer was washed with water and then dried over MgSO₄. The drying agent was filtered off, the solvent was distilled off under reduced pressure, and the obtained residue was purified by column chromatography (SNAP Ultra 50g, hexane/EtOAc = 80/20 to 0/100) to obtain the title compound (0.89 g) (colorless solid).
MS (ESI pos.) m/z: 289 [M+H]+

### Reference Example 105-2: N-Ethyl-N-[(2S)-1-hydroxypropan-2-yl]-5-methyl-2-(2H-1,2,3-triazol-2-yl)benzamide

The title compound can also be obtained by the following procedure. A solution of (2S)-2-{ethyl[5-methyl-2-(2H-1,2,3-triazol-2-yl)benzoyl]amino}propyl benzoate obtained in Reference Example 104 (0.40 g, 1.02 mmol) and a 3M aqueous solution of KOH (0.51 mL, 1.53 mmol) in MeOH (4 mL) was stirred at room temperature for 12 hours. EtOAc was added to the reaction solution, then the resulting mixture was washed with a saturated aqueous solution of NaHCO₃, water, and brine, and then the solvent was distilled off under reduced pressure. The obtained residue was purified by column chromatography (Grace NH 12g, hexane/EtOAc = 84/16 to 0/100) to obtain the title compound (0.29 g) (colorless oil).

### Reference Example 105-3: N-Ethyl-N-[(2S)-1-hydroxypropan-2-yl]-5-methyl-2-(2H-1,2,3-triazol-2-yl)benzamide

The title compound can also be obtained by the following procedure. By using (2S)-2-(ethylamino)propan-1-ol (0.14 g, 1.35 mmol) and 5-methyl-2-(2H-1,2,3-triazol-2-yl)benzoic acid (0.25 g, 1.23 mmol) as the raw materials, the same procedure as in Reference Example 10 was carried out to obtain the title compound (0.31 g) (colorless oil).

### Reference Example 106: N-[(2S)-1-Azidepropan-2-yl]-N-ethyl-5-methyl-2-(2H-1,2,3-triazol-2-yl)benzamide

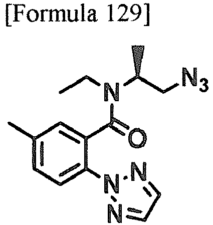

To a solution of N-ethyl-N-[(2S)-1-hydroxypropan-2-yl]-5-methyl-2-(2H-1,2,3-triazol-2-yl)benzamide obtained in Reference Example 105 (0.51 g, 1.77 mmol) in chloroform (10 mL), triethylamine (0.49 mL, 3.54 mmol) and methanesulfonyl chloride (0.21 mL, 2.65 mmol) were added, and then the resulting mixture was stirred at room temperature for 1 hour. A saturated aqueous solution of NaHCO₃ was added to the reaction mixture at 0°C, then the resulting mixture was extracted with chloroform, and then the solvent was distilled off under reduced pressure. Sodium azide (0.13 g, 1.95 mmol) was added to a solution of the obtained residue in DMF (10 mL), and the resulting mixture was heated to 80°C and stirred for 3 hours. The reaction mixture was allowed to cool to room temperature, then water was added thereto, followed by extraction with EtOAc. The organic layer was dried over Na₂SO₄, the drying agent was filtered off, and the solvent was distilled off under reduced pressure to obtain the title compound (0.48 g) (pale yellow oil).
MS (ESI pos.) m/z: 314 [M+H]+

### Reference Example 107: N-[(2S)-1-Chloropropan-2-yl]-N-ethyl-S-methyl-2-(2H-1,2,3-triazol-2-yl)benzamide

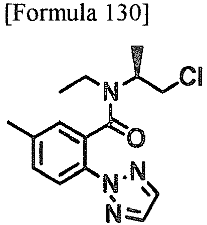

Thionyl chloride (0.03 mL, 0.42 mmol) was added to a solution of N-ethyl-N-[(2S)-1-hydroxypropan-2-yl]-5-methyl-2-(2H-1,2,3-triazol-2-yl)benzamide obtained in Reference Example 105 (0.10 g, 0.35 mmol) in chloroform (1.5 mL), and the resulting mixture was heated to reflux for 5 hours. The solvent was distilled off under reduced pressure before dissolution of the obtained residue in EtOAc. NH silica gel (about 1 g) was added thereto, then the resulting mixture was stirred at room temperature for 10 minutes, then insolubles were filtered off, and then the solvent was concentrated under reduced pressure to obtain the title compound (0.11 g) (colorless oil).
MS (ESI pos.) m/z: 307 [M+H]+

### Reference Example 108: N-[(2S)-1-Cyanopropan-2-yl]-N-ethyl-5-methyl-2-(2H-1,2,3-triazol-2-yl)benzamide

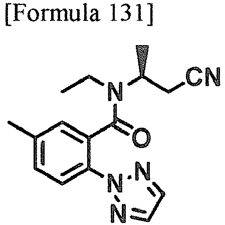

By using N-[(2S)-1-chloropropan-2-yl]-N-ethyl-5-methyl-2-(2H-1,2,3-triazol-2-yl)benzamide obtained in Reference Example 107 (0.11 g, 0.35 mmol) as the raw material, the same procedure as in Reference Example 44 was carried out to obtain the title compound (0.10 g) (colorless oil).
MS (ESI pos.) m/z: 298 [M+H]+

### Reference Example 109: (4S)-2,4-Dimethyl-1,3-oxazolidine

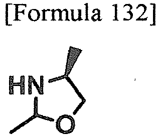

MgSO₄ (12.0 g, 99.9 mmol) was added to a solution of (2S)-2-aminopropan-1-ol (5.0 g, 66.6 mmol) in chloroform (67 mL) under ice-cooling. A 90% aqueous solution of acetaldehyde (3.91 mL, 79.9 mmol) was added dropwise to the obtained suspension, then the resulting mixture was stirred for 30 minutes and then at room temperature overnight. The reaction solution was filtered, and then the filtrate was concentrated under reduced pressure to obtain the title compound (4.0 g) (colorless oil).
MS (ESI pos.) m/z: 102 [M+H]+

### Reference Example 110: (2S)-2-(Ethylamino)propan-1-ol

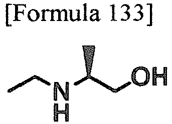

NaBH₄ (0.38 g, 10.1 mmol) was added to a solution of (4S)-2,4-dimethyl-1,3-oxazolidine obtained in Reference Example 109 (1.0 g, 5.07 mmol) in EtOH (5 mL) under ice-cooling, and the resulting mixture was stirred at room temperature for 18 hours. H₂O (1 mL) was added to the reaction solution, then the resulting mixture was filtered, and then the filtrate was concentrated under reduced pressure. The obtained residue was poured into H₂O, followed by extraction with Et₂O, and then the extract was dried over MgSO₄. The drying agent was filtered off, then the solvent was distilled off under reduced pressure to obtain the title compound (0.6 g) (colorless oil).
MS (ESI pos.) m/z: 104 [M+H]+

### Example 1: N-Ethyl-N-{(2S)-1-[3-(5-fluoropyridin-2-yl)-1H-pyrazol-1-yl]propan-2-yl}-5-methyl-2-(2H-1,2,3-triazol-2-yl)benzamide

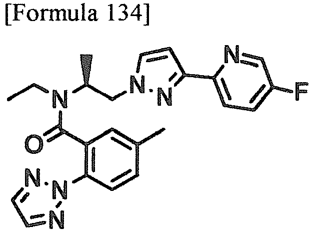

To a solution of (2S)-N-ethyl-1-[3-(5-fluoropyridin-2-yl)-1H-pyrazol-1-yl]propan-2-amine dihydrochloride obtained in Reference Example 8 (0.14 g, 0.44 mmol) in DMF (5.0 mL), 5-methyl-2-(2H-1,2,3-triazol-2-yl)benzoic acid (0.11 g, 0.52 mmol), HATU (0.25 g, 0.65 mmol), and DIPEA(0.68 mL, 3.92 mmol) were added, and the resulting mixture was stirred at room temperature for 24 hours. Water was added to the reaction solution, followed by extraction with EtOAc. The organic layer was washed with a saturated aqueous solution of NaHCO₃ and brine and dried over MgSO₄, then the drying agent was filtered off, and then the solvent was distilled off under reduced pressure. The obtained residue was purified by column chromatography (KP-OH 25g, hexane/EtOAc = 80/20 to 20/80) and HPLC to obtain the title compound (0.027 g) (colorless and amorphous).
LCMS retention time 5.07 min.
MS (ESI pos.) m/z: 434 [M+H]+

Examples 2 to 17 were obtained by the same procedure as in Example 1. The structural formula, the names, and LCMS data of the obtained compounds are shown in Tables 2-1 and 2-2.

**[Table 2-1]**

| Example No. | Structural formula | Compound name | MS (ESI pos.) m/z | LCMS measurement condition Retention time (min) |
|---|---|---|---|---|
| Example 2 | 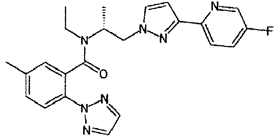 | N-Ethyl-N-{(2R)-1-[3-(5-fluoropyridin-2-yl)-1H-pyrazol-1-yl]propan-2-yl}-5-methyl-2-(2H-1,2,3-triazol-2-yl) benzamide | 434(M+H)+ | Condition 1 5.08 |
| Example 3 | 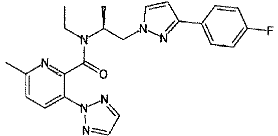 | N-Ethyl-N-{(2S)-1-[3-(4-fluorophenyl)-1H-pyrazol-1-yl]propan-2-yl}-6-methyl -3-(2H-1,2,3-triazol-2-yl)pyridine-2-carboxamide | 434(M+H)+ | Condition 2 0.974, 1.053 |
| Example 4 | 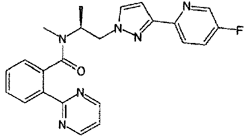 | N-{(2S)-1-[3-(5-Fluoropyridin-2-yl)-1H-pyrazol-1-yl]propan-2-yl}-N-methyl -2-(pyrimidin-2-yl)benzamide | 417(M+H)+ | Condition 2 0.819 |
| Example 5 | 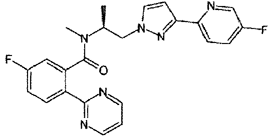 | 5-Fluoro-N-{(2S)-1-[3-(5-fluoropyridin -2-yl)-1H-pyrazol-1-yl]propan-2-yl}-N -methyl-2-(pyrimidin-2-yl)benzamide | 435(M+H)+ | Condition 2 0.733, 0.828, 0.868 |
| Example 6 | 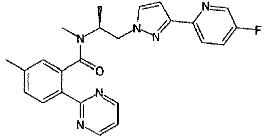 | N-{(2S)-1-[3-(5-Fluoropyridin-2-yl)-1H -pyrazol-1-yl]propan-2-yl}-N,5-dimethyl-2-(pyrimidin-2-yl)benzamide | 431 (M+H)+ | Condition 2 0.781, 0.830, 0.881 |
| Example 7 | 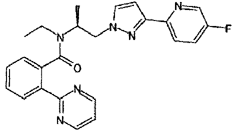 | N-Ethyl-N-{(2S)-1-[3-(5-fluoropyridin-2-yl)-1H-pyrazol-1-yl]propan-2-yl}-2-(pyrimidin-2-yl)benzamide | 431(M+H)+ | Condition 2 0.86 |
| Example 8 | 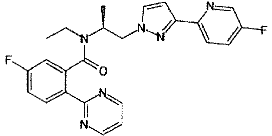 | N-Ethyl-5-fluoro-N-{(2S)-1-[3-(5-fluoropyridin-2-yl)-1H-pyrazol-1-yl] propan-2-yl}-2-(pyrimidin-2-yl) benzamide | 449(M+H)+ | Condition 2 0.814, 0.973 |
| Example 9 | 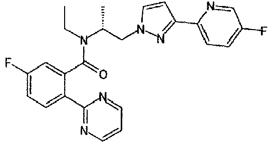 | N-Ethyl-5-fluoro-N-{(2R)-1-[3-(5-fluoropyridin-2-yl)-1H-pyrazol-1-yl] propan-2-yl}-2-(pyrimidin-2-yl) benzamide | 449(M+H)+ | Condition 2 0.98 |

**[Table 2-2]**

| Example No. | Structural formula | Compound name | MS (ESI pos.) m/z | LCMS measurement condition Retention time (min) |
|---|---|---|---|---|
| Example 10 | 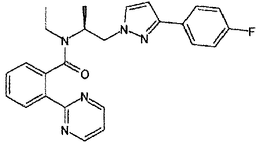 | N-Ethyl-N-{(2S)-1-[3-(4-fluorophenyl)-1H-pyrazol-1-yl]propan-2-yl}-2-(pyrimidin-2-yl)benzamide | 430(M+H)+ | Condition 2 1.007 |
| Example 11 | 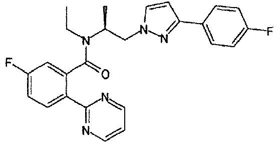 | N-Ethyl-5-fluoro-N-{(2S)-1-[3-(4-fluorophenyl)-1H-pyrazol-1-yl] propan-2-yl}-2-(pyrimidin-2-yl) benzamide | 448(M+H)+ | Condition 2 0.962, 1.087 |
| Example 12 | 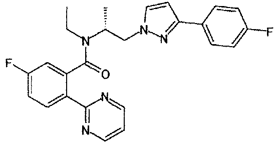 | N-Ethyl-5-fluoro-N-{(2R)-1-[3-(4-fluorophenyl)-1H-pyrazol-1-yl]propan-2-yl}-2-(pyrimidin-2-yl)benzamide | 448(M+H)+ | Condition 2 1.1 |
| Example 13 | 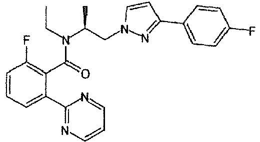 | N-Ethyl-2-fluoro-N-{(2S)-1-[3-(4-fluorophenyl)-1H-pyrazol-1-yl]propan-2-yl}-6-(pyrimidin-2-yl)benzamide | 448(M+H)+ | Condition 2 0.981, 1.038, 1.096 |
| Example 14 | 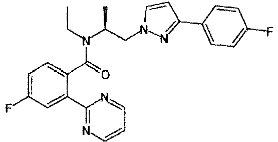 | N-Ethyl-4-fluoro-N-{(2S)-1-[3-(4-fluorophenyl)-1H-pyrazol-1-yl]propan-2-yl}-2-(pyrimidin-2-yl)benzamide | 448(M+H)+ | Condition 2 1.052 |
| Example 15 | 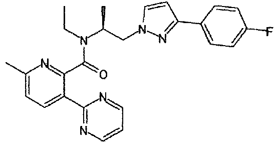 | N-Ethyl-N-{(2S)-1-[3-(4-fluorophenyl)-1H-pyrazol-1-yl]propan-2-yl}-6-methyl -3-(pyrimidin-2-yl)pyridine-2-carboxamide | 445(M+H)+ | Condition 2 0.943, 1.036 |
| Example 16 | 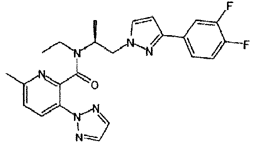 | N-{(2S)-1-[3-(3,4-Difluorophenyl)-1H-pyrazol-1-yl]propan-2-yl}-N-ethyl-6-methyl-3-(2H-1,2,3-triazol-2-yl)pyridine -2-carboxamide | 452(M+H)+ | Condition 2 1.04, 1.12 |
| Example 17 | 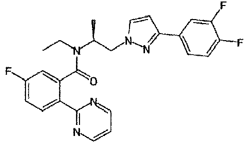 | N-{(2S)-1-[3-(3,4-Difluorophenyl)-1H-pyrazol-1-yl]propan-2-yl}-N-ethyl-5-fluoro-2-(pyrimidin-2-yl)benzamide | 466(M+H)+ | Condition 2 1.13 |

### Example 18: N-Ethyl-N- {(2S)-1-[3-(5-fluoropyridin-2-yl)-1H-pyrazol-1-yl]propan-2-yl}-2-(2H-1,2,3-triazol-2-yl)benzamide

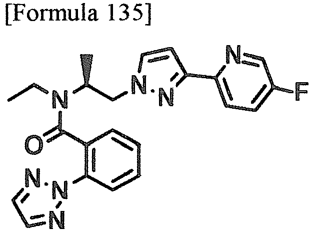

To a solution of N-{(2S)-1-[3-(5-fluoropyridin-2-yl)-1H-pyrazol-1-yl]propan-2-yl}-2-(2H-1,2,3-triazol-2-yl)benzamide obtained in Reference Example 10 (0.13 g, 0.33 mmol) in DMF (5.0 mL), 60% NaH (0.020 g, 0.50 mmol) was added, and the resulting mixture was stirred at room temperature for 30 minutes. EtI (0.032 mL, 0.40 mmol) was added to the reaction solution, then the resulting mixture was stirred at room temperature for 24 hours. Water was added to the reaction solution, followed by extraction with EtOAc. The organic layer was washed with a saturated aqueous solution of NaHCO₃ and brine and dried over MgSO₄, then the drying agent was filtered off, and then the solvent was distilled off under reduced pressure. The obtained residue was purified by column chromatography (KP-NH 28g, hexane/EtOAc = 80/20 to 40/60) to obtain the title compound (0.058 g) (colorless and amorphous).
LCMS retention time 4.82 min.
MS (ESI pos.) m/z: 420 [M+H]+

Examples 19 to 33 were obtained by the same procedure as in Example 18. The structural formula, the names, and LCMS data of the obtained compounds are shown in Tables 3-1 and 3-2.

**[Table 3-1]**

| Example No. | Structural formula | Compound name | MS (ESI pos.) m/z | LCMS measurement condition Retention time (min) |
|---|---|---|---|---|
| Example 19 | 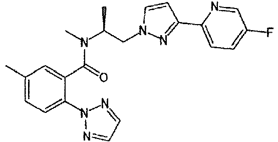 | N-{(2S)-1-[3-(5-Fluoropyridin-2-yl)-1H-pyrazol-1-yl]propan-2-yl}-N,5-dimethyl-2-(2H-1,2,3-triazol-2-yl) benzamide | 420(M+H)+ | Condition 1 4.78 |
| Example 20 | 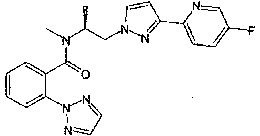 | N-{(2S)-1-[3-(5-Fluoropyridin-2-yl)-1H-pyrazol-1-yl]propan-2-yl}-N-methyl -2-(2H-1,2,3-triazol-2-yl)benzamide | 406(M+H)+ | Condition 1 4.63 |
| Example 21 | 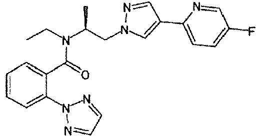 | N-Ethyl-N-{(2S)-1-[4-(5-fluoropyridin-2-yl)-1H-pyrazol-1-yl]propan-2-yl}-2-(2H-1,2,3-triazol-2-yl)benzamide | 420(M+H)+ | Condition 1 4.65 |
| Example 22 | 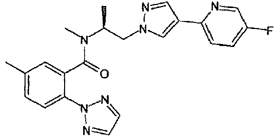 | N-{(2S)-1-[4-(5-Fluoropyridin-2-yl)-1H-pyrazol-1-yl]propan-2-yl}-N,5-dimethyl-2-(2H-1,2,3-triazol-2-yl) benzamide | 420(M+H)+ | Condition 1 4.56 |
| Example 23 | 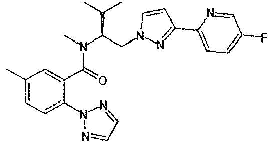 | N-{(2S)-1-[3-(5-Fluoropyridin-2-yl)-1H-pyrazol-1-yl]-3-methylbutan-2-yl}-N,5-dimethyl-2-(2H-1,2,3-triazol-2-yl) benzamide | 448(M+H)+ | Condition 2 1.03 |
| Example 24 | 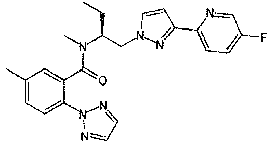 | N-{(2S)-1-[3-(5-Fluoropyridin-2-yl)-1H-pyrazol-1-yl]butan-2-yl}-N,5-dimethyl-2-(2H-1,2,3-triazol-2-yl) benzamide | 434(M+H)+ | Condition 2 0.98 |
| Example 25 | 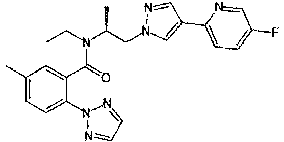 | N-Ethyl-N-{(2S)-1-[4-(5-fluoropyridin-2-yl)-1H-pyrazol-1-yl]propan-2-yl}-5-methyl-2-(2H-1,2,3-triazol-2-yl) benzamide | 434(M+H)+ | Condition 2 0.91 |
| Example 26 | 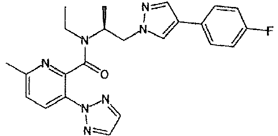 | N-Ethyl-N-{(2S)-1-[4-(4-fluorophenyl)-1H-pyrazol-1-yl]propan-2-yl}-6-methyl -3-(2H-1,2,3-triazol-2-yl)pyridine-2-carboxamide | 434(M+H)+ | Condition 2 0.958, 1.039 |

**[Table 3-2]**

| Example No. | Structural formula | Compound name | MS (ESI pos.) m/z | LCMS measurement condition Retention time (min) |
|---|---|---|---|---|
| Example 27 | 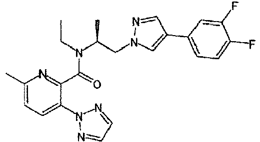 | N-{(2S)-1-[4-(3,4-Difluorophenyl)-1H-pyrazol-1-yl]propan-2-yl}-N-ethyl-6-methyl-3-(2H-1,2,3-triazol-2-yl) pyridine-2-carboxamide | 452(M+H)+ | Condition 2 0.984, 1.070 |
| Example 28 | 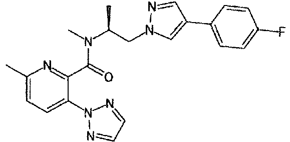 | N-{(2S)-1-[4-(4-Fluorophenyl)-1H-pyrazol-1-yl]propan-2-yl}-N,6-dimethyl -3-(2H-1,2,3-triazol-2-yl)pyridine-2-carboxamide | 420(M+H)+ | Condition 2 0.885, 0.927 |
| Example 29 | 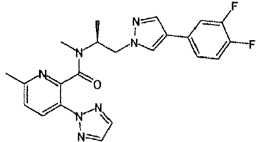 | N-{(2S)-1-[4-(3,4-Difluorophenyl)-1H-pyrazol-1-yl]propan-2-yl}-N,6-dimethyl -3-(2H-1,2,3-triazol-2-yl)pyridine-2-carboxamide | 438(M+H)+ | Condition 2 0.921. 0.963 |
| Example 30 | 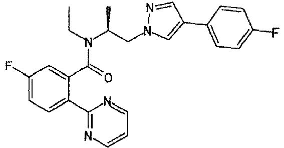 | N-Ethyl-5-fluoro-N-{(2S)-1-[4-(4-fluorophenyl)-1H-pyrazol-1-yl]propan-2-yl}-2-(pyrimidin-2-yl)benzamide | 448(M+H)+ | Condition 2 1.027 |
| Example 31 | 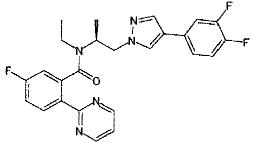 | N-{(2S)-1-[4-(3,4-Difluorophenyl)-1H-pyrazol-1-yl]propan-2-yl}-N-ethyl-5-fluoro-2-(pyrimidin-2-yl)benzamide | 466(M+H)+ | Condition 2 1.062 |
| Example 32 | 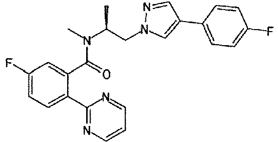 | 5-Fluoro-N-{(2S)-1-[4-(4-fluorophenyl) -1H-pyrazol-1-yl]propan-2-yl}-N-methyl-2-(pyrimidin-2-yl)benzamide | 434(M+H)+ | Condition 2 0.967 |
| Example 33 | 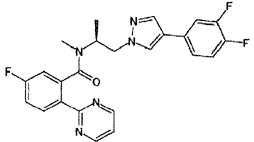 | N-{(2S)-1-[4-(3,4-Difluorophenyl)-1H-pyrazol-1-yl]propan-2-yl}-5-fluoro-N-methyl-2-(pyrimidin-2-yl)benzamide | 452(M+H)+ | Condition 2 0.995 |

All of the LCMS measurement operations shown below were carried out under condition 2.

### Example 34: N-Ethyl-5-fluoro-N-{(2S)-1-[4-(5-fluoropyridin-2-yl)-1H-pyrazol-1-yl]propan-2-yl}-2-(pyrimidin-2-yl)benzamide

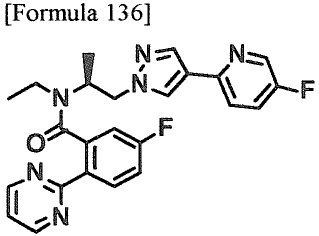

To a solution of 5-fluoro-N-{(2S)-1-[4-(5-fluoropyridin-2-yl)-1H-pyrazol-1-yl]propan-2-yl}-2-(pyrimidin-2-yl)benzamide obtained in Reference Example 81 (0.028 g, 0.067 mmol) in DMF (1.5 mL), 60% NaH (0.0039 g, 0.10 mmol) was added, and the resulting mixture was stirred at room temperature for 10 minutes. EtI (0.0064 ml, 0.080 mmol) was added to the reaction solution, and the resulting mixture was stirred at room temperature for 1.5 hours. 60% NaH (0.0039 g, 0.10 mmol) was added to the reaction solution, the mixture was stirred at room temperature for 10 minutes, then EtI (0.0064 ml, 0.080 mmol) was added thereto, and the resulting mixture was stirred at room temperature for 16 hours. A saturated aqueous solution of NH₄Cl was added to the reaction solution, followed by extraction with chloroform. The organic layer was concentrated under reduced pressure, and the obtained residue was purified by column chromatography (KP-NH 11g, hexane/EtOAc = 88/12 to 0/100). The obtained oil was freeze-dried to obtain the title compound (0.019 g) (colorless and amorphous).
LCMS retention time 0.94 min.
MS (ESI pos.) m/z: 449 [M+H]+

Examples 35 to 40 were obtained by the same procedure as in Example 34. The structural formula, the names, and LCMS data of the obtained compounds are shown in Table 4.

**[Table 4]**

| Example No. | Structural formula | Compound name | MS (ESI pos.) m/z | Retention time (min) |
|---|---|---|---|---|
| Example 35 | 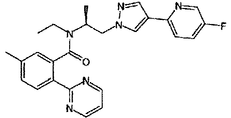 | N-Ethyl-N-{(2S)-1-[4-(5-fluoropyridin-2-yl)-1H-pyrazol-1-yl]propan-2-yl}-5-methyl-2-(pyrimidin-2-yl)benzamide | 445(M+H)+ | 0.960 |
| Example 36 | 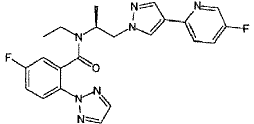 | N-Ethyl-5-fluoro-N-{(2S)-1-[4-(5-fluoropyridin-2-yl)-1H-pyrazol-1-yl] propan-2-yl}-2-(2H-1,2,3-triazol-2-yl) benzamide | 438(M+H)+ | 0.894 |
| Example 37 | 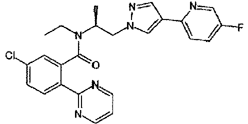 | 5-Chloro-N-ethyl-N-{(2S)-1-[4-(5-fluoropyridin-2-yl)-1H-pyrazol-1-yl] propan-2-yl}-2-(pyrimidin-2-yl) benzamide | 465(M+H)+ | 1.004 |
| Example 38 | 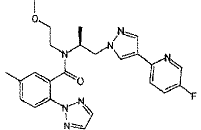 | N-{(2S)-1-[4-(5-Fluoropyridin-2-yl)-1H-pyrazol-1-yl]propan-2-yl}-N-(2-methoxyethyl)-5-methyl-2-(2H-1,2,3-triazol-2-yl)benzamide | 464(M+H)+ | 0.896 |
| Example 39 | 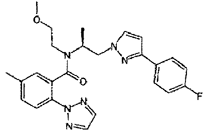 | N-{(2S)-1-[3-(4-Fluorophenyl)-1H-pyrazol-1-yl]propan-2-yl}-N-(2-methoxyethyl)-5-methyl-2-(2H-1,2,3-triazol-2-yl)benzamide | 463(M+H)+ | 1.071 |
| Example 40 | 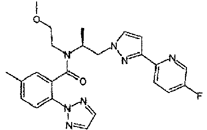 | N-{(2S)-1-[3-(5-Fluoropyridin-2-yl)-1H-pyrazol-1-yl]propan-2-yl}-N-(2-methoxyethyl-5-methyl-2-(2H-1,2,3-triazol-2-yl)benzamide | 464(M+H)+ | 0.928 |

### Example 41: N-{(2S)-1-[4-(5-Fluoropyridin-2-yl)-1H-pyrazol-1-yl]propan-2-yl}-N-(2-hydroxyethyl)-5-methyl-2-(2H-1,2,3-triazol-2-yl)benzamide

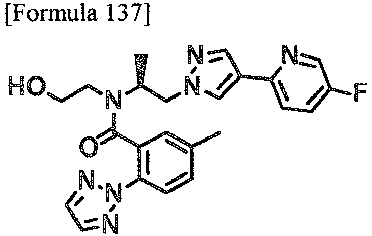

To a solution of N-{(2S)-1-[4-(5-fluoropyridin-2-yl)-1H-pyrazol-1-yl]propan-2-yl}-5-methyl-2-(2H-1,2,3-triazol-2-yl)benzamide obtained in Reference Example 15 (0.080 g, 0.20 mmol) in DMF (2.0 mL), 60% NaH (0.009 g, 0.22 mmol) was added, and the resulting mixture was stirred at room temperature for 30 minutes. To the reaction solution, 2-(2-bromoethoxy)tetrahydro-2H-pyran (0.0036 ml, 0.24 mmol) was added, and the resulting mixture was stirred at room temperature for 60 hours. To the reaction solution, 60% NaH (0.044 g, 1.10 mmol) and 2-(2-bromoethoxy)tetrahydro-2H-pyran (0.182 ml, 1.20 mmol) were added, and the resulting mixture was stirred at room temperature for 16 hours. Water was added to the reaction solution, followed by extraction with EtOAc. The organic layer was washed with brine and then dried over Na₂SO₄. The drying agent was filtered off, and then the solvent was distilled off under reduced pressure. The obtained residue was purified by column chromatography (SNAP Ultra 10g, hexane/EtOAc = 80/20 to 0/100) to obtain the title compound (0.067 g) (colorless and amorphous). The obtained amorphous was dissolved in MeOH (0.31 mL), then a 4M solution of HCl-EtOAc (0.047 mL, 0.19 mmol) was added thereto, and the resulting mixture was stirred at room temperature for 2 hours. The solvent was distilled off under reduced pressure, followed by extraction with EtOAc. The organic layer was washed with brine and then dried over Na₂SO₄. The drying agent was filtered off, and then the solvent was distilled off under reduced pressure. The obtained residue was purified by column chromatography (SNAP Ultra 10g, hexane/EtOAc = 80/20 to 0/100). Et₂O was added to the obtained oil for precipitation of a solid, and the precipitated solid was filtered out to obtain the title compound (0.045 g) (colorless solid).
LCMS retention time 0.758 min.
MS (ESI pos.) m/z: 450 [M+H]+

Examples 42 to 44 were obtained by the same procedure as in Example 41. The structural formula, the names, and LCMS data of the obtained compounds are shown in Table 5.

**[Table 5]**

| Example No. | Structural formula | Compound name | MS (ESI pos.) m/z | Retention time (min) |
|---|---|---|---|---|
| Example 42 | 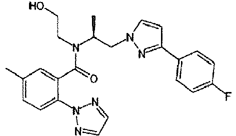 | N-{(2S)-1-[3-(4-Fluorophenyl)-1H-pyrazol-1-yl]propan-2-yl}-N-(2-hydroxyethyl)-5-methyl-2-(2H-1,2,3-triazol-2-yl)benzamide | 449(M+H)+ | 0.926 |
| Example 43 | 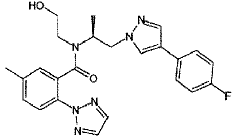 | N-{(2S)-1-[4-(4-Fluorophenyl)-1H-pyrazol-1-yl]propan-2-yl}-N-(2-hydroxyethyl)-5-methyl-2-(2H-1,2,3-triazol-2-yl)benzamide | 449(M+H)+ | 0.908 |
| Example 44 | 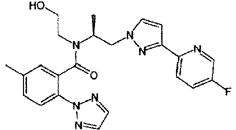 | N-{(2S)-1-[3-(5-Fluoropyridin-2-yl)-1H-pyrazol-1-yl]propan-2-yl}-N-(2-hydroxyethyl)-5-methyl-2-(2H-1,2,3-triazol-2-yl)benzamide | 450(M+H)+ | 0.787 |

### Example 45: N-{(2S)-1-[5-(5-Fluoropyridin-2-yl)-1,2,4-oxadiazol-3-yl]propan-2-yl}-N,5-dimethyl-2-(2H-1,2,3-triazol-2-yl)benzamide

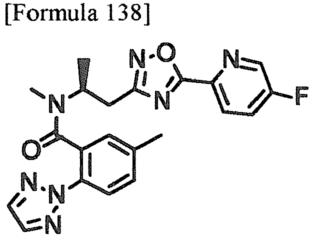

To a solution of (2S)-1-[5-(5-fluoropyridin-2-yl)-1,2,4-oxadiazol-3-yl]-N-methylpropan-2-amine obtained in Reference Example 47 (0.037 g, 0.16 mmol) in DMF (1.5 mL), 5-methyl-2-(2H-1,2,3-triazol-2-yl)benzoic acid (0.032 g, 0.16 mmol), HATU (0.071 g, 0.19 mmol), and DIPEA (0.082 ml, 0.47 mmol) were added, and the resulting mixture was stirred at room temperature for 60 hours. Water was added to the reaction solution, followed by extraction with chloroform. The solvent was distilled off under reduced pressure, and the obtained residue was purified by HPLC and PLC (NH silica 1.0 mm, hexane/EtOAc = 1/1). The obtained oil was freeze-dried to obtain the title compound (0.023 g) (colorless and amorphous).
LCMS retention time 0.841, 0.925 min.
MS (ESI pos.) m/z: 422 [M+H]+

Examples 46 to 61 were obtained by the same procedure as in Example 45. The structural formula, the names, and LCMS data of the obtained compounds are shown in Tables 6-1 to 6-3.

**[Table 6-1]**

| Example No. | Structural formula | Compound name | MS (ESI pos.) m/z | Retention time (min) |
|---|---|---|---|---|
| Example 46 | 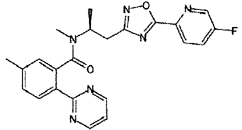 | N-{(2S)-1-[5-(5-Fluoropyridin-2-yl)-1,2,4-oxadiazol-3-yl]propan-2-yl}-N,5-dimethyl-2-(pyrimidin-2-yl)benzamide | 433(M+H)+ | 0.799, 0.826, 0.901 |
| Example 47 | 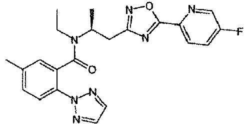 | N-Ethyl-N-{(2S)-1-[5-(5-fluoropyridin-2-yl)-1,2,4-oxadiazol-3-yl]propan-2-yl} -5-methyl-2-(2H-1,2,3-triazol-2-yl) benzamide | 436(M+H)+ | 0.940 |
| Example 48 | 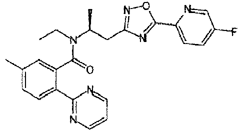 | N-Ethyl-N-{(2S)-1-[5-(5-fluoropyridin-2-yl)-1,2,4-oxadiazol-3-yl]propan-2-yl} -5-methyl-2-(pyrimidin-2-yl)benzamide | 447(M+H)+ | 0.902 |
| Example 49 | 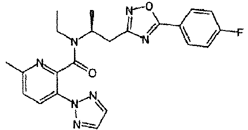 | N-Ethyl-N-{(2S)-1-[5-(4-fluorophenyl)-1,2,4-oxadiazol-3-yl]propan-2-yl}-6-methyl-3-(2H-1,2,3-triazol-2-yl)pyridine -2-carboxamide | 436(M+H)+ | 0.988, 1.046 |
| Example 50 | 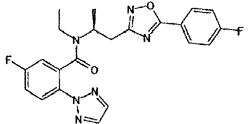 | N-Ethyl-5-fluoro-N-{(2S)-1-[5-(4-fluorophenyl)-1,2,4-oxadiazol-3-yl] propan-2-yl}-2-(2H-1,2,3-triazol-2-yl) benzamide | 439(M+H)+ | 1.069 |
| Example 51 | 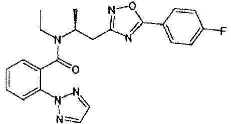 | N-Ethyl-N-{(2S)-1-(5-(4-fluorophenyl)-1,2,4-oxadiazol-3-yl]propan-2-yl}-2-(2H-1,2,3-triazol-2-yl)benzamide | 421(M+H)+ | 1.038 |

**[Table 6-2]**

| Example No. | Structural formula | Compound name | MS (ESI pos.) m/z | Retention time (min) |
|---|---|---|---|---|
| Example 52 | 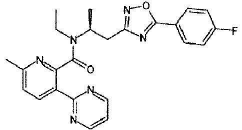 | N-Ethyl-N-{(2S)-1-[5-(4-fluorophenyl)-1,2,4-oxadiazol-3-yl]propan-2-yl}-6-methyl-3-(pyrimidin-2-yl)pyridine-2-carboxamide | 447(M+H)+ | 0.946. 1.021 |
| Example 53 | 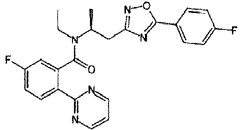 | N-Ethyl-5-fluoro-N-{(2S)-1-[5-(4-fluorophenyl)-1,2,4-oxadiazol-3-yl] propan-2-yl}-2-(pyrimidin-2-yl) benzamide | 450(M+H)+ | 1.054 |
| Example 54 | 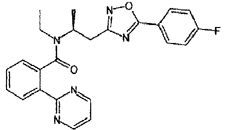 | N-Ethyl-N-{(2S)-1-[5-(4-fluorophenyl)-1,2,4-oxadiazol-3-yl]propan-2-yl}-2-(pyrimidin-2-yl)benzamide | 432(M+H)+ | 1.007 |
| Example 55 | 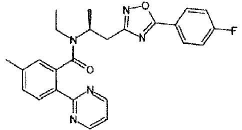 | N-Ethyl-N-{(2S)-1-[5-(4-fluorophenyl)-1,2,4-oxadiazol-3-yl]propan-2-yl}-5-methyl-2-(pyrimidin-2-yl)benzamide | 446(M+H)+ | 1.065 |
| Example 56 | 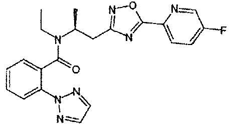 | N-Ethyl-N-{(2S)-1-[5-(5-fluoropyridin-2-yl)-1,2,4-oxadiazol-3-yl]propan-2-yl} -2-(2H-1,2,3-triazol-2-yl)benzamide | 422(M+H)+ | 0.883 |
| Example 57 | 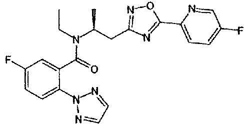 | N-Ethyl-5-fluoro-N-{(2S)-1-[5-(5-fluoropyridin-2-yl)-1,2,4-oxadiazol-3-yl] propan-2-yl}-2-(2H-1,2,3-triazol-2-yl) benzamide | 440(M+H)+ | 0.917 |

**[Table 6-3]**

| Example No. | Structural formula | Compound name | MS (ESI pos.) m/z | Retention time (min) |
|---|---|---|---|---|
| Example 58 | 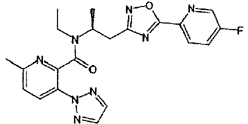 | N-Ethyl-N-{(2S)-1-[5-(5-fluoropyridin-2-yl)-1,2,4-oxadiazol-3-yl]propan-2-yl} -6-methyl-3-(2H-1,2,3-triazol-2-yl) pyridine-2-carboxamide | 437(M+H)+ | 0.805, 0.878 |
| Example 59 | 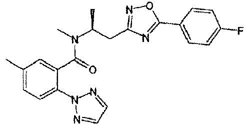 | N-{(2S)-1-[5-(4-Fluorophenyl)-1,2,4-oxadiazol-3-yl]propan-2-yl}-N,5-dimethyl-2-(2H-1,2,3-triazol-2-yl) benzamide | 421(M+H)+ | 1.064 |
| Example 60 | 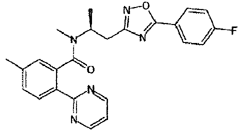 | N-{(2S)-1-[5-(4-Fluorophenyl)-1,2,4-oxadiazol-3-yl]propan-2-yl}-N,5-dimethyl-2-(pyrimidin-2-yl)benzamide | 432(M+H)+ | 1.037 |
| Example 61 | 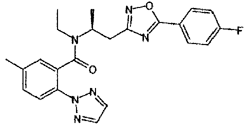 | N-Ethyl-N-{(2S)-1-[5-(4-fluorophenyl)-1,2,4-oxadiazol-3-yl]propan-2-yl}-5-methyl-2-(2H-1,2,3-triazol-2-yl) benzamide | 435(M+H)+ | 1.098 |

### Example 47-2: N-Ethyl-N-{(2S)-1-[5-(5-fluoropyridin-2-yl)-1,2,4-oxadiazol-3-yl]propan-2-yl}-5-methyl-2-(2H-1,2,3-triazol-2-yl)benzamide

The title compound can also be obtained by the following procedure. By using N-[(2S)-1-cyanopropan-2-yl]-N-ethyl-5-methyl-2-(2H-1,2,3-triazol-2-yl)benzamide obtained in Reference Example 108 (0.099 g, 0.33 mmol) and 5-fluoropyridine-2-carboxylic acid (0.064 g, 0.36 mmol) as the raw materials, the same procedure as in Reference Example 45 was carried out to obtain the title compound (0.070 g) (colorless oil).

### Example 62: N-Ethyl-N-{(2S)-1-[5-(4-fluorophenyl)-2H-tetrazol-2-yl]propan-2-yl}-5-methyl-2-(2H-1,2,3-triazol-2-yl)benzamide

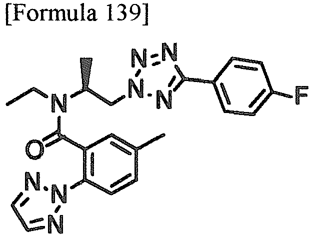

By using N-{(2S)-1-[5-(4-fluorophenyl)-2H-tetrazol-2-yl]propan-2-yl}-5-methyl-2-(2H-1,2,3-triazol-2-yl)benzamide obtained in Reference Example 74 (0.040 g, 0.098 mmol) as the raw material, the same procedure as in Example 34 was carried out to obtain the title compound (0.025 g) (pale yellow solid).
LCMS retention time 0.710 min.
MS (ESI pos.) m/z: 435 [M+H]+

Example 63 was obtained by the same procedure as in Example 62. The structural formula, the name, and LCMS data of the obtained compound are shown in Table 7.

**[Table 7]**

| Example No. | Structural formula | Compound name | MS (ESI pos.) m/z | Retention time (min) |
|---|---|---|---|---|
| Example 63 | 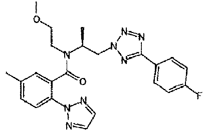 | N-{(2S)-1-[5-(4-Fluorophenyl)-2H-tetrazol-2-yl]propan-2-yl}-N-(2-methoxyethyl)-5-methyl-2-(2H-1,2,3-triazol-2-yl)benzamide | 465(M+H)+ | 1.084 |

### Example 64: N-{(2S)-1-[5-(4-Fluorophenyl)-2H-tetrazol-2-yl]propan-2-yl}-N-(2-hydroxyethyl)-5-methyl-2-(2H-1,2,3-triazol-2-yl)benzamide

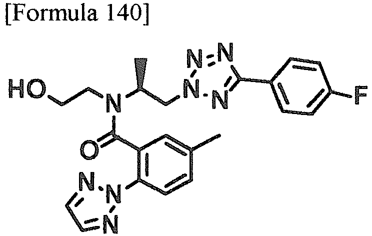

By using N-{(2S)-1-[5-(4-fluorophenyl)-2H-tetrazol-2-yl]propan-2-yl}-5-methyl-2-(2H-1,2,3-triazol-2-yl)benzamide obtained in Reference Example 74 (0.070 g, 0.17 mmol) as the raw material, the same procedure as in Example 41 was carried out to obtain the title compound (0.008 g) (pale yellow oil).
LCMS retention time 0.914 min.
MS (ESI pos.) m/z: 451 [M+H]+

### Example 65: N-Ethyl-N-{(2S)-1-[5-(4-fluorophenyl)-2H-tetrazol-2-yl]propan-2-yl}-6-methyl-3-(2H-1,2,3-triazol-2-yl)pyridine-2-carboxamide

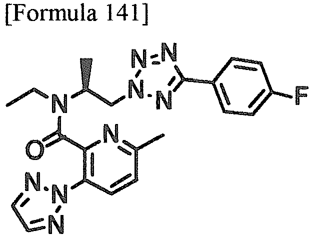

By using (2S)-N-ethyl-1-[5-(4-fluorophenyl)-2H-tetrazol-2-yl]propan-2-amine obtained in Reference Example 76 (0.050 g, 0.20 mmol) and 6-methyl-3-(2H-1,2,3-triazol-2-yl)pyridine-2-carboxylic acid (0.045 g, 0.22 mmol) as the raw materials, the same procedure as in Example 45 was carried out to obtain the title compound (0.0042 g) (colorless solid). LCMS retention time 0.500, 0.545 min.
MS (ESI pos.) m/z: 436 [M+H]+

Examples 66 to 70 were obtained by the same procedure as in Example 65. The structural formula, the names, and LCMS data of the obtained compounds are shown in Table 8.

**[Table 8]**

| Example No. | Structural formula | Compound name | MS (ESI pos.) m/z | Retention time (min) |
|---|---|---|---|---|
| Example 66 | 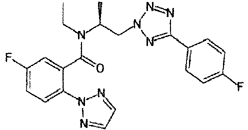 | N-Ethyl-5-fluoro-N-{(2S)-1-[5-(4-fluorophenyl)-2H-tetrazol-2-yl]propan-2-yl}-2-(2H-1,2,3-triazol-2-yl) benzamide | 439(M+H)+ | 0.560, 0.577 |
| Example 67 | 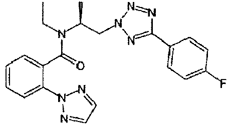 | N-Ethyl-N-{(2S)-1-[5-(4-fluorophenyl)-2H-tetrazol-2-yl]propan-2-yl}-2-(2H-1,2,3-triazol-2-yl)benzamide | 421(M+H)+ | 0.564 |
| Example 68 | 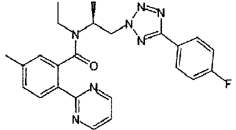 | N-Ethyl-N-{(2S)-1-[5-(4-fluorophenyl)-2H-tetrazol-2-yl]propan-2-yl}-5-methyl-2-(pyrimidin-2-yl)benzamide | 446(M+H)+ | 0.583 |
| Example 69 | 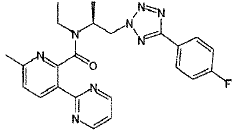 | N-Ethyl-N-{(2S)-1-[5-(4-fluorophenyl)-2H-tetrazol-2-yl]propan-2-yl}-6-methyl-3-(pyrimidin-2-yl)pyridine-2-carboxamide | 447(M+H)+ | 0.480, 0.533 |
| Example 70 | 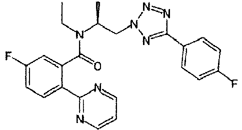 | N-Ethyl-5-fluoro-N-{(2S)-1-[5-(4-fluorophenyl)-2H-tetrazol-2-yl]propan-2-yl}-2-(pyrimidin-2-yl)benzamide | 450(M+H)+ | 0.556 |

### Example 71: N-Ethyl-N-{(2S)-1-[4-(4-fluorophenyl)-1H-1,2,3-triazol-1-yl]propan-2-yl}-5-methyl-2-(2H-1,2,3-triazol-2-yl)benzamide

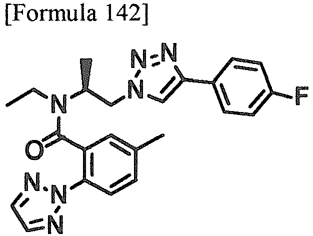

By using N-{(2S)-1-[4-(4-fluorophenyl)-1H-1,2,3-triazol-1-yl]propan-2-yl}-5-methyl-2-(2H-1,2,3-triazol-2-yl)benzamide obtained in Reference Example 79 (0.10 g, 0.25 mmol) as the raw material, the same procedure as in Example 34 was carried out to obtain the title compound (0.042 g) (pale yellow solid).
LCMS retention time 0.989 min.
MS (ESI pos.) m/z: 434 [M+H]+

Example 72 was obtained by the same procedure as in Example 71. The structural formula, the name, and LCMS data of the obtained compound are shown in Table 9.

**[Table 9]**

| Example No. | Structural formula | Compound name | MS (ESI pos.) m/z | Retention time (min) |
|---|---|---|---|---|
| Example 72 | 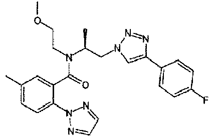 | N-{(2S)-1-[4-(4-Fluorophenyl-1H-1,2,3-triazol-1-yl]propan-2-yl}-N-(2-methoxyethyl)-5-methyl-2-(2H-1,2,3-triazol-2-yl)benzamide | 464(M+H)+ | 0.971 |

### Example 73: N-{(2S)-1-[4-(4-Fluorophenyl)-1H-1,2,3-triazol-1-yl]propan-2-yl]-N-(2-hydroxyethyl)-5-methyl-2-(2H-1,2,3-triazol-2-yl)benzamide

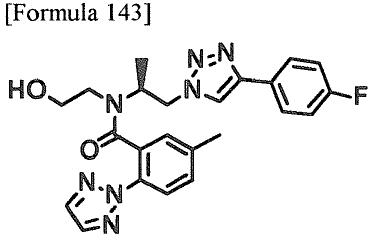

By using N-{(2S)-1-[4-(4-fluorophenyl)-1H-1,2,3-triazol-1-yl]propan-2-yl}-5-methyl-2-(2H-1,2,3-triazol-2-yl)benzamide obtained in Reference Example 79 (0.18 g, 0.44 mmol) as the raw material, the same procedure as in Example 41 was carried out to obtain the title compound (0.10 g) (colorless solid).
LCMS retention time 0.823 min.
MS (ESI pos.) m/z: 450 [M+H]+

### Example 74: N-Ethyl-N-{1-[1-(5-fluoropyridin-2-yl)-1H-pyrazol-4-yl]propan-2-yl}-5-methyl-2-(2H-1,2,3 -triazol-2-yl)benzamide

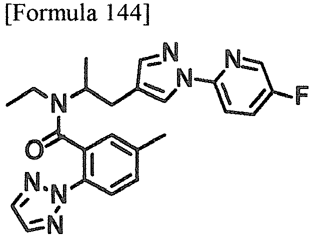

By using N-{1-[1-(5-fluoropyridin-2-yl)-1H-pyrazol-4-yl]propan-2-yl}-5-methyl-2-(2H-1,2,3-triazol-2-yl)benzamide obtained in Reference Example 61 (0.005 g, 0.012 mmol) as the raw material, the same procedure as in Example 34 was carried out to obtain the title compound (0.003 g) (colorless and amorphous).
LCMS retention time 1.173 min.
MS (ESI pos.) m/z: 434 [M+H]+

Example 75 was obtained by the same procedure as in Example 74. The structural formula, the name, and LCMS data of the obtained compound are shown in Table 10.

**[Table 10]**

| Example No. | Structural formula | Compound name | MS (ESI pos.) m/z | Retention time (min) |
|---|---|---|---|---|
| Example 75 | 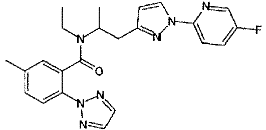 | N-Ethyl-N-{1-[1-(5-fluoropyridin-2-yl)-1H-pyrazol-3-yl]propan-2-yl} -5-methyl-2-(2H-1,2,3-triazol-2-yl) benzamide | 434(M+H)+ | 1.072 |

### Example 76: N-Ethyl-N-{(2S)-1-[5-(4-fluorophenyl)-1,3,4-oxadiazol-2-yl]propan-2-yl}-5-methyl-2-(2H-1,2,3-triazol-2-yl)benzamide

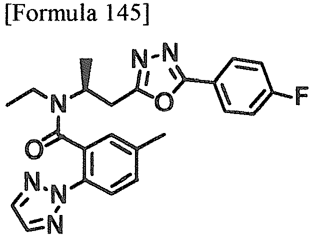

A solution of N-ethyl-N-{(2S)-4-[2-(4-fluorobenzoyl)hydrazinyl]-4-oxobutan-2-yl}-5-methyl-2-(2H-1,2,3-triazol-2-yl)benzamide obtained in Reference Example 71 (0.026 g, 0.057 mmol) and 3,3,3-triethyl-1-(methoxycarbonyl)diazathian-3-ium-1-ide 2,2-dioxide (0.041 g, 0.17 mmol) in THF (1 mL) was stirred at 50°C for 1.5 hours. To the reaction solution, 3,3,3-triethyl-1-(methoxycarbonyl)diazathian-3-ium-1-ide 2,2-dioxide (0.041 g, 0.17 mmol) was added, and the resulting mixture was stirred at 50°C for 1.5 hours. The reaction mixture was allowed to cool to room temperature, then the solvent was distilled off under reduced pressure, and then the obtained residue was purified by column chromatography (SNAP Ultra 10g, hexane/EtOAc = 75/25 to 0/100). The obtained oil was freeze-dried to obtain the title compound (0.025 g) (colorless and amorphous).
LCMS retention time 1.059 min.
MS (ESI pos.) m/z: 435 [M+H]+

### Example 77: N-Ethyl-N-{(2S)-1-[4-(5-fluoropyridin-2-yl)-1H-1,2,3-triazol-1-yl]propan-2-yl}-6-methyl-3-(2H-1,2,3-triazol-2-yl)pyridine-2-carboxamide

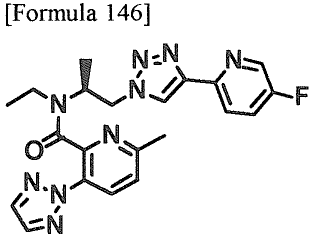

By using (2S)-N-ethyl-1-[4-(5-fluoropyridin-2-yl)-1H-1,2,3-triazol-1-yl]propan-2-amine obtained in Reference Example 89 (0.030 g, 0.12 mmol) as the raw material, the same procedure as in Example 45 was carried out to obtain the title compound (0.028 g) (colorless solid).
LCMS retention time 0.374, 0.438 min.
MS (ESI pos.) m/z: 436 [M+H]+

Examples 78 to 84 were obtained by the same procedure as in Example 77. The structural formula, the names, and LCMS data of the obtained compounds are shown in Table 11.

**[Table 11]**

| Example No. | Structural formula | Compound name | MS (ESI pos.) m/z | Retention time (min) |
|---|---|---|---|---|
| Example 78 | 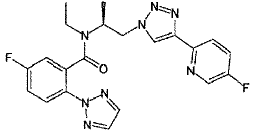 | N-Ethyl-5-fluoro-N-{(2S)-1--[4-(5-fluoropyridin-2-yl)-1H-1,2,3-triazol-1-yl]propan-2-yl}-2-(2H-1,2,3-triazol-2-yl)benzamide | 461(M+Na)+ | 0.358 |
| Example 79 | 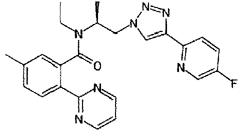 | N-Ethyl-N-{(2S)-1-[4-(5-fluoropyridin-2-yl)-1H-1,2,3-triazol-1-yl]propan-2-yl}-5-methyl-2-(pyrimidin-2-yl)benzamide | 446(M+H)+ | 0.344 |
| Example 80 | 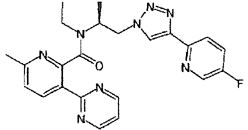 | N-Ethyl-N-{(2S)-1-[4-(5-fluoropyridin-2-yl)-1H-1,2,3-triazol-1-yl]propan-2-yl}-6-methyl-3-(pyrimidin-2-yl)pyridine-2-carboxamide | 447(M+H)+ | 0.338 |
| Example 81 | 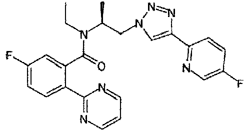 | N-Ethyl-5-fluoro-N-{(2S)-1-[4-(5-fluoropyridin-2-yl)-1H-1,2,3-triazol-1-yl]propan-2-yl}-2-(pyrimidin-2-yl) benzamide | 472(M+Na)+ | 0.338 |
| Example 82 | 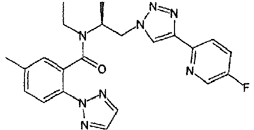 | N-Ethyl-N-{(2S)-1-[4-(5-fluoropyridin-2-yl)-1H-1,2,3-triazol-1-yl]propan-2-yl}-5-methy-2-(2H-1,2,3-triazol-2-yl)benzamide | 435(M+H)+ | 0.535 |
| Example 83 | 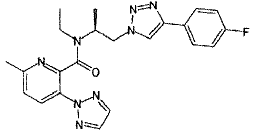 | N-Ethyl-N-{(2S)-1-[4-(4-fluorophenyl)-1H-1,2,3-triazol-1-yl] propan-2-yl}-6-methyl-3-(2H-1,2,3-triazol-2-yl)pyridine-2-carboxamide | 435(M+H)+ | 0.518, 0.573 |
| Example 84 | 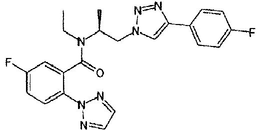 | N-Ethyl-5-fluoro-N-{(2S)-1-[4-(4-fluorophenyl)-1H-1,2,3-triazol-1-yl] propan-2-yl}-2-(2H-1,2,3-triazol-2-yl)benzamide | 438(M+H)+ | 0.566 |

### Example 85: N-Ethyl-N-{(2S)-1-[5-(5-fluoropyridin-2-yl)-2H-tetrazol-2-yl]propan-2-yl}-5-methyl-2-(2H-1,2,3-triazol-2-yl)benzamide

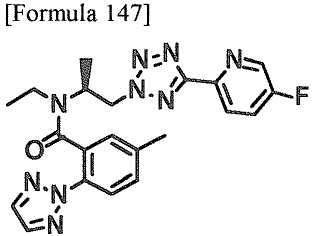

By using (2S)-N-ethyl-1-[4-(5-fluoropyridin-2-yl)-1H-1,2,3-triazol-1-yl]propan-2-amine obtained in Reference Example 93 (0.050 g, 0.20 mmol) as the raw material, the same procedure as in Example 45 was carried out to obtain the title compound (0.033 g) (colorless solid).
LCMS retention time 0.556 min.
MS (ESI pos.) m/z: 436 [M+H]+

Examples 86 to 88 were obtained by the same procedure as in Example 85. The structural formula, the names, and LCMS data of the obtained compounds are shown in Table 12.

**[Table 12]**

| Example No. | Structural formula | Compound name | MS (ESI pos.) m/z | Retention time (min) |
|---|---|---|---|---|
| Example 86 | 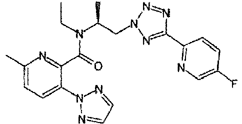 | N-Ethyl-N-{(2S)-1-[5-(5-fluoropyridin-2-yl)-2H-tetrazol-2-yl]propan-2-yl}-6-methyl-3-(2H-1,2,3-triazol-2-yl)pyridine-2-carboxamide | 437(M+H)+ | 0.471, 0.520 |
| Example 87 | 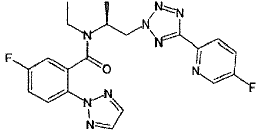 | N-Ethyl-5-fluoro-N-{(2S)-1-[5-(5-fluoropyridin-2-yl)-2H-tetrazol-2-yl]propan-2-yl}-2-(2H-1,2,3-triazol-2-yl)benzamide | 440(M+H)+ | 0.547 |
| Example 88 | 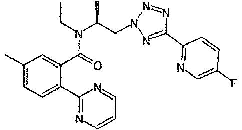 | N-Ethyl-N-{(2S)-1-[5-(5-fluoropyridin-2-yl)-2H-tetrazol-2-yl]propan-2-yl}-5-methyl-2-(pyrimidin-2-yl)benzamide | 447(M+H)+ | 0.536 |

Examples 89 to 91 were obtained by the same procedure as in Example 71. The structural formula, the names, and LCMS data of the obtained compounds are shown in Table 13.

**[Table 13]**

| Example No. | Structural formula | Compound name | MS (ESI pos.) m/z | Retention time (min) |
|---|---|---|---|---|
| Example 89 | 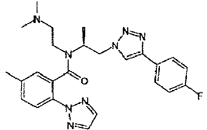 | N-[2-(Dimethylamino)ethyl]-N-{(2S)-1-[4-(4-fluorophenyl)-1H-1,2,3-triazol-1-yl] propan-2-yl}-5-methyl-2-(2H-1,2,3-triazol -2-yl)benzamide | 477(M+H)+ | 0.56 |
| Example 90 | 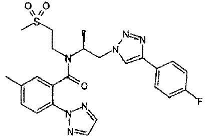 | N-{(2S)-1-[4-(4-Fluorophenyl)-1H-1,2,3-triazol-1-yl]propan-2-yl}-5-methyl-N-[2-(methylsuflonyl)ethyl]-2-(2H-1,2,3-triazol-2-yl)benzamide | 512(M+H)+ | 0.878 |
| Example 91 | 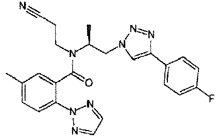 | N-(2-Cyanoethyl)-N-{(2S)-1-[4-(4-fluorophenyl)-1H-1,2,3-triazol-1-yl]propan -2-yl)-5-methyl-2-(2H-1,2,3-triazol-2-yl) benzamide | 459(M+H)+ | 0.929 |

### Example 92: N-{(2S)-1-[4-(4-Chlorophenyl)-1H-1,2,3-triazol-1-yl]propan-2-yl}-N-ethyl-5-methyl-2-(2H-1,2,3-triazol-2-yl)benzamide

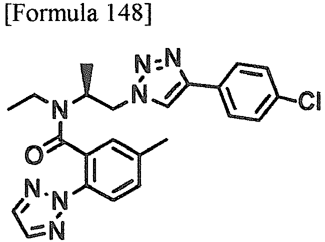

To a solution of N-[(2S)-1-azidepropan-2-yl]-N-ethyl-5-methyl-2-(2H-1,2,3-triazol-2-yl)benzamide obtained in Reference Example 106 (0.064 g, 0.21 mmol) and 1-chloro-4-ethynylbenzene (0.028 g, 0.21 mmol) in DMF/4-methylpiperidine (1.6 mL/0.4 mL), CuI (0.002 g, 0.010 mmol) and L-ascorbic acid (0.007 g, 0.041 mmol) were added, and the resulting mixture was stirred at 80°C for 2 hours. The reaction mixture was allowed to cool to room temperature, then water was added at 0°C, followed by extraction with EtOAc. The obtained organic layer was washed with brine and then dried over Na₂SO₄. The drying agent was filtered off, the reaction mixture was concentrated under reduced pressure, and then the obtained residue was purified by column chromatography (SNAP Ultra 10g, hexane/EtOAc = 88/12 to 0/100) to obtain the title compound (0.065 g) (colorless solid).
LCMS retention time 1.064 min.
MS (ESI pos.) m/z: 450 [M+H]+

Examples 93 to 99 were obtained by the same procedure as in Example 92. The structural formula, the names, and LCMS data of the obtained compounds are shown in Table 14.

**[Table 14]**

| Example No. | Structural formula | Compound name | MS (ESI pos.) m/z | Retention time (min) |
|---|---|---|---|---|
| Example 93 | 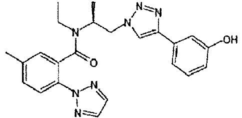 | N-Ethyl-N-{(2S)-1-[4-(3-hydroxyphenyl)-1H-1,2,3-triazol-1-yl] propan-2-yl}-5-methyl-2-(2H-1,2,3-triazol-2-yl)benzamide | 432(M+H)+ | 0.838 |
| Example 94 | 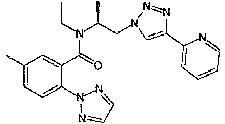 | N-Ethyl-5-methyl-N-{(2S)-1-[4-(pyridin-2-yl)-1H-1,2,3-triazol-1-yl] propan-2-yl}-2-(2H-1,2,3-triazol-2-yl) benzamide | 417(M+H)+ | 0.787 |
| Example 95 | 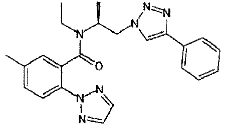 | N-Ethyl-5-methyl-N-{(2S)-1-(4-phenyl-1H-1,2,3-triazol-1-yl)propan-2-yl]-2-(2H-1,2,3-triazol-2-yl)benzamide | 416(M+H)+ | 0.96 |
| Example 96 | 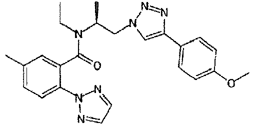 | N-Ethyl-N-{(2S)-1-[4-(4-methoxyphenyl)-1H-1,2,3-triazol-1-yl] propan-2-yl}-5-methyl-2-(2H-1,2,3-triazol-2-yl)benzamide | 446(M+H)+ | 0.95 |
| Example 97 | 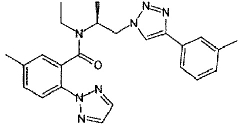 | N-Ethyl-5-methyl-N-{(2S)-1-[4-(3-methylphenyl)-1H-1,2,3-triazol-1-yl] propan-2-yl}-2-(2H-1,2,3-triazol-2-yl) benzamide | 430(M+H)+ | 1.04 |
| Example 98 | 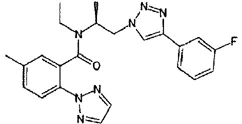 | N-Ethyl-N-{(2S)-1-[4-(3-fluorophenyl) -1H-1,2,3-triazol-1-yl]propan-2-yl}-5-methyl-2-(2H-1,2,3-triazol-2-yl) benzamide | 434(M+H)+ | 1.006 |
| Example 99 | 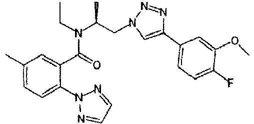 | N-Ethyl-N-{(2S)-1-[4-(4-fluoro-3-methoxyphenyl)-1H-1,2,3-triazol-1-yl] propan-2-yl}-5-methyl-2-(2H-1,2,3-triazol-2-yl)benzamide | 464(M+H)+ | 0.987 |

### Example 100: N-Ethyl-N-{(2S)-1-[4-(4-hydroxyphenyl)-1H-1,2,3-triazol-1-yl]propan-2-yl}-5-methyl-2-(2H-1,2,3-triazol-2-yl)benzamide

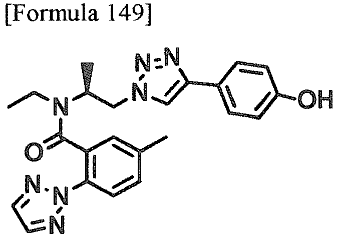

By using N-[(2S)-1-azidepropan-2-yl]-N-ethyl-5-methyl-2-(2H-1,2,3-triazol-2-yl)benzamide obtained in Reference Example 106 (0.044 g, 0.14 mmol) and benzyl 4-ethynylphenyl ether (0.029 g, 0.14 mmol) as the raw materials, the same procedure as in Example 92 was carried out to obtain colorless oil (0.006 g). The obtained oil was dissolved in MeOH (1 mL), then 10% Pd/C (0.01 g) was added thereto, and the resulting mixture was stirred at room temperature for 15 hours under hydrogen atmosphere. The reaction mixture was filtered through Celite®, then the obtained filtrate was concentrated under reduced pressure to obtain the title compound (0.0045 g) (pale yellow oil).
LCMS retention time 0.784 min.
MS (ESI pos.) m/z: 432 [M+H]+

### Example 101: N-Ethyl-N-{(2S)-1-[4-(3-methoxyphenyl)-1H-1,2,3-triazol-1-yl]propan-2-yl}-5-methyl-2-(2H-1,2,3-triazol-2-yl)benzamide

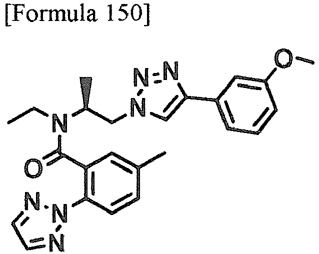

By using N-ethyl-N-{(2S)-1-[4-(3-hydroxyphenyl)-1H-1,2,3-triazol-1-yl]propan-2-yl}-5-methyl-2-(2H-1,2,3-triazol-2-yl)benzamide obtained in Example 93 (0.020 g, 0.046 mmol) and MeI (0.004 ml, 0.070 mmol) as the raw materials, the same procedure as in Example 34 was carried out to obtain the title compound (0.004 g) (colorless solid). LCMS retention time 0.964 min.
MS (ESI pos.) m/z: 446 [M+H]+

### Example 102: N-{(2S)-1-[4-(4-Fluorophenyl)-1H-pyrazol-1-yl]propan-2-yl}-N-(2-hydroxypropyl)-5-methyl-2-(2H-1,2,3-triazol-2-yl)benzamide

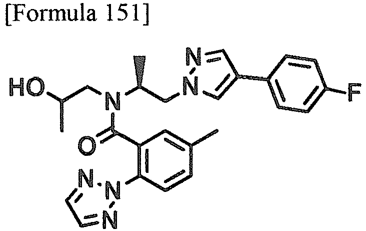

By using N-{(2S)-1-[4-(4-fluorophenyl)-1H-pyrazol-1-yl]propan-2-yl}-5-methyl-2-(2H-1,2,3-triazol-2-yl)benzamide obtained in Reference Example 86 (0.10 g, 0.25 mmol) and 2-[(1-bromopropan-2-yl)oxy]tetrahydro-2H-pyran (1.3 g, 5.9 mmol) as the raw materials, the same procedure as in Example 41 was carried out to obtain the title compound (0.019 g) (colorless and amorphous).
LCMS retention time 0.981 min.
MS (ESI pos.) m/z: 463 [M+H]+

### Test Example (measurement of orexin antagonistic activity)

The antagonistic activities of the test compounds on human orexin-1 receptor (hOXIR) and orexin-2 receptor (hOX2R) were measured by modifying from the method described in literature (Toshikatsu Okumura et al., Biochemical and Biophysical Research Communications, 280, 976-981, 2001). Chinese hamster ovary (CHO) cells forcibly expressing the hOXIR and hOX2R were seeded into a 96 well Black clear bottom plate (Nunc) at 20,000 cells per well,, which were cultured in Ham's F-12 medium containing 0.1 mM MEM non-essential amino acids, 0.5 mg/mL G418, 10% fetal bovine serum (all by Invitrogen) for 16 hours under the conditions of 37°C, 5% CO₂. After removing the medium, 100 µL of 0.5 µM Fluo-4AM ester (Dojin) in an assay buffer (25 mM HEPES (Dojin), Hank's balanced salt solution (Invitrogen), 0.1% bovine serum albumin, 2.5 mM probenecid, 200 µg/mL Amaranth (all by Sigma-Aldrich), pH 7.4) was added and the cells were incubated for 60 minutes at 37°C, 5% CO₂. After removing the assay buffer containing fluo-4AM ester, the test compound was dissolved in dimethyl sulfoxide to be 10 mM and diluted with the assay buffer, 150 of which was added and incubated for 30 minutes.

The ligand peptide, in which 2 amino acids of human orexin-Aare substituted (Pyr-Pro-Leu-Pro-Asp-Ala-Cys-Arg-Gln-Lys-Thr-Ala-Ser-Cys-Arg-Leu-Tyr-Glu-Leu-Leu-His-Gly-Ala-Gly-Asn-His-Ala-Ala-Gly-Ile-Leu-Thr-Leu-NH2; Peptide Institute, Inc.), were diluted with an assay buffer to give the final concentration of 300 pM for hOXIR and 3 nM for hOX2R, and 50 µL of the ligand solution was added to start the reaction. The reaction was measured for the fluorescence intensity of each well every second for 3 minutes using Functional Drug Screening System (FDSS; Hamamatsu Photonics K.K.), and the antagonistic activity was determined using the maximum fluorescence intensity as the indicator of intracellular Ca2+ concentration. The antagonistic activity of test compound was calculated when the fluorescence intensity of wells to which only the dilution buffer was added is 100% and the fluorescence intensity of wells to which the buffer containing no ligand or compound was added is 0%, and the 50% inhibition concentration (IC₅₀ value) was determined from the fluorescence intensities when the several concentrations of compounds were added.
The IC₅₀ values of the compounds of the present invention are shown in Table 15.

**[Table 15]**

| Example No. | IC₅₀ Value | | Example No. | IC₅₀ Value | | Example No. | IC₅₀ Value | |
|---|---|---|---|---|---|---|---|---|
| | OX1 (nM) | OX2 (nM) | | OX1 (nM) | OX2 (nM) | | OX1 (nM) | OX2 (nM) |
| 1 | 1.0 | 1.1 | 35 | 3.4 | 62.8 | 69 | 1.6 | 1896.4 |
| 2 | 17.4 | 185.9 | 36 | 6.8 | 214.2 | 70 | 1.1 | 452.2 |
| 3 | 1.0 | 4.5 | 37 | 7.1 | 83.1 | 71 | 1.3 | 21.8 |
| 4 | 1.7 | 67.0 | 38 | 25.5 | 350.6 | 72 | 101.5 | 401.2 |
| 5 | 0.5 | 75.5 | 39 | 1.2 | 5.5 | 73 | 80.3 | 5.7 |
| 6 | 1.2 | 8.4 | 40 | 1.4 | 12.4 | 74 | 1.3 | 13.1 |
| 7 | 6.8 | 43.1 | 41 | 30.9 | 86.3 | 75 | 1.4 | 1.7 |
| 8 | 1.5 | 35.3 | 42 | 1.1 | 21.8 | 76 | 1.0 | 59.7 |
| 9 | 988.3 | 1423.7 | 43 | 3.5 | 13.6 | 77 | 48.8 | 1448.5 |
| 10 | 0.2 | 12.8 | 44 | 1.3 | 25.9 | 78 | 20.9 | 592.8 |
| 11 | 0.2 | 14.4 | 45 | 3.3 | 75.5 | 79 | 63.6 | 426.9 |
| 12 | 364.2 | 579.9 | 46 | 21.6 | 390.3 | 80 | 686.9 | 7807.1 |
| 13 | 1.9 | 53.9 | 47 | 0.8 | 9.5 | 81 | 176.7 | 3151.0 |
| 14 | 1.3 | 7.0 | 48 | 0.9 | 28.2 | 82 | 2.2 | 13.3 |
| 15 | 1.2 | 18.0 | 49 | 1.1 | 32.2 | 83 | 9.9 | 308.2 |
| 16 | 1.8 | 9.7 | 50 | 0.8 | 16.8 | 84 | 3.1 | 51.4 |
| 17 | 2.1 | 10.1 | 51 | 0.2 | 9.3 | 85 | 1.3 | 27.5 |
| 18 | 0.7 | 7.7 | 52 | 9.2 | 375.7 | 86 | 37.7 | 1025.4 |
| 19 | 1.1 | 5.8 | 53 | 6.6 | 75.6 | 87 | 9.8 | 282.7 |
| 20 | 3.4 | 55.9 | 54 | 2.0 | 75.7 | 88 | 13.4 | 41.2 |
| 21 | 8.1 | 188.1 | 55 | 1.4 | 9.4 | 89 | 228.5 | 6934.0 |
| 22 | 3.8 | 52.9 | 56 | 5.0 | 602.4 | 90 | 23.7 | 2069.8 |
| 23 | 133.9 | 1892.0 | 57 | 5.1 | 1082.4 | 91 | 202.9 | 279.5 |
| 24 | 9.8 | 41.4 | 58 | 22.2 | 1621.5 | 92 | 2.5 | 64.6 |
| 25 | 0.7 | 6.4 | 59 | 0.5 | 10.1 | 93 | 1.4 | 7.4 |
| 26 | 1.7 | 55.9 | 60 | 1.3 | 45.0 | 94 | 9.3 | 108.5 |
| 27 | 1.4 | 13.8 | 61 | 0.7 | 3.3 | 95 | 0.6 | 17.7 |
| 28 | 18.6 | 89.3 | 62 | 0.7 | 2.3 | 96 | 159.7 | 8365.5 |
| 29 | 10.1 | 25.6 | 63 | 2.2 | 66.9 | 97 | 2.4 | 21.3 |
| 30 | 1.2 | 35.7 | 64 | 1.0 | 47.9 | 98 | 6.3 | 9.8 |
| 31 | 1.0 | 10.2 | 65 | 1.3 | 144.3 | 99 | 1.8 | 1.5 |
| 32 | 20.7 | 96.9 | 66 | 1.6 | 129.2 | 100 | 10.4 | 774.3 |
| 33 | 11.9 | 20.8 | 67 | 1.8 | 75.9 | 101 | 0.9 | 1.5 |
| 34 | 9.8 | 334.1 | 68 | 0.8 | 33.7 | 102 | 1.4 | 27.9 |

### Industrial Applicability

The compounds of the present invention are verified to have the OX receptor antagonistic activities. Thus, the compounds of the present invention or the pharmaceutically acceptable salts thereof can be used as a therapeutic or preventive drug for diseases regulated by OX receptor antagonistic activities such as sleep disorder, depression, anxiety disorder, panic disorder, schizophrenia, drug dependence, Alzheimer's disease, Parkinson's disease, Huntington's disease, eating disorder, headache, migraine, pain, gastrointestinal disease, epilepsy, inflammation, immunological diseases, endocrine related disease and hypertension.

## Claims

1. A compound represented by formula (Ia): 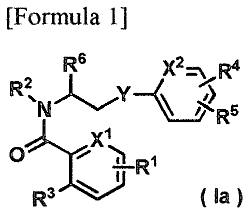 wherein,
X¹ and X² are the same or different and represent a nitrogen atom or a formula CH;
Y represents any of the structures in a following formula group (a): 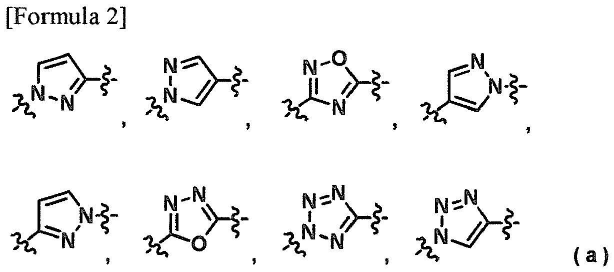
R¹ represents a hydrogen atom, a halogen atom or a C₁₋₆ alkyl group;
R² represents a C₁₋₆ alkyl group (the C₁₋₆ alkyl group may optionally be substituted with one substituent selected from the group consisting of a hydroxy group, a C₁₋₆ alkoxy group, a C₁₋₆ alkylsulfonyl group, a diC₁₋₆ alkylamino group and a cyano group);
R³ represents a triazolyl group or a pyrimidinyl group;
R⁴ and R⁵ are the same or different and represent a hydrogen atom, a halogen atom, a C₁₋₆ alkyl group, a hydroxyl group or a C₁₋₆ alkoxy group; and
R⁶ represents a C₁₋₆ alkyl group;
or a pharmaceutically acceptable salt thereof.

2. A compound represented by formula (Ia): 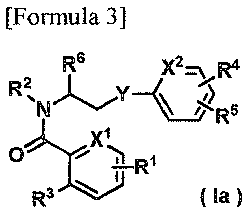 wherein,
X¹ and X² are the same or different and represent a nitrogen atom or a formula CH;
Y represents any of the structures in a following formula group (a): 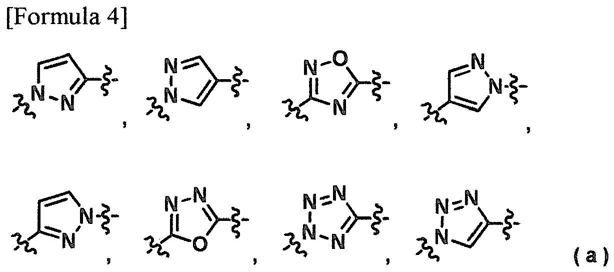
R¹ represents a hydrogen atom, a halogen atom or a C₁₋₆ alkyl group;
R² represents a C₁₋₆ alkyl group (the C₁₋₆ alkyl group may optionally be substituted with one substituent selected from the group consisting of a hydroxy group and a C₁₋₆ alkoxy group);
R³ represents a triazolyl group or a pyrimidinyl group;
R⁴ represents a halogen atom;
R⁵ represents a hydrogen atom or a halogen atom; and
R⁶ represents a C₁₋₆ alkyl group;
or a pharmaceutically acceptable salt thereof.

3. The compound or a pharmaceutically acceptable salt thereof according to claim 1 or 2, wherein Y in the above formula (Ia) is any of the structures in a following formula group (a1):

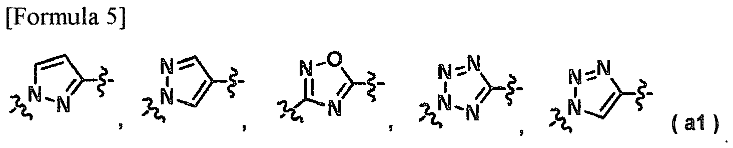

4. The compound or a pharmaceutically acceptable salt thereof according to any one of claims 1 to 3, wherein R¹ in the above formula (Ia) is a hydrogen atom, a fluorine atom or a methyl group.

5. The compound or a pharmaceutically acceptable salt thereof according to any one of claims 1 to 4, wherein R² in the above formula (Ia) is a methyl group or an ethyl group (the ethyl group may optionally be substituted with one substituent selected from the group consisting of a hydroxyl group and a methoxy group).

6. The compound or a pharmaceutically acceptable salt thereof according to any one of claims 1 to 5, wherein R⁴ in the above formula (Ia) is a fluorine atom.

7. The compound or a pharmaceutically acceptable salt thereof according to any one of claims 1 to 6, wherein R⁵ in the above formula (Ia) is a hydrogen atom or a fluorine atom.

8. The compound or a pharmaceutically acceptable salt thereof according to any one of claims 1 to 7, wherein R⁶ in the above formula (Ia) is a C₁₋₃ alkyl group.

9. The compound or a pharmaceutically acceptable salt thereof according to any one of claims 1 to 8, wherein Y in the above formula (Ia) is any of the structures in a following formula group (a2):

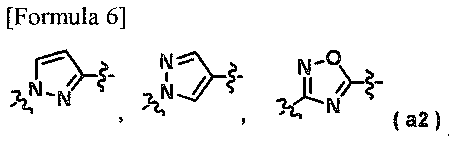

10. The compound or a pharmaceutically acceptable salt thereof according to any one of claims 1 to 8, wherein Y in the above formula (Ia) is a following formula (a3):

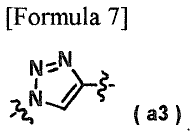

11. The compound or a pharmaceutically acceptable salt thereof according to any one of claims 1 to 10, wherein R² in the above formula (Ia) is an ethyl group.

12. The compound or a pharmaceutically acceptable salt thereof according to any one of claims 1 to 11, wherein R⁶ in the above formula (Ia) is a methyl group.

13. The compound or a pharmaceutically acceptable salt thereof according to any one of claims 1 to 12, wherein the above formula (Ia) is represented by formula (IIa):

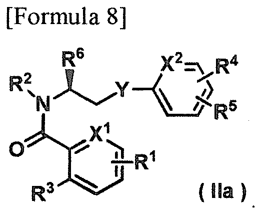

14. The compound or a pharmaceutically acceptable salt thereof according to any one of claims 1 to 13, wherein the above formula (Ia) is represented by formula (IIIa):

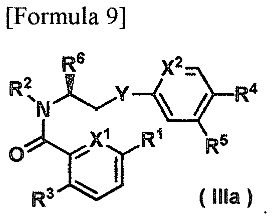

15. A compound represented by formula (I): 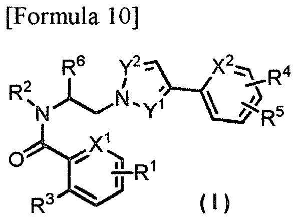 wherein,
X¹ and X² are the same or different and represent a nitrogen atom or a formula CH;
either one of Y¹ and Y² represents a nitrogen atom, and the other represents CH;
R¹ represents a hydrogen atom, a halogen atom or a C₁₋₆ alkyl group;
R² represents a C₁₋₆ alkyl group;
R³ represents a triazolyl group or a pyrimidinyl group;
R⁴ represents a halogen atom;
R⁵ represents a hydrogen atom or a halogen atom; and
R⁶ represents a C₁₋₆ alkyl group;
or a pharmaceutically acceptable salt thereof.

16. The compound or a pharmaceutically acceptable salt thereof according to claim 15, wherein R¹ in the above formula (I) is a hydrogen atom, a fluorine atom or a methyl group.

17. The compound or a pharmaceutically acceptable salt thereof according to any of claim 15 or 16, wherein R² in the above formula (I) is a methyl group or an ethyl group.

18. The compound or a pharmaceutically acceptable salt thereof according to any one of claims 15 to 17, wherein R⁴ in the above formula (I) is a fluorine atom.

19. The compound or a pharmaceutically acceptable salt thereof according to any one of claims 15 to 18, wherein R⁵ in the above formula (I) is a hydrogen atom or a fluorine atom.

20. The compound or a pharmaceutically acceptable salt thereof according to any one of claims 15 to 19, wherein R⁶ in the above formula (I) is a C₁₋₃ alkyl group.

21. The compound or a pharmaceutically acceptable salt thereof according to any one of claims 15 to 20, wherein R⁶ in the above formula (I) is a methyl group.

22. The compound or a pharmaceutically acceptable salt thereof according to any one of claims 15 to 21, wherein the above formula (I) is represented by formula (II):

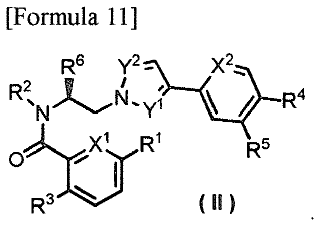

23. The compound or a pharmaceutically acceptable salt thereof according to claim 1, which is a species or a mixture of two or more species selected from:
N-ethyl-N-{(2S)-1-[3-(5-fluoropyridin-2-yl)-1H-pyrazol-1-yl]propan-2-yl}-5-methyl-2-(2H-1,2,3-triazol-2-yl)benzamide,
N-ethyl-N-{(2R)-1-[3-(5-fluoropyridin-2-yl)-1H-pyrazol-1-yl]propan-2-yl}-5-methyl-2-(2H-1,2,3-triazol-2-yl)benzamide,
N-ethyl-N-{(2S)-1-[3-(4-fluorophenyl)-1H-pyrazol-1-yl]propan-2-yl}-6-methyl-3-(2H-1,2,3-triazol-2-yl)pyridine-2-carboxamide,
N-{(2S)-1-[3-(5-fluoropyridin-2-yl)-1H-pyrazol-1-yl]propan-2-yl}-N-methyl-2-(pyrimidin-2-yl)benzamide,
5-fluoro-N-{(2S)-1-[3-(5-fluoropyridin-2-yl)-1H-pyrazol-1-yl]propan-2-yl}-N-methyl-2-(pyrimidin-2-yl)benzamide,
N-{(2S)-1-[3-(5-fluoropyridin-2-yl)-1H-pyrazol-1-yl]propan-2-yl}-N, 5-dimethyl-2-(pyrimidin-2-yl)benzamide,
N-ethyl-N-{(2S)-1-[3-(5-fluoropyridin-2-yl)-1H-pyrazol-1-yl]propan-2-yl}-2-(pyrimidin-2-yl)benzamide,
N-ethyl-5-fluoro-N-{(2S)-1-[3-(5-fluoropyridin-2-yl)-1H-pyrazol-1-yl]propan-2-yl}-2-(pyrimidin-2-yl)benzamide,
N-ethyl-N-{(2S)-1-[3-(4-fluorophenyl)-1H-pyrazol-1-yl]propan-2-yl}-2-(pyrimidin-2-yl)benzamide,
N-ethyl-5-fluoro-N-{(2S)-1-[3-(4-fluorophenyl)-1H-pyrazol-1-yl]propan-2-yl}-2-(pyrimidin-2-yl)benzamide,
N-ethyl-2-ftuoro-N-{(2S)-1-[3-(4-fluorophenyl)-1H-pyrazol-1-yl]propan-2-yl}-6-(pyrimidin-2-yl)benzamide,
N-ethyl-4-fluoro-N-{(2S)-1-[3-(4-fluorophenyl)-1H-pyrazol-1-yl]propan-2-yl}-2-(pyrimidin-2-yl)benzamide,
N-ethyl-N-{(2S)-1-[3-(4-fluorophenyl)-1H-pyrazol-1-yl]propan-2-yl}-6-methyl-3-(pyrimidin-2-yl)pyridine-2-carboxamide,
N-{(2S)-1-[3-(3,4-difluorophenyl)-1H-pyrazol-1-yl]propan-2-yl}-N-ethyl-6-methyl-3-(2H-1,2,3-triazol-2-yl)pyridine-2-carboxamide,
N- (2S)-1-[3-(3,4-difluorophenyl)-1H-pyrazol-1-yl]propan-2-yl}-N-ethyl-5-fluoro-2-(pyrimidin-2-yl)benzamide,
N-ethyl-N-{(2S)-1-[3-(5-fluoropyridin-2-yl)-1H-pyrazol-1-yl]propan-2-yl}-2-(2H-1,2,3-triazol-2-yl)benzamide,
N-{(2S)-1-[3-(5-fluoropyridin-2-yl)-1H-pyrazol-1-yl]propan-2-yl}-N, 5-dimethyl-2-(2H-1,2,3-triazol-2-yl)benzamide,
N-{(2S)-1-[3-(5-fluoropyridin-2-yl)-1H-pyrazol-1-yl]propan-2-yl}-N-methyl-2-(2H-1,2,3-triazol-2-yl)benzamide,
N-ethyl-N-{(2S)-1-[4-(5-fluoropyridin-2-yl)-1H-pyrazol-1-yl]propan-2-yl}-2-(2H-1,2,3-triazol-2-yl)benzamide,
N-{(2S)-1-[4-(5-fluoropyridin-2-yl)-1H-pyrazol-1-yl]propan-2-yl}-N,5-dimethyl-2-(2H-1,2,3-triazol-2-yl)benzamide,
N-{(2S)-1-[3-(5-fluoropyridin-2-yl)-1H-pyrazol-1-yl]butan-2-yl}-N,5-dimethyl-2-(2H-1,2,3-triazol-2-yl)benzamide,
N-ethyl-N-{(2S)-1-[4-(5-fluoropyridin-2-yl)-1H-pyrazol-1-yl]propan-2-yl}-5-methyl-2-(2H-1,2,3-triazol-2-yl)benzamide,
N-ethyl-N-{(2S)-1-[4-(4-fluorophenyl)-1H-pyrazol-1-yl]propan-2-yl}-6-methyl-3-(2H-1,2,3-triazol-2-yl)pyridine-2-carboxamide,
N-{(2S)-1-[4-(3,4-difluorophenyl)-1H-pyrazol-1-yl]propan-2-yl}-N-ethyl-6-methyl-3-(2H-1,2,3-triazol-2-yl)pyridine-2-carboxamide,
N-{(2S)-1-[4-(4-fluorophenyl)-1H-pyrazol-1-yl]propan-2-yl}-N,6-dimethyl-3-(2H-1,2,3-triazol-2-yl)pyridine-2-carboxamide,
N-{(2S)-1-[4-(3,4-difluorophenyl)-1H-pyrazol-1-yl]propan-2-yl}-N,6-dimethyl-3-(2H-1,2,3-triazol-2-yl)pyridine-2-carboxamide,
N-ethyl-5-fluoro-N-{(2S)-l-[4-(4-fluorophenyl)-1H-pyrazol-l-yl]propan-2-yl}-2-(pyrimidin-2-yl)benzamide,
N-{(2S)-1-[4-(3,4-difluorophenyl)-1H-pyrazol-1-yl]propan-2-yl}-N-ethyl-5-fluoro-2-(pyrimidin-2-yl)benzamide,
5-fluoro-N-{(2S)-1-[4-(4-fluorophenyl)-1H-pyrazol-1-yl]propan-2-yl}-N-methyl-2-(pyrimidin-2-yl)benzamide,
N-{(2S)-1-[4-(3,4-difluorophenyl)-1H-pyrazol-1-yl]propan-2-yl}-5-fluoro-N-methyl-2-(pyrimidin-2-yl)benzamide,
N-ethyl-5-fluoro-N-{(2S)-1-[4-(5-fluoropyridin-2-yl)-1H-pyrazol-1-yl)propan-2-yl}-2-(pyrimidin-2-yl)benzamide,
N-ethyl-N-{(2S)-1-[4-(5-fluoropyridin-2-yl)-1H-pyrazol-1-yl]propan-2-yl}-5-methyl-2-(pyrimidin-2-yl)benzamide,
N-ethyl-5-fluoro-N-{(2S)-1-[4-(5-fluoropyridin-2-yl)-1H-pyrazol-1-yl)propan-2-yl-2-(2H-1,2,3-triazol-2-yl)benzamide,
5-chloro-N-ethyl-N-{(2S)-1-[4-(5-fluoropyridin-2-yl)-1H-pyrazol-1-yl]propan-2-yl}-2-(pyrimidin-2-yl)benzamide,
N-{(2S)-1-[4-(5-fluoropyridin-2-yl)-1H-pyrazol-1-yl]propan-2-yl}-N-(2-methoxyethyl)-5-methyl-2-(2H-1,2,3-triazol-2-yl)benzamide,
N- {(2S)-1-[3-(4-fluorophenyl)-1H-pyrazol-1-yl]propan-2-yl}-N-(2-methoxyethyl)-5-methyl-2-(2H-1,2,3-triazol-2-yl)benzamide,
N-{(2S)-1-[3-(5-fluoropyridin-2-yl)-1H-pyrazol-1-yl]propan-2-yl}-N-(2-methoxyethyl)-5-methyl-2-(2H-1,2,3-triazol-2-yl)benzamide,
N-{(2S)-1-[4-(5-fluoropyridin-2-yl)-1H-pyrazol-1-yl]propan-2-yl}-N-(2-hydroxyethyl)-5-methyl-2-(2H-1,2,3-triazol-2-yl)benzamide,
N-{(2S)-1-[3-(4-fluorophenyl)-1H-pyrazol-1-yl]propan-2-yl}-N-(2-hydroxyethyl)-5-methyl-2-(2H-1,2,3-triazol-2-yl)benzamide,
N-{(2S)-1-[4-(4-fluorophenyl)-1H-pyrazol-1-yl]propan-2-yl}-N-(2-hydroxyethyl)-5-methyl-2-(2H-1,2,3-triazol-2-yl)benzamide,
N-{(2S)-1-[3-(5-fluoropyridin-2-yl)-1H-pyrazol-1-yl]propan-2-yl}-N-(2-hydroxyethyl)-5-methyl-2-(2H-1,2,3-triazol-2-yl)benzamide,
N-{(2S)-1-[5-(5-fluoropyridin-2-yl)-1,2,4-oxadiazol-3-yl]propan-2-yl}-N,5-dimethyl-2-(2H-1,2,3-triazol-2-yl)benzamide,
N-{(2S)-1-[5-(5-fluoropyridin-2-yl)-1,2,4-oxadiazol-3-yl]propan-2-yl}-N,5-dimethyl-2-(pyrimidin-2-yl)benzamide,
N-ethyl-N-{(2S)-1-[5-(5-fluoropyridin-2-yl)-1,2,4-oxadiazol-3-yl]propan-2-yl}-5-methyl-2-(2H-1,2,3-triazol-2-yl)benzamide,
N-ethyl-N-{(2S)-1-[5-(5-fluoropyridin-2-yl)-1,2,4-oxadiazol-3-yl]propan-2-yl}-5-methyl-2-(pyrimidin-2-yl)benzamide,
N-ethyl-N-{(2S)-1-[5-(4-fluorophenyl)-1,2,4-oxadiazol-3-yl]propan-2-yl}-6-methyl-3-(2H-1,2,3-triazol-2-yl)pyridine-2-carboxamide,
N-ethyl-5-fluoro-N-{(2S)-1-[5-(4-fluorophenyl)-1,2,4-oxadiazol-3-yl]propan-2-yl}-2-(2H-1,2,3-triazol-2-yl)benzamide,
N-ethyl-N-{(2S)-1-[5-(4-fluorophenyl)-1,2,4-oxadiazol-3-yl]propan-2-yl}-2-(2H-1,2,3-triazol-2-yl)benzamide,
N-ethyl-N-{(2S)-1-[5-(4-fluorophenyl)-1,2,4-oxadiazol-3-yl]propan-2-yl}-6-methyl-3-(pyrimidin-2-yl)pyridine-2-carboxamide,
N-ethyl-5-fluoro-N-{(2S)-1-[5-(4-fluorophenyl)-1,2,4-oxadiazol-3-yl]propan-2-yl]-2-(pyrimidin-2-yl)benzamide,
N-ethyl-N-{(2S)-1-[5-(4-fluorophenyl)-1,2,4-oxadiazol-3-yl]propan-2-yl}-2-(pyrimidin-2-yl)benzamide,
N-ethyl-N-{(2S)-1-[5-(4-fluorophenyl)-1,2,4-oxadiazol-3-yl]propan-2-yl}-5-methyl-2-(pyrimidin-2-yl)benzamide,
N-ethyl-N-{(2S)-1-[5-(5-fluoropyridin-2-yl)-1,2,4-oxadiazol-3-yl]propan-2-yl}-2-(2H-1,2,3-triazol-2-yl)benzamide,
N-ethyl-5-fluoro-N-{(2S)-1-[5-(5-fluoropyridin-2-yl)-1,2,4-oxadiazol-3-yl]propan-2-yl}-2-(2H-1,2,3-triazol-2-yl)benzamide,
N-ethyl-N-{(2S)-1-[5-(5-fluoropyridin-2-yl)-1,2,4-oxadiazol-3-yl]propan-2-yl}-6-methyl-3-(2H-1,2,3-triazol-2-yl)pyridine-2-carboxamide,
N-{(2S)-1-[5-(4-fluorophenyl)-1,2,4-oxadiazol-3-yl]propan-2-yl}-N,5-dimethyl-2-(2H-1,2,3-triazol-2-yl)benzamide,
N-{(2S)-1-[5-(4-fluorophenyl)-1,2,4-oxadiazol-3-yl]propan-2-yl}-N,5-dimethyl-2-(pyrimidin-2-yl)benzamide,
N-ethyl-N-{(2S)-1-[5-(4-fluorophenyl)-1,2,4-oxadiazol-3-yl]propan-2-yl}-5-methyl-2-(2H-1,2,3-triazol-2-yl)benzamide,
N-ethyl-N-{(2S)-1-[5-(4-fluorophenyl)-2H-tetrazol-2-yl]propan-2-yl}-5-methyl-2-(2H-1,2,3-triazol-2-yl)benzamide,
N-{(2S)-1-[5-(4-fluorophenyl)-2H-tetrazol-2-yl]propan-2-yl}-N-(2-methoxyethyl)-5-methyl-2-(2H-1,2,3-triazol-2-yl)benzamide,
N-{(2S)-1-[5-(4-fluorophenyl)-2H-tetrazol-2-yl]propan-2-yl}-N-(2-hydroxyethyl)-5-methyl-2-(2H-1,2,3-triazol-2-yl)benzamide,
N-ethyl-N-{(2S)-1-[5-(4-fluorophenyl)-2H-tetrazol-2-yl]propan-2-yl}-6-methyl-3-(2H-1,2,3-triazol-2-yl)pyridine-2-carboxamide,
N-ethyl-5-fluoro-N-{(2S)-1-[5-(4-fluorophenyl)-2H-tetrazol-2-yl]propan-2-yl}-2-(2H-1,2,3-triazol-2-yl)benzamide,
N-ethyl-N-{(2S)-1-[5-(4-fluorophenyl)-2H-tetrazol-2-yl]propan-2-yl}-2-(2H-1,2,3-triazol-2-yl)benzamide,
N-ethyl-N-{(2S)-1-[5-(4-fluorophenyl)-2H-tetrazol-2-yl]propan-2-yl}-5-methyl-2-(pyrimidin-2-yl)benzamide,
N-ethyl-N-{(2S)-1-[5-(4-fluorophenyl)-2H-tetrazol-2-yl]propan-2-yl}-6-methyl-3-(pyrimidin-2-yl)pyridine-2-carboxamide,
N-ethyl-5-fluoro-N-{(2S)-1-[5-(4-fluorophenyl)-2H-tetrazol-2-yl]propan-2-yl}-2-(pyrimidin-2-yl)benzamide,
N-ethyl-N-{(2S)-1-[4-(4-fluorophenyl)-1H-1,2,3-triazol-1-yl]propan-2-yl}-5-methyl-2-(2H-1,2,3-triazol-2-yl)benzamide,
N-{(2S)-1-[4-(4-fluorophenyl)-1H-1,2,3-triazol-1-yl]propan-2-yl}-N-(2-hydroxyethyl)-5-methyl-2-(2H-1,2,3-triazol-2-yl)benzamide,
N-ethyl-N-{1-[1-(5-fluoropyridin-2-yl)-1H-pyrazol-4-yl]propan-2-yl}-5-methyl-2-(2H-1,2,3-triazol-2-yl)benzamide,
N-ethyl-N-{1-[1-(5-fluoropyridin-2-yl)-1H-pyrazol-3-yl]propan-2-yl}-5-methyl-2-(2H-1,2,3-triazol-2-yl)benzamide,
N-ethyl-N-{(2S)-1-[5-(4-fluorophenyl)-l,3,4-oxadiazol-2-yl]propan-2-yl}-5-methyl-2-(2H-1,2,3-triazol-2-yl)benzamide,
N-ethyl-N-{(2S)-1-[4-(5-fluoropyridin-2-yl)-1H-1,2,3-triazol-1-yl]propan-2-yl}-5-methyl-2-(2H-1,2,3-triazol-2-yl)benzamide,
N-ethyl-N-{(2S)-1-[4-(4-fluorophenyl)-1H-1,2,3-triazol-1-yl]propan-2-yl}-6-methyl-3-(2H-1,2,3-triazol-2-yl)pyridine-2-carboxamide,
N-ethyl-5-fluoro-N-{(2S)-1-[4-(4-fluorophenyl)-1H-1,2,3-triazol-1-yl]propan-2-yl}-2-(2H-1,2,3-triazol-2-yl)benzamide,
N-ethyl-N-{(2S)-1-[5-(5-fluoropyridin-2-yl)-2H-tetrazol-2-yl]propan-2-yl}-5-methyl-2-(2H-1,2,3-triazol-2-yl)benzamide,
N-ethyl-5-fluoro-N-{(2S)-1-[5-(5-fluoropyridin-2-yl)-2H-tetrazol-2-yl]propan-2-yl}-2-(2H-1,2,3-triazol-2-yl)benzamide,
N-ethyl-N-{(2S)-1-[5-(5-fluoropyridin-2-yl)-2H-tetrazol-2-yl]propan-2-yl}-5-methyl-2-(pyrimidin-2-yl)benzamide,
N-[2-(dimethylamino)ethyl]-N-{(2S)-1-[4-(4-fluorophenyl)-1H-1,2,3-triazol-1-yl]propan-2-yl}-5-methyl-2-(2H-1,2,3-triazol-2-yl)benzamide,
N-{(2S)-1-[4-(4-fluorophenyl)-1H-1,2,3-triazol-1-yl]propan-2-yl}-5-methyl-N-[2-(methylsulfony)ethyl]-2-(2H-1,2,3-triazol-2-yl)benzamide,
N-{(2S)-1-[4-(4-chlorophenyl)-1H-1,2,3-triazol-1-yl]propan-2-yl}-N-ethyl-5-methyl-2-(2H-1,2,3-triazol-2-yl)benzamide,
N-ethyl-N-{(2S)-1-[4-(3-hydroxyphenyl)-1H-1,2,3-triazol-1-yl]propan-2-yl}-5-methyl-2-(2H-1,2,3-triazol-2-yl)benzamide,
N-ethyl-5-methyl-N-{(2S)-1-[4-(pyridin-2-yl)-1H-1,2,3-triazol-1-yl]propan-2-yl}-2-(2H-1,2,3-triazol-2-yl)benzamide,
N-ethyl-5-methyl-N-[(2S)-1-(4-phenyl-1H-1,2,3-triazol-1-yl)propan-2-yl]-2-(2H-1,2,3-triazol-2-yl)benzamide,
N-ethyl-5-methyl-N-{(2S)-1-[4-(3-methylphenyl)-1H-1,2,3-triazol-1-yl]propan-2-yl}-2-(2H-1,2,3-triazol-2-yl)benzamide,
N-ethyl-N-{(2S)-1-[4-(3-fluorophenyl)-1H-1,2,3-triazol-1-yl]propan-2-yl}-5-methyl-2-(2H-1,2,3-triazol-2-yl)benzamide,
N-ethyl-N-{(2S)-1-[4-(4-fluoro-3-methoxyphenyl)-1H-1,2,3-triazol-1-yl]propan-2-yl}-5-methyl-2-(2H-1,2,3-triazol-2-yl)benzamide,
N-ethyl-N-{(2S)-1-[4-(4-hydroxyphenyl)-1H-1,2,3-triazol-1-yl]propan-2-yl}-5-methyl-2-(2H-1,2,3-triazol-2-yl)benzamide,
N-ethyl-N-{(2S)-1-[4-(3-methoxyphenyl)-1H-1,2,3-triazol-1-yl]propan-2-yl}-5-methyl-2-(2H-1,2,3-triazol-2-yl)benzamide, and
N-{(2S)-1-[4-(4-fluorophenyl)-1H-pyrazol-1-yl]propan-2-yl}-N-(2-hydroxypropyl)-5-methyl-2-(2H-1,2,3-triazol-2-yl)benzamide.

24. A pharmaceutical composition containing the compound or a pharmaceutically acceptable salt thereof according to any one of claims 1 to 23, as an active ingredient.

25. A therapeutic or preventive drug for sleep disorder, depression, anxiety disorder, panic disorder, schizophrenia, drug dependence, Alzheimer's disease, Parkinson's disease, Huntington's disease, eating disorder, headache, migraine, pain, gastrointestinal disease, epilepsy, inflammation, immunological diseases, endocrine related disease or hypertension, containing the compound or a pharmaceutically acceptable salt thereof according to any one of claims 1 to 23, as an active ingredient.
